# EUROPEAN PATENT SPECIFICATION

(11) **EP 1 727 828 B1**
(45) Date of publication and mention of the grant of the patent: **30.12.2009**
(21) Application number: 05734245.3
(22) Date of filing: 29.03.2005
(51) Int. Cl.: C07K 14/245, A61K 39/108, C07K 16/12

(54) **COMPOSITIONS OF POLYPEPTIDES SPECIFIC TO PATHOGENIC STRAINS AND THEIR USE AS VACCINES AND IN IMMUNOTHERAPY**
ZUSAMMENSETZUNGEN VON FÜR PATHOGENE STÄMME SPEZIFISCHEN POLYPEPTIDEN UND DEREN VERWENDUNG ALS IMPFSTOFFE UND IN DER IMMUNTHERAPIE
COMPOSITIONS POLYPEPTIDIQUES SPECIFIQUES D'UNE SOUCHE PATHOGENE D' E. COLI ET LEUR UTILISATION COMME VACCINS ET EN IMMUNOTHERAPIE

(30) Priority: 26.03.2004 EP 04290818
(43) Date of publication of application: 06.12.2006
(60) Divisional application: 09163010.3
(73) Proprietor: Mutabilis, 93230 Romainville (FR)
(72) Inventor: ESCAICH, Sonia, F-75012 Paris (FR)
(74) Representative: Peaucelle, Chantal
(86) International application number: PCT/EP2005/003972
(87) International publication number: WO 2005/097823

(56) References cited:
- WO-A-01/21636
- WO-A-03/074553

## Description

The invention relates to a new composition of polypeptides specific to pathogenic strains, particularly to extra-intestinal *E*. *coli* strains.

It more particularly relates to a combination of antigenic polypeptides and combination of antibodies directed against said polypeptides and to their use as vaccines and in immunotherapy, respectively.

Although *Escherichia coli* is probably the best known bacterial species and is one of the most common isolated in clinical microbiology laboratories, misconceptions abound regarding the various types of *E*. *coli* and the infections they cause. *E*. *coli* strains of biological significance to humans can be broadly classified in 3 major groups:
1. Commensal strains, which are part of the normal flora.
2. Intestinal pathogenic strains, which are not part of the normal flora. This group contains various pathotypes (EPEC, EHEC, ETEC, EIEC) not including *Shigella*.
3. Extra-intestinal strains (ExPEC) which are responsible for infections outside the gastro-intestinal (GI) tract, but can also be part of the normal flora. All hosts, either immunocompromised or not are susceptible to these infections.

ExPEC strains are responsible for the majority of the urinary tract infections (UTI) particularly cystitis, pyelonephritis, and catheter associated infections.

They are also responsible for abdominal infections, nosocomial pneumoniae, neonatal meningitidis, soft tissue infections, and bone infections. Each one of these localizations can lead to bacteremia with a risk of sepsis in case of organ failure. ExPEC strains are indeed the most common Gram negative bacilli isolated from blood cultures.

750 000 cases of bacterial sepsis occur each year in the US, and are responsible for 225 000 deaths. In a recent study on 1690 cases of sepsis, it was shown that the main bacteria species identified is ExPEC (16% of the cases) and then *S.aureus* (14% of the cases).

These numbers demonstrate the importance of ExPEC strains in both hospital and community acquired infections.

ExPEC strains correspond to a homogenous subset of *E. coli* strains. Analysis of phylogenetic relationships among *E. coli* strains by MLEE has revealed that *E. coli* belong to 4 main phylogenetic groups designated A, B1, B2 and D.

The pathogenesis of ExPEC strains is that of extra-cellular microorganisms, i.e., they are well adapted to growth in the extra-cellular fluids and efficiently resist phagocytosis by polymorphonuclear. Initial studies have shown that virulence factors known to be important for the extra-cellular growth are mainly found in B2/D *E*. *coli*., thus suggesting that B2/D subgroups contain most of the ExPEC strains. This was reinforced by experiments performed on animals showing that B2/D strains are more virulent than A and B1 strains. Subsequent epidemiological studies have indeed confirmed these hypotheses. B2/D isolates are those predominantly responsible for neonatal meningitidis (87%) and community or nosocomial acquired urosepsis, (93% and 85%, respectively).

Similar results have been reported for cystitis (70% are due to the sole B2 *E. coli*), thus demonstrating that the importance of ExPEC strains.

These recent findings demonstrate that the B2/D subgroup of strains is the *E. coli* core genome the best adapted to growth in extra-cellular fluids.

In addition to this core genome, ExPEC strains have various pathogenicity islands which encode virulence factors associated with the different pathogenesis of extra-intestinal *E*. *coli* infections (UTI, urosepsis, neonatal meningitidis...). Among the main virulence factors are the capsule, which is well-known to be important for extra-cellular growth, and the iron chelation systems (aerobactin and enterochelin, for example). In addition, depending on the pathogenesis, these strains can produce toxins (CNF, hemolysin...), adhesins (pap, sfa...) and other iron chelation systems.

The notion that B2/D *E. coli* correspond to a distinct subset of pathogenic *E*. *coli* strains is reinforced by the fact that B2/D *E. coli* are not broadly isolated from the stools of humans. They were recovered from only 11% of individuals, whereas A and B1 subgroups are present in the stools of 74% of the individuals of a human population.

As mentioned above the pathogenesis of ExPEC strains relies on their ability to multiply in the extra-cellular fluids and to resist bactericidal activity of the complement and phagocytosis by polymorphonuclear. Therefore, as for other extra-cellular pathogens (*Haemophilus influenzae, Streptococcus pneumoniae* and *Neisseria meningitidis*) a protective antigen against ExPEC has to induce antibodies that promote opsonisation and/or the bactericidal activity of serum.

Considering the above statements, an efficient antigen has to be largely represented among the population of B2/D *E*. *coli*.

Similarly to other extra-cellular pathogens, the capsular polysaccharide would be an ideal antigen, however most pathogenic B2 strains express the K1 polysaccharide. The latter has a structure identical to that of group B meningococcus, which is non-immunogenic and shares common antigens with the brain. Another possible target may be the lipopolysaccharide (LPS). However there are a large number of different LPS serotypes that are shared by various subgroups.

The inventors have now found that a specific composition of polypeptides coded by the B2/D genome, but absent from A and B1 *E. coli* strains, is particularly useful as antigen and can specifically prevent the pathologies due to ExPEC strains. Homologs of this antigenic component can be found in other pathogenic bacterial species and therefore is useful to prevent the pathologies caused by these bacteria. Accordingly, any reference to products specific to ExPEC strains and to their uses will encompass components in these species.

For example homologous antigens could be present in the following species and be as such used for prevention of disease due to the bacteria:
*Pseudomonas aeruginosa, Escherichia* coli O157:H7, *Yersinia pestis, Vibrio cholerae, Legionella pneumophila, Salmonella enterica, Salmonella typhimurium, Haemophilus influenzae, Neisseria meningitidis, Neisseria gonorrhoeae,*
*Bacillus anthracis, Burkholderia cepacia, Campylobacter jejuni, Chlamydia pneumoniae, Chlamydia trachomatis, Clostridium botulinum, Clostridium difficile, Cryptococcus neoformans, Enterobacter cloacae, Enterococcus faecalis, Helicobacter pylori, Klebsiella pneumoniae, Mycobacterium leprae, Mycobacterium tuberculoses, Pseudomonas aeruginosa, Salmonella paratyphi, Salmonella typhi, Staphylococcus aureus, Klebsiella pneumonia, Listeria monocytogenes, Moxarella catarrhalis, Shigella dysenteriae, Shigella flexneri, Shigella sonnei, Staphylococcus epidermidis, Streptococcus pneumoniae,* and any species falling within the genera of any of the above species.

It is then an object of the invention to provide a new combination of isolated antigenic polypeptides, and a new combination of isolated polynucleotides belonging to the core B2/D genome and not present in commensal *E. coli*.

Another object of the invention is to provide a new combination of antibodies raised against the antigenic polypeptides of said combination

It is still another object of the invention to provide vectors and host cells containing said polynucleotides.

The invention also relates to means for detecting and treating a development of *E*. *coli* in a human or animal comportment which is extra-intestinal (systemic and non-diarrhoeal infections, such as septicaemia, pyelonephritis, or meningitis in the newborn).

The combination of isolated antigenic polypeptides used according to the invention is selected among polypeptides specific to B2/D *E. coli* strains and not present in A and B1 isolates of *E. coli*. It is encoded by genes belonging to the core B2/D genome and are not present in commensal *E. coli*.

The composition of the invention of polypeptides specific to pathogenic strains comprises a polypeptide of a first group, having sequence SEQ ID N°145 and a polypeptide of a second group, having sequence SEQ ID N° 159The above-mentioned polypeptide of the first group and the polynucleotide coding for said polypeptide are disclosed in WO 03/074553 in the name of Mutabilis SA.

The polypeptide of SEQ ID N° 159 and the polynucleotide having SEQ ID N° 160 coding for said polypeptide are disclosed in WO 0121636 in the name of New-York University.

The invention also relates to the use in combination of isolated polynucleotide of SEQ ID N°145 and of isolated polynucleotide coding for the polypeptide of sequence SEQ ID N°159. The invention relates to the use such as above defined wherein the isolated polynucleotide used in combination have sequences SEQ ID N°146 and SEQ ID N°160 and in combination with.

The present application is also aimed towards any expression vector comprising both isolated polynucleotides such as above defined.. The term "polynucleotide" encompasses any nucleotidic sequence such as DNA, including cDNA, RNA, including mRNA.

The invention also relates to any host cell transformed by genetic engineering, **characterized in that** it comprises, by transfection, an expression vector such as above defined. The combination of said antigenic polypeptides are capable of inducing an antibody response for prevention of infections due to ExPEC strains regardless of the pathogenesis and of the infection site (UTI, pyelonephritis, sepsis, bacteremia, neonatal meningitis).

The invention thus relates to vaccine compositions specific to *E. coli* extra-intestinal infections, comprising an effective amount of the composition of antigenic polypeptides such as above defined with a carrier.

Such vaccine compositions are particularly useful for preventing urinary system infections, pyelonephritis, sepsis, bacteremia, neonatal meningitis.
The vaccine compositions of the invention are indicated for:
- Immunodepressed patients, ideally before the start of the immunosuppressive therapy: patients suffering from cancer, diabetes, leukemia, transplant patients, patients receiving long-term steroids therapy.
- Patients before surgery where there is a high risk of *E. coli* infections (abdominal surgery).
- Patients with recurrent UTI, especially after one episode of pyelonephritis,
- The prevention of neonatal infections will require vaccination of the mother, implying vaccination long before pregnancy to avoid potential problem. Ideally such a vaccine should be associated with a Group B *Streptococcus* polysaccharide vaccine in order to also prevent late onset neonatal infections. It should be pointed out that the induction of a level of antibodies against B2/D *E. coli* in pregnant women would also prevent UTI, which are always a risk in the context of a pregnancy.
   The invention also relates to the *E. coli* vaccine composition such as above defined adapted to specific indication in combination with components directed against other bacteria such as *Staphylococcus aureus* or group B *Streptococcus* or other bacteria implicated in systemic infections.

The formulation and the dose of said vaccine compositions can be developed and adjusted by those skilled in the art as a function of the indication targeted, of the method of administration desired, and of the patient under consideration (age, weight).

These compositions comprise one or more physiologically inert vehicles, and in particular any excipient suitable for the

For example the vaccine could be a suspension of the purified polypeptide in sterile water with aluminum based mineral salt as adjuvant and be administered subcutaneously with a first and boosting injection.
- The compositions of antibodies specific to polypeptidic antigens of pathogenic strains particularly to extra-intestinal *E.coli* strains, comprising combinations of antibodies directed against
   the polypeptide of sequence SEQ ID N° 145 and the antibodies directed against the polypeptide of sequence SEQ ID N°159. In compositions of the invention said antibodies are monoclonal antibodies.

They are capable of binding to said polypeptides in physiological-type conditions (*in vivo* or mimicking *in vino*) when administered to a human or animal organism, and ELISA-type conditions when said binding product is intended to be used in assays and methods in vitro. Such combinations of antibodies advantageously inhibit the extra-intestinal growth of ExPEX strains in human or animal.

The invention thus relates to pharmaceutical compositions comprising an effective amount of a combination of antibodies such as above defined.

Such pharmaceutical compositions are particularly useful for immunotherapy applications for treatment and prevention of severe infections in at risk populations such as neonates or patients undergoing surgical procedures, or having urinary tract infections to prevent septicemia. For these applications specific human monoclonal antibody (Mab) will be derived from said peptides or polypeptides.

Such pharmaceutical compositions comprising an effective amount of a combination of antibodies such as above defined are also useful for treating neonatal infections, in association with antibodies against *Staphylococcus aureus* and/or antibodies against group B *Streptococcus.*

The pharmaceutical composition of the invention used for alleviating and/or preventing and/or treating an undesirable growth of *E.coli* comprises an effective amount of the composition of polypeptides such as above defined, in combination with a pharmaceutically acceptable carrier.

The methods for manufacturing the above antibodies using the polypeptides of the combination according to the invention are available to those skilled in the art. They are conventional methods which comprise, in particular, the immunization of animals such as rabbits and the harvesting of the serum produced, followed optionally by the purification of the serum obtained. A technique suitable for the production of monoclonal antibodies is that of Köhler and Milstein (Nature 1975, 256:495-497).

Said antibodies do not recognize the cells of the human or animal to which it is intended.

The antibodies or fragments thereof are advantageously humanized when intended for a human administration.

Alternatively, humanized Mab could be derived from murine or rat Mab specific of the antigen. These fully humanized Mab are constructed using conventional molecular techniques to graft complementarity-determining regions from the parent murine or rat antibacterial antibody into human IgG1 kappa heavy and light-chain frameworks.

The detection of the presence or absence of the above compounds can in particular be carried out by nucleotide hybridization, by PCR amplification or by detection of their polypeptide products. Detention of the presence of such compounds makes it possible to conclude that a B2/D *E. coli* strain is present.

The invention also relates to pharmaceutical compositions for alleviating and/or preventing and/or treating an undesirable growth of *E. coli* comprising an effective amount of a polypeptide of sequence SEQ ID N°145, for the first group, and SEQ ID N° 159 for the second group, in combination with a pharmaceutically acceptable carrier.

The present application is also aimed towards any use of said combination of polypeptides such as above defined for the manufacture of a composition, in particular of a pharmaceutical composition, intended to alleviate and/or to prevent and/or to treat an undesirable growth of *E. coli*, such as an *E. coli* infection, (for example systemic and non-diarrhoeal infections), the presence of extra-intestinal *E. coli* or a sanitary contamination.

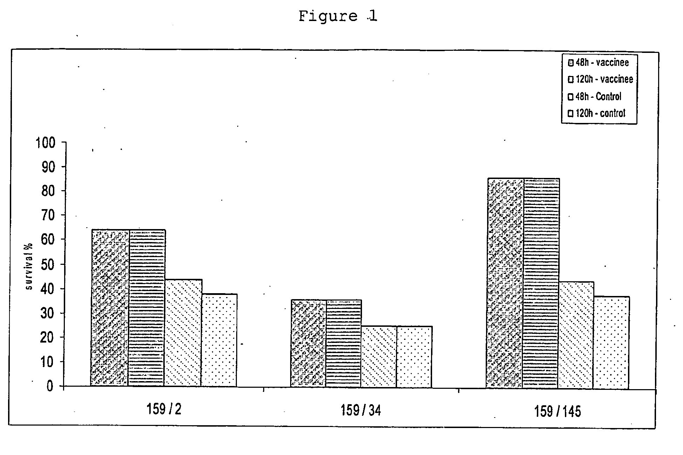

The present invention is illustrated by the examples, which follow and which are given in a non limiting capacity. In said examples, it is referred to Figure 1 which represents histograms of the results showing an increase of survival in the animals vaccinated with polypeptides combinations.

Example of vaccination to demonstrate immunogenicity of polypeptides:

### Example 1:

Example of antigens combination to induce an immune response that protects mice after experimental challenge with a pathogenic strain of *E.coli* (ExPEc):

### Experimental protocol:

- Balb/c mice, female, 6 weeks old were immunized on day 1 by subcutaneous injection of a solution containing a combination of two purified polypeptides (20 micrograms of each) and Complete Freund's adjuvant (CFA) in PBS, and control mice were injected with CFA in PBS buffer.
- 3 weeks later a boost injection of the same combination of polypeptides in solution (10 micrograms of each) with the incomplete Freund's adjuvant was performed. Before challenge on day 42, sera was collected on day 41 to analyze the antibody response in the vaccinated animals:
- WB analysis of sera from immunized mice were performed to detect the antibody response to the recombinant protein used for immunization as described above.
- An ELISA assay was used to measure polypeptides specific antibody titres obtained in vaccinated animals:
   Experimental challenge to measure protection induced by antigens combination:

On day 42, vaccinated and control mice were challenged with an *E.coli* ExPEC virulent strain belonging to B2 group at a dose equal to the LD 50 (5.10⁵ cfu/mice) by intraperitoneal injection. The end point of the assay was the survival to the lethal challenge. Mortality observed in each group of animals was recorded at 48h and 120h. The results are shown in Table 1 are expressed as a percentage of survival in the vaccine group versus the control mice group.

**Table 1: Protection obtained in mice challenged after immunization with combination of polypeptides encoded by the corresponding ORFs.**

| **Combination of two polypeptides** | | **% Survival at 48 h (nb of mice alive/nb total)** | | **% Survival at 120h (nb of mice alive/nb total)** | |
|---|---|---|---|---|---|
| SEQ ID / polypeptide **1** | SEQ ID polypeptide **2** | **Vaccine** | **Control** | **Vaccine** | **Control** |
| **159 / 2** | | **64 (9114)** | **44 (7/16)** | **64 (9/14)** | **38 (6/16)** |
| **159 / 34** | | **36 (5/14)** | **25 (4/16)** | **36 (5114)** | **25 (4/16)** |
| **159 / 145** | | **86 (12/14)** | **44 (7/16)** | **86 (12/14)** | **38 (6/16)** |

Figure 1 represents histograms of the results and shows an increase of survival in the animals vaccinated with the polypeptide combination according to the invention (the two other combinations of polypeptides are not part of the invention).

## Claims

1. Composition of polypeptides specific to pathogenic strains comprising a polypeptide of a first group, having sequence SEQ ID N°145 and a polypeptide of a second group, having SEQ ID N° 159.

2. Use in combination of isolated polynucleotide coding for the polypeptide of sequence SEQ ID N°145 and of isolated polynucleotide coding for the polypeptide of sequence SEQ ID N°159.

3. Use according to claim 2, wherein the isolated polynucleotides used in combination have sequences SEQ ID N°146 and SEQ ID N°160.

4. An expression vector comprising both the isolated polynucleotides of claim 2 or 3.

5. A host cell comprising an expression vector according to claim 4.

6. Vaccine compositions specific to *E*. *coli* extra-intestinal infections, comprising an effective amount of the composition of antigenic polypeptide of claim 1 with a carrier.

7. The vaccine compositions of claim 6, for use in preventing urinary system infections, pyelonephritis, sepsis, bacteremia, neonatal meningitidis.

8. The vaccine composition of claim 6 or 7, adapted to specific indication in combination with components directed against other bacteria, such as *S. Aureus* or group *B Streptococcus*, or other bacteria implicated in systemic infections.

9. Compositions of antibodies specific to polypeptidic antigens of pathogenic strains particularly to extra-intestinal *E. Coli* strains, comprising combinations of antibodies directed against the polypeptide of sequence SEQ ID N°145 and antibodies directed against the polypeptide of sequence SEQ ID N°159.

10. Compositions according to claim 9, wherein said antibodies are monoclonal antibodies.

11. Pharmaceutical compositions comprising a combination of antibodies according to claim 9 or 10.

12. Pharmaceutical compositions according to claim 11 comprising an effective amount of a combination of antibodies according to claim 5 or 6, for use in treating neonatal infections, in association with antibodies against *Staphylococcus aureus* and/or antibodies against group B *Streptococcus.*

13. A pharmaceutical composition according to claim 11 or 12 for use in treatment or prevention of severe infection due to Expec in neonates and patients at risk for such infections.

14. Pharmaceutical compositions for use in alleviating and/or preventing and/or treating an undesirable growth of *E*. *Coli* comprising an effective amount of the composition of polypeptides according to claim 1, in combination with a pharmaceutically acceptable carrier.

## Patentansprüche

1. Zusammensetzung von Polypeptiden, welche für pathogene Stämme spezifisch sind, umfassend ein Polypeptid einer ersten Gruppe, welches Sequenz SEQ ID N°145 aufweist und ein Polypeptid aus einer zweiten Gruppe, welches SEQ ID N°159 aufweist.

2. Verwendung in Kombination des isolierten Polynucleotids, welches für das Polypeptid der Sequenz SEQ ID N°145 codiert und des isolierten Polynucleotids, welches für das Polypeptid der Sequenz SEQ ID N°159 codiert.

3. Verwendung nach Anspruch 2, wobei die isolierten Polynucleotide, welche in Kombination verwendet werden, Sequenzen SEQ ID N°146 und SEQ ID N°160 aufweisen.

4. Expressionsvektor, welcher beide der isolierten Polynucleotide nach Anspruch 2 oder 3 umfasst.

5. Wirtzelle umfassend einen Expressionsvektor nach Anspruch 4.

6. Impfstoffzusammensetzungen, welche spezifisch für extraintestinale Infektionen von *E*. *coli* sind, umfassend eine wirksame Menge der Zusammensetzung des antigenischen Polypeptids aus Anspruch 1 mit einem Träger.

7. Impfstoffzusammensetzungen nach Anspruch 6, für die Verwendung in der Prävention von Harnweginfektionen, Pyelonephritis, Sepsis, Bakteriämie, neonataler Meningitis.

8. Impfstoffzusammensetzung nach Anspruch 6 oder 7, welche zur spezifischen Indikation in Verbindung mit Bestandteilen, welche sich gegen andere Bakterien wie *S*. *aureus* oder Gruppe B *Streptokokken* oder andere Bakterien, die in systemischen Infektionen impliziert sind richten, angepasst ist.

9. Zusammensetzungen von Antikörpern, welche spezifisch für polypeptidische Antigene von pathogenen Stämmen, insbesondere zu extraintestinalen *E. coli-*Stämmen sind, umfassend Kombinationen von Antikörpern, welche sich gegen das Polypeptid der Sequenz SEQ ID N°145 richten und Antikörpern, welche sich gegen das Polypeptid der Sequenz SEQ ID N°159 richten.

10. Zusammensetzungen nach Anspruch 9, wobei die Antikörper monoklonale Antikörper sind.

11. Pharmazeutische Zusammensetzungen, umfassend eine Kombination von Antikörpern gemäß Anspruch 9 oder 10.

12. Pharmazeutische Zusammensetzungen nach Anspruch 11, umfassend eine wirksame Menge von einer Kombination der Antikörper aus Anspruch 5 oder 6 für die Verwendung zur Behandlung von neonatalen Infektionen, in Verbindung mit Antikörpern gegen *Staphylococcus aureus* und/oder Antikörpern gegen Gruppe B *Streptokokken.*

13. Pharmazeutische Zusammensetzung nach Anspruch 11 oder 12 für die Verwendung zur Behandlung oder Prävention von ernsten Infektionen aufgrund von ExPEC in Neugeborenen und Patienten, die für solche Infektionen gefährdet sind.

14. Pharmazeutische Zusammensetzungen für die Verwendung zur Linderung und/oder Prävention und/oder Behandlung eines unerwünschten Wachstums von *E. coli*, umfassend eine wirksame Menge von der Zusammensetzung der Polypeptide nach Anspruch 1, in Verbindung mit einem pharmazeutisch akzeptablen Träger.

## Revendications

1. Composition de polypeptides spécifiques pour des souches pathogènes, comprenant un polypeptide d'un premier groupe, de séquence SEQ ID N° 145, et un polypeptide d'un deuxième groupe, de séquence SEQ ID N° 159.

2. Utilisation combinée d'un polynucléotide isolé codant le polypeptide de séquence SEQ ID N° 145 et d'un polynucléotide isolé codant le polypeptide de séquence SEQ ID N° 159.

3. Utilisation selon la revendication 2, dans laquelle les polynucléotides isolés utilisés de manière combinée sont représentés par les séquences SEQ ID N° 146 et SEQ ID N° 160.

4. Vecteur d'expression comprenant les deux polynucléotides isolés selon la revendication 2 ou 3.

5. Cellule hôte comprenant un vecteur d'expression selon la revendication 4.

6. Compositions vaccinales spécifiques pour les infections extra-intestinales à *E. coli*, comprenant une quantité efficace de la composition de polypeptides antigéniques de la revendication 1 avec un véhicule.

7. Compositions vaccinales selon la revendication 6, à utiliser pour la prévention des infections du système urinaire, de la pyélonéphrite, de la septicémie, de la bactériémie, de la méningite néonatale.

8. Composition vaccinale selon la revendication 6 ou 7, adaptée pour certaines indications spécifiques, combinée à des composants dirigés contre d'autres bactéries telles que *S*. *aureus* ou les streptocoques du groupe B, ou contre d'autres bactéries impliquées dans les infections systémiques.

9. Compositions d'anticorps spécifiques pour des antigènes polypeptidiques de souches pathogènes, en particulier les souches d'*E. coli* extra-intestinales, comprenant des combinaisons d'anticorps dirigés contre le polypeptide de séquence SEQ ID N° 145 et d'anticorps dirigés contre le polypeptide de séquence SEQ ID N° 159.

10. Compositions selon la revendication 9, dans lesquelles lesdits anticorps sont des anticorps monoclonaux.

11. Compositions pharmaceutiques comprenant une combinaison d'anticorps selon la revendication 9 ou 10.

12. Compositions pharmaceutiques selon la revendication 11, comprenant une quantité efficace d'une combinaison d'anticorps selon la revendication 5 ou 6, à utiliser pour le traitement d'infections néonatales, en association avec des anticorps dirigés contre *Staphylococcus aureus* et/ou des anticorps dirigés contre les streptocoques du groupe B.

13. Composition pharmaceutique selon la revendication 11 ou 12, à utiliser pour le traitement ou la prévention d'infections graves dues à des souches ExPEC chez les nouveaux-nés et chez les patients à risque pour de telles infections.

14. Compositions pharmaceutiques à utiliser pour l'atténuation et/ou la prévention et/ou le traitement du développement non souhaitable d'*E. coli*, comprenant une quantité efficace de la composition de polypeptides selon la revendication 1, combinée à un véhicule acceptable sur le plan pharmaceutique.


### SEQUENCE LISTING

<110> MUTABILIS
<120> Comprising of polypeptides specific to pathogenic strains and their use as vaccines and in immunotherapy
<130> .2209
<160> 160
<170> PatentIn version 3.1
<210> 1
   <211> 163
   <212> PRT
   <213> Escherichia coli
<400> 1
   Met Lys Leu Lys Ala Ile Ile Leu Ala Thr Gly Leu Ile Asn Cys Ile
   1 5 10 15
   Val Phe Ser Ala Gln Ala Val Asp Thr Thr Ile Thr Val Thr Gly Asn
   20 25 30
   Val Leu Gln Arg Thr Cys Asn Val Pro Gly Asn Val Asp Val Ser Leu
   35 40 45
   Gly Asn Leu Tyr Val Ser Asp Phe Pro Asn Ala Gly Ser Gly Ser Pro
   50 55 60
   Trp Val Asn Phe Asp Leu Ser Leu Thr Gly Cys Gln Asn Met Asn Thr
   65 70 75 80
   Val Arg Ala Thr Phe Ser Gly Thr Ala Asp Gly Gln Thr Tyr Tyr Ala
   85 90 95
   Asn Thr Gly Asn Ala Gly Gly Ile Lys Ile Glu Ile Gln Asp Arg Asp
   100 105 110
   Gly Ser Asn Ala Ser Tyr His Asn Gly Met Phe Lys Thr Leu Asn Val
   115 120 125
   Gln Asn Asn Asn Ala Thr Phe Asn Leu Lys Ala Arg Ala Val Ser
   Lys
   130 135 140
   Gly Gln Val Thr Pro Gly Asn Ile Ser Ser Val Ile Thr Val Thr
   Tyr
   145 150 155
   160
   Thr Tyr Ala
<210> 2
   <211> 673
   <212> PRT
   <213> Escherichia coli
<400> 2
   Met Lys Met Thr Arg Leu Tyr Pro Leu Ala Leu Gly Gly Leu Leu Leu
   1 5 10 15
   Pro Ala Ile Ala Asn Ala Gln Thr Ser Gln Gln Asp Glu Ser Thr Leu
   20 25 30
   Val Val Thr Ala Ser Lys Gln Ser Ser Arg Ser Ala Ser Ala Asn Asn
   35 40 45
   Val Ser Ser Thr Val Val Ser Ala Pro Glu Leu Ser Asp Ala Gly Val
   50 55 60
   Thr Ala Ser Asp Lys Leu Pro Arg Val Leu Pro Gly Leu Asn Ile Glu
   65 70 75 80
   Asn Ser Gly Asn Met Leu Phe Ser Thr Ile Ser Leu Arg Gly Val Ser
   85 90 95
   Ser Ala Gln Asp Phe Tyr Asn Pro Ala Val Thr Leu Tyr Val Asp Gly
   100 105 110
   Val Pro Gln Leu Ser Thr Asn Thr Ile Gln Ala Leu Thr Asp Val Gln
   115 120 125
   Ser Val Glu Leu Leu Arg Gly Pro Gln Gly Thr Leu Tyr Gly Lys Ser
   130 135 140
   Ala Gln Gly Gly Ile Ile Asn Ile Val Thr Gln Gln Pro Asp Ser Thr
   145 150 155 160
   Pro Arg Gly Tyr Ile Glu Gly Gly Val Ser Ser Arg Asp Ser Tyr Arg
   165 170 175
   Ser Lys Phe Asn Leu Ser Gly Pro Ile Gln Asp Gly Leu Leu Tyr Gly
   180 185 190
   Ser Val Thr Leu Leu Arg Gln Val Asp Asp Gly Asp Met Ile Asn Pro
   195 200 205
   Ala Thr Gly Ser Asp Asp Leu Gly Gly Thr Arg Ala Ser Ile Gly Asn
   210 215 220
   Val Lys Leu Arg Leu Ala Pro Asp Asp Gln Pro Trp Glu Met Gly Phe
   225 230 235 240
   Ala Ala Ser Arg Glu Cys Thr Arg Ala Thr Gln Asp Ala Tyr Val Gly
   245 250 255
   Trp Asn Asp Ile Lys Gly Arg Lys Leu Ser Ile Ser Asp Gly Ser Pro
   260 265 270
   Asp Pro Tyr Met Arg Arg Cys Thr Asp Ser Gln Thr Leu Ser Gly Lys
   275 280 285
   Tyr Thr Thr Asp Asp Trp Val Phe Asn Leu Ile Ser Ala Trp Gln Gln
   290 295 300
   Gln His Tyr Ser Arg Thr Phe Pro Ser Gly Ser Leu Ile Val Asn Met
   305 310 315 320
   Ser Gln Arg Trp Asn Gln Asp Val Gln Glu Leu Arg Ala Ala Thr Leu
   325 330 335
   Gly Asp Ala Arg Thr Val Asp Met Val Phe Gly Leu Tyr Arg Gln Asn
   340 345 350
   Thr Arg Glu Lys Leu Asn Ser Ala Tyr Asp Met Pro Thr Met Pro Tyr
   355 360 365
   Leu Ser Ser Thr Gly Tyr Thr Thr Ala Glu Thr Leu Ala Ala Tyr Ser
   370 375 380
   Asp Leu Thr Trp His Leu Thr Asp Arg Phe Asp Ile Gly Gly Gly Val
   385 390 395 400
   Arg Phe Ser His Asp Lys Ser Ser Thr Gln Tyr His Gly Ser Met
   Leu
   405 410 415
   Gly Asn Pro Phe Gly Asp Gln Gly Lys Ser Asn Asp Asp Gln Val
   Leu
   420 425 430
   Gly Gln Leu Ser Ala Gly Tyr Met Leu Thr Asp Asp Trp Arg Val
   Tyr
   435 440 445
   Thr Arg Val Ala Gln Gly Tyr Lys Pro Ser Gly Tyr Asn Ile Val
   Pro
   450 455 460
   Thr Ala Gly Leu Asp Ala Lys Pro Phe Val Ala Glu Lys Ser Ile
   Asn
   465 470 475
   480
   Tyr Glu Leu Gly Thr Arg Tyr Glu Thr Ala Asp Val Thr Leu Gln
   Ala
   485 490 495
   Ala Thr Phe Tyr Thr His Thr Lys Asp Met Gln Leu Tyr Ser Gly
   Pro
   500 505 510
   Val Gly Met Gln Thr Leu Ser Asn Ala Gly Lys Ala Asp Ala Thr
   Gly
   515 520 525
   Val Glu Leu Glu Ala Lys Trp Arg Phe Ala Pro Gly Trp Ser Trp
   Asp
   530 535 540
   Ile Asn Gly Asn Val Ile Arg Ser Glu Phe Thr Asn Asp Ser Glu
   Leu
   545 550 555
   560
   Tyr His Gly Asn Arg Val Pro Phe Val Pro Arg Tyr Gly Ala Gly Ser
   565 570 575
   Ser Val Asn Gly Val Ile Asp Thr Arg Tyr Gly Ala Leu Met Pro Arg
   580 585 590
   Leu Ala Val Asn Leu Val Gly Pro His Tyr Phe Asp Gly Asp Asn Gln
   595 600 605
   Leu Arg Gln Gly Thr Tyr Ala Thr Leu Asp Ser Ser Leu Gly Trp Gln
   610 615 620
   Ala Thr Glu Arg Met Asn Ile Ser Val Tyr Val Asp Asn Leu Phe Asp
   625 630 635 640
   Arg Arg Tyr Arg Thr Tyr Gly Tyr Met Asn Gly Ser Ser Ala Val Ala
   645 650 655
   Gln Val Asn Met Gly Arg Thr Val Gly Ile Asn Thr Arg Ile Asp Phe
   660 665 670
   Phe
<210> 3
   <211> 246
   <212> Escherichia coli
<400>
   Met Asn Lys Val Phe Val Val Ser Val Val Ala Ala Ala Cys Val Phe
   1 5 10 15
   Ala Val Asn Ala Gly Ala Lys Glu Gly Lys Ser Gly Phe Tyr Leu
   Thr
   20 25 30
   Gly Lys Ala Gly Ala Ser Val Met Ser Leu Ser Asp Gln Arg Phe
   Leu
   35 40 45
   Ser Gly Asp Glu Glu Glu Thr Ser Lys Tyr Lys Gly Gly Asp Asp
   His
   50 55 60
   Asp Thr Val Phe Ser Gly Gly Ile Ala Val Gly Tyr Asp Phe Tyr
   Pro
   65 70 75 80
   Gln Phe Ser Ile Pro Val Arg Thr Glu Leu Glu Phe Tyr Ala Arg
   Gly
   85 90 95
   Lys Ala Asp Ser Lys Tyr Asn Val Asp Lys Asp Ser Trp Ser Gly
   Gly
   100 105 110
   Tyr Trp Arg Asp Asp Leu Lys Asn Glu Val Ser Val Asn Thr Leu
   Met
   115 120 125
   Leu Asn Ala Tyr Tyr Asp Phe Arg Asn Asp Ser Ala Phe Thr Pro
   Trp
   130 135 140
   Val Ser Ala Gly Ile Gly Tyr Ala Arg Ile His Gln Lys Thr Thr
   Gly
   145 150 155
   160
   Ile Ser Thr Trp Asp Tyr Glu Tyr Gly Ser Ser Gly Arg Glu Ser
   Leu
   165 170 175
   Ser Arg Ser Gly Ser Ala Asp Asn Phe Ala Trp Ser Leu Gly Ala Gly
   180 185 190
   Val Arg Tyr Asp Val Thr Pro Asp Ile Ala Leu Asp Leu Ser Tyr Arg
   195 200 205
   Tyr Leu Asp Ala Gly Asp Ser Ser Val Ser Tyr Lys Asp Glu Trp Gly
   210 215 220
   Asp Lys Tyr Lys Ser Glu Val Asp Val Lys Ser His Asp Ile Met Leu
   225 230 235 240
   Gly Met Thr Tyr Asn Phe
   245
<210>4
   <211>166
   <212> PRT
   <213> Escherichia coli
<400>
   Met Lys Leu Lys Ala Ile Ile Leu Ala Thr Gly Leu Ile Asn Cys Ile
   1 5 10 15
   Ala Phe Ser Ala Gln Ala Val Asp Thr Thr Ile Thr Val Thr Gly Arg
   20 25 30
   Val Leu Pro Arg Thr Cys Thr Ile Gly Asn Gly Gly Asn Pro Asn Ala
   35 40 45
   Thr Val Val Leu Asp Asn Ala Tyr Thr Ser Asp Leu Ile Ala Ala Asn
   50 55 60
   Ser Thr Ser Gln Trp Lys Asn Phe Ser Leu Thr Leu Thr Asn Cys
   Gln
   65 70 75 80
   Asn Val Asn Asn Val Thr Ser Phe Gly Gly Thr Ala Glu Asn Thr
   Asn
   85 90 95
   Tyr Tyr Arg Asn Thr Gly Asp Ala Thr Asn Ile Met Val Glu Leu
   Gln
   100 105 110
   Glu Gln Gly Asn Gly Asn Thr Pro Leu Lys Val Gly Ser Thr Lys
   Val
   115 120 125
   Val Thr Val Ser Asn Gly Gln Ala Thr Phe Asn Leu Lys Val Arg
   Ala
   130 135 140
   Val Ser Lys Gly Asn Ala Gly Ala Gly Ser Ile Asn Ser Gln Ile
   Thr
   145 150 155
   160
   Val Thr Tyr Thr Tyr Ala
   165
<210>5
   <211> 1295
   <212> PRT
   <212> Escherichia coli
<400> 5
   Met Asn Lys Ile Tyr Ser Leu Lys Tyr Ser Ala Ala Thr Gly Gly
   Leu
   1 5 10 15
   Ile Ala Val Ser Glu Leu Ala Lys Arg Val Ser Gly Lys Thr Asn
   Arg
   20 25 30
   Lys Leu Val Ala Thr Met Leu Ser Leu Ala Val Ala Gly Thr Val Asn
   35 40 45
   Ala Ala Asn Ile Asp Ile Ser Asn Val Trp Ala Arg Asp Tyr Leu Asp
   50 55 60
   Leu Ala Gln Asn Lys Gly Ile Phe Gln Pro Gly Ala Thr Asp Val Thr
   65 70 75 80
   Ile Thr Leu Lys Asn Gly Asp Lys Phe Ser Phe His Asn Leu Ser Ile
   85 90 95
   Pro Asp Phe Ser Gly Ala Ala Ala Ser Gly Ala Ala Thr Ala Ile Gly
   100 105 110
   Gly Ser Tyr Ser Val Thr Val Ala His Asn Lys Lys Asn Pro Gln Ala
   115 120 125
   Ala Glu Thr Gln Val Tyr Ala Gln Ser Ser Tyr Arg Val Val Asp Arg
   130 135 140
   Arg Asn Ser Asn Asp Phe Glu Ile Gln Arg Leu Asn Lys Phe Val Val
   145 150 155 160
   Glu Thr Val Gly Ala Thr Pro Ala Glu Thr Asn Pro Thr Thr Tyr Ser
   165 170 175
   Asp Ala Leu Glu Arg Tyr Gly Ile Val Thr Ser Asp Gly Ser Lys Lys
   180 185 190
   Ile Ile Gly Phe Arg Ala Gly Ser Gly Gly Thr Ser Phe Ile Asn Gly
   195 200 205
   Glu Ser Lys Ile Ser Thr Asn Ser Ala Tyr Ser His Asp Leu Leu Ser
   210 215 220
   Ala Ser Leu Phe Glu Val Thr Gln Trp Asp Ser Tyr Gly Met Met Ile
   225 230 235 240
   Tyr Lys Asn Asp Lys Thr Phe Arg Asn Leu Glu Ile Phe Gly Asp Ser
   245 250 255
   Gly Ser Gly Ala Tyr Leu Tyr Asp Asn Lys Leu Glu Lys Trp Val Leu
   260 265 270
   Val Gly Thr Thr His Gly Ile Ala Ser Val Asn Gly Asp Gln Leu Thr
   275 280 285
   Trp Ile Thr Lys Tyr Asn Asp Lys Leu Val Ser Glu Leu Lys Asp Thr
   290 295 300
   Tyr Ser His Lys Ile Asn Leu Asn Gly Asn Asn Val Thr Ile Lys Asn
   305 310 315 320
   Thr Asp Ile Thr Leu His Gln Asn Asn Ala Asp Thr Thr Gly Thr Gln
   325 330 335
   Glu Lys Ile Thr Lys Asp Lys Asp Ile Val Phe Thr Asn Gly Gly Asp
   340 345 350
   Val Leu Phe Lys Asp Asn Leu Asp Phe Gly Ser Gly Gly Ile Ile Phe
   355 360 365
   Asp Glu Gly His Glu Tyr Asn Ile Asn Gly Gln Gly Phe Thr Phe Lys
   370 375 380
   Gly Ala Gly Ile Asp Ile Gly Lys Glu Ser Ile Val Asn Trp Asn Ala
   385 390 395 400
   Leu Tyr Ser Ser Asp Asp Val Leu His Lys Ile Gly Pro Gly Thr Leu
   405 410 415
   Asn Val Gln Lys Lys Gln Gly Ala Asn Ile Lys Ile Gly Glu Gly Asn
   420 425 430
   Val Ile Leu Asn Glu Glu Gly Thr Phe Asn Asn Ile Tyr Leu Ala Ser
   435 440 445
   Gly Asn Gly Lys Val Ile Leu Asn Lys Asp Asn Ser Leu Gly Asn Asp
   450 455 460
   Gln Tyr Ala Gly Ile Phe Phe Thr Lys Arg Gly Gly Thr Leu Asp Leu
   465 470 475 480
   Asn Gly His Asn Gln Thr Phe Thr Arg Ile Ala Ala Thr Asp Asp Gly
   485 490 495
   Thr Thr Ile Thr Asn Ser Asp Thr Thr Lys Glu Ala Val Leu Ala
   Ile
   500 505 510
   Asn Asn Glu Asp Ser Tyr Ile Tyr His Gly Asn Ile Asn Gly Asn
   Ile
   515 520 525
   Lys Leu Thr His Asn Ile Asn Ser Gln Asp Lys Lys Thr Asn Ala
   Lys
   530 535 540
   Leu Ile Leu Asp Gly Ser Val Asn Thr Lys Asn Asp Val Glu Val
   Ser
   545 550 555
   560
   Asn Ala Ser Leu Thr Met Gln Gly His Ala Thr Glu His Ala Ile
   Phe
   565 570 575
   Arg Ser Ser Ala Asn His Cys Ser Leu Val Phe Leu Cys Gly Thr
   Asp
   580 585 590
   Trp Val Thr Val Leu Lys Glu Thr Glu Ser Ser Tyr Asn Lys Lys
   Phe
   595 600 605
   Asn Ser Asp Tyr Lys Ser Asn Asn Gln Gln Thr Ser Phe Asp Gln
   Pro
   610 615 620
   Asp Trp Lys Thr Gly Val Phe Lys Phe Asp Thr Leu His Leu Asn
   Asn
   625 630 635
   640
   Ala Asp Phe Ser Ile Ser Arg Asn Ala Asn Val Glu Gly Asn Ile
   Ser
   645 650 655
   Ala Asn Lys Ser Ala Ile Thr Ile Gly Asp Lys Asn Val Tyr Ile
   Asp
   660 665 670
   Asn Leu Ala Gly Lys Asn Ile Thr Asn Asn Gly Phe Asp Phe Lys
   Gln
   675 680 685
   Thr Ile Ser Thr Asn Leu Ser Ile Gly Glu Thr Lys Phe Thr Gly
   Gly
   690 695 700
   Ile Thr Ala His Asn Ser Gln Ile Ala Ile Gly Asp Gln Ala Val
   Val
   705 710 715
   720
   Thr Leu Asn Gly Ala Thr Phe Leu Asp Asn Thr Pro Ile Ser Ile
   Asp
   725 730 735
   Lys Gly Ala Lys Val Ile Ala Gln Asn Ser Met Phe Thr Thr Lys
   Gly
   740 745 750
   Ile Asp Ile Ser Gly Glu Leu Thr Met Met Gly Ile Pro Glu Gln
   Asn
   755 760 765
   Ser Lys Thr Val Thr Pro Gly Leu His Tyr Ala Ala Asp Gly Phe
   Arg
   770 775 780
   Leu Ser Gly Gly Asn Ala Asn Phe Ile Ala Arg Asn Met Ala Ser
   Val
   785 790 795
   800
   Thr Gly Asn Ile Tyr Ala Asp Asp Ala Ala Thr Ile Thr Leu Gly
   Gln
   805 810 815
   Pro Glu Thr Glu Thr Pro Thr Ile Ser Ser Ala Tyr Gln Ala Trp
   Ala
   820 825 830
   Glu Thr Leu Leu Tyr Gly Phe Asp Thr Ala Tyr Arg Gly Ala Ile
   Thr
   835 840 845
   Ala Pro Lys Ala Thr Val Ser Met Asn Asn Ala Ile Trp His Leu
   Asn
   850 855 860
   Ser Gln Ser Ser Ile Asn Arg Leu Glu Thr Lys Asp Ser Met Val
   Arg
   865 870 875
   880
   Phe Thr Gly Asp Asn Gly Lys Phe Thr Thr Leu Thr Val Asn Asn
   Leu
   885 890 895
   Thr Ile Asp Asp Ser Ala Phe Val Leu Arg Ala Asn Leu Ala Gln
   Ala
   900 905 910
   Asp Gln Leu Val Val Asn Lys Ser Leu Ser Gly Lys Asn Asn Leu
   Leu
   915 920 925
   Leu Val Asp Phe Ile Glu Lys Asn Gly Asn Ser Asn Gly Leu Asn
   Ile
   930 935 940
   Asp Leu Val Ser Ala Pro Lys Gly Thr Ala Val Asp Val Phe Lys
   Ala
   945 950 955
   960
   Thr Thr Arg Ser Ile Gly Phe Ser Asp Val Thr Pro Val Ile Glu
   Gln
   965 970 975
   Lys Asn Asp Thr Asp Lys Ala Thr Trp Thr Leu Ile Gly Tyr Lys Ser
   980 985 990
   Val Ala Asn Ala Asp Ala Ala Lys Lys Ala Thr Leu Leu Met Ser Gly
   995 1000 1005
   Gly Tyr Lys Ala Phe Leu Ala Glu Val Asn Asn Leu Asn Lys Arg
   1010 1015 1020
   Met Gly Asp Leu Arg Asp Ile Asn Gly Glu Ser Gly Ala Trp Ala
   1025 1030 1035
   Arg Ile Ile Ser Gly Thr Gly Ser Ala Gly Gly Gly Phe Ser Asp
   1040 1045 1050
   Asn Tyr Thr His Val Gln Val Gly Ala Asp Asn Lys His Glu Leu
   1055 1060 1065
   Asp Gly Leu Asp Leu Phe Thr Gly Val Thr Met Thr Tyr Thr Asp
   1070 1075 1080
   Ser His Ala Gly Ser Asp Ala Phe Ser Gly Glu Thr Lys Ser Val
   1085 1090 1095
   Gly Ala Gly Leu Tyr Ala Ser Ala Met Phe Glu Ser Gly Ala Tyr
   1100 1105 1110
   Ile Asp Leu Ile Gly Lys Tyr Val His His Asp Asn Glu Tyr Thr
   1115 1120 1125
   Ala Thr Phe Ala Gly Leu Gly Thr Arg Asp Tyr Ser Ser His Ser
   1130 1135 1140
   Trp Tyr Ala Gly Ala Glu Val Gly Tyr Arg Tyr His Val Thr Asp
   1145 1150 1155
   Ser Ala Trp Ile Glu Pro Gln Ala Glu Leu Val Tyr Gly Ala Val
   1160 1165 1170
   Ser Gly Lys Gln Phe Ser Trp Lys Asp Gln Gly Met Asn Leu Thr
   1175 1180 1185
   Met Lys Asp Lys Asp Phe Asn Pro Leu Ile Gly Arg Thr Gly Val
   1190 1195 1200
   Asp Val Gly Lys Ser Phe Ser Gly Lys Asp Trp Lys Val Thr Ala
   1205 1210 1215
   Arg Ala Gly Leu Gly Tyr Gln Phe Asp Leu Phe Ala Asn Gly Glu
   1220 1225 1230
   Thr Val Leu Arg Asp Ala Ser Gly Glu Lys Arg Ile Lys Gly Glu
   1235 1240 1245
   Lys Asp Gly Arg Met Leu Met Asn Val Gly Leu Asn Ala Glu Ile
   1250 1255 1260
   Arg Asp Asn Leu Arg Phe Gly Leu Glu Phe Glu Lys Ser Ala Phe
   1265 1270 1275
   Gly Lys Tyr Asn Val Asp Asn Ala Ile Asn Ala Asn Phe Arg Tyr
   1280 1285 1290
   Ser Phe
   1295
<210> 6
   <211> 142
   <213> Escherichia coli
<400> 6
   Met Ile Asn Ile Pro Ser Pro Thr Ala Val Val Met Ala Leu Val
   Ala
   1 5 10 15
   Ile Ser Thr Leu Pro Ser Pro Ser Arg Val Lys Leu Met Pro Tyr Pro
   20 25 30
   Pro Arg Ala His Asn Thr Thr Gly Leu Leu Pro Val Arg Glu Ile Cys
   35 40 45
   Phe Pro His His Gly Asp Asp Gly Arg Asn Ser Ile Glu Pro Ser Ile
   50 55 60
   Ser Arg Ala Ala His Thr Asp Arg Leu Arg Phe Val Cys Met Thr Arg
   65 70 75 80
   Thr Gly Ser Thr Thr Ser Arg Pro Phe Cys Pro Ile Pro Arg Ser Pro
   85 90 95
   Ala Leu Asn Ala Ser Gly Gln Gln Asp Ser Gly Phe Trp Gly Val Ser
   100 105 110
   Ser Ile Pro Gly Asp Ile Leu Met Phe Gln Leu His Val Leu Ile Val
   115 120 125
   Phe Ile Cys Lys Ile Asn Leu Ser Asp Asn Asn Ile Ser Tyr
   130 135 140
<210> 7
   <211> 318
   <212> PRT
   <213> Escherichia coli
<400> 7
   Met Tyr Ala Arg Glu Tyr Arg Ser Thr Arg Pro His Lys Ala Ile Phe
   1 5 10 15
   Phe His Leu Ser Cys Leu Thr Leu Ile Cys Ser Ala Gln Val Tyr Ala
   20 25 30
   Lys Pro Asp Met Arg Pro Leu Gly Pro Asn Ile Ala Asp Lys Gly Ser
   35 40 45
   Val Phe Tyr His Phe Ser Ala Thr Ser Phe Asp Ser Val Asp Gly Thr
   50 55 60
   Arg His Tyr Arg Val Trp Thr Ala Val Pro Asn Thr Thr Ala Pro Ala
   65 70 75 80
   Ser Gly Tyr Pro Ile Leu Tyr Met Leu Asp Gly Asn Ala Val Met Asp
   85 90 95
   Arg Leu Asp Asp Glu Leu Leu Lys Gln Leu Ser Glu Lys Thr Pro Pro
   100 105 110
   Val Ile Val Ala Val Gly Tyr Gln Thr Asn Leu Pro Phe Asp Leu Asn
   115 120 125
   Ser Arg Ala Tyr Asp Tyr Thr Pro Ala Ala Glu Ser Arg Lys Thr Asp
   130 135 140
   Leu His Ser Gly Arg Phe Ser Arg Lys Ser Gly Gly Ser Asn Asn Phe
   145 150 155 160
   Arg Gln Leu Leu Glu Thr Arg Ile Ala Pro Lys Val Glu Gln Gly Leu
   165 170 175
   Asn Ile Asp Arg Gln Arg Arg Gly Leu Trp Gly His Ser Tyr Gly Gly
   180 185 190
   Leu Phe Val Leu Asp Ser Trp Leu Ser Ser Ser Tyr Phe Arg Ser Tyr
   195 200 205
   Tyr Ser Ala Ser Pro Ser Leu Gly Arg Gly Tyr Asp Ala Leu Leu Ser
   210 215 220
   Arg Val Thr Ala Val Glu Pro Leu Gln Phe Cys Thr Lys His Leu Ala
   225 230 235 240
   Ile Met Glu Gly Ser Ala Thr Gln Gly Asp Asn Arg Glu Thr His Ala
   245 250 255
   Val Gly Val Leu Ser Lys Ile His Thr Thr Leu Thr Ile Leu Lys Asp
   260 265 270
   Lys Gly Val Asn Ala Val Phe Trp Asp Phe Pro Asn Leu Gly His Gly
   275 280 285
   Pro Met Phe Asn Ala Ser Phe Arg Gln Ala Leu Leu Asp Ile Ser Gly
   290 295 300
   Glu Asn Ala Asn Tyr Thr Ala Gly Cys His Glu Leu Ser His
   305 310 315
<210> 8
   <211> 725
   <212> PRT
   <213> Escherichia coli
<400> 8
   Met Arg Ile Asn Lys Ile Leu Trp Ser Leu Thr Val Leu Leu Val Gly
   1 5 10 15
   Leu Asn Ser Gln Val Ser Val Ala Lys Tyr Ser Asp Asp Asp Asn Asp
   20 25 30
   Glu Thr Leu Val Val Glu Ala Thr Ala Glu Gln Val Leu Lys Gln Gln
   35 40 45
   Pro Gly Val Ser Val Ile Thr Ser Glu Asp Ile Lys Lys Thr Pro Pro
   50 55 60
   Val Asn Asp Leu Ser Asp Ile Ile Arg Lys Met Pro Gly Val Asn Leu
   65 70 75 80
   Thr Gly Asn Ser Ala Ser Gly Thr Arg Gly Asn Asn Arg Gln Ile Asp
   85 90 95
   Ile Arg Gly Met Gly Pro Glu Asn Thr Leu Ile Leu Ile Asp Gly Val
   100 105 110
   Pro Val Thr Ser Arg Asn Ser Val Arg Tyr Ser Trp Arg Gly Glu Arg
   115 120 125
   Asp Thr Arg Gly Asp Thr Asn Trp Val Pro Pro Glu Gln Val Glu Arg
   130 135 140
   Ile Glu Val Ile Arg Gly Pro Ala Ala Ala Arg Tyr Gly Ser Gly Ala
   145 150 155 160
   Ala Gly Gly Val Val Asn Ile Ile Thr Lys Arg Pro Thr Asn Asp Trp
   165 170 175
   His Gly Ser Leu Ser Leu Tyr Thr Asn Gln Pro Glu Ser Ser Glu Glu
   180 185 190
   Gly Ala Thr Arg Arg Ala Asn Phe Ser Leu Ser Gly Pro Leu Ala Gly
   195 200 205
   Asp Ala Leu Thr Thr Arg Leu Tyr Gly Asn Leu Asn Lys Thr Asp Ala
   210 215 220
   Asp Ser Trp Asp Ile Asn Ser Pro Val Gly Thr Lys Asn Ala Ala Gly
   225 230 235 240
   His Glu Gly Val Arg Asn Lys Asp Ile Asn Gly Val Val Ser Trp Lys
   245 250 255
   Leu Asn Pro Gln Gln Ile Leu Asp Phe Glu Val Gly Tyr Ser Arg Gln
   260 265 270
   Gly Asn Ile Tyr Ala Gly Asp Thr Gln Asn Ser Ser Ser Ser Ala Val
   275 280 285
   Thr Glu Ser Leu Ala Lys Ser Gly Lys Glu Thr Asn Arg Leu Tyr Arg
   290 295 300
   Gln Asn Tyr Gly Ile Thr His Asn Gly Ile Trp Asp Trp Gly Gln Ser
   305 310 315 320
   Arg Phe Gly Val Tyr Tyr Glu Lys Thr Asn Asn Thr Arg Met Asn Glu
   325 330 335
   Gly Leu Ser Gly Gly Gly Glu Gly Arg Ile Leu Ala Gly Glu Lys Phe
   340 345 350
   Thr Thr Asn Arg Leu Ser Ser Trp Arg Thr Ser Gly Glu Leu Asn Ile
   355 360 365
   Pro Leu Asn Val Met Val Asp Gln Thr Leu Thr Val Gly Ala Glu Trp
   370 375 380
   Asn Arg Asp Lys Leu Asp Asp Pro Ser Ser Thr Ser Leu Thr Val Asn
   385 390 395 400
   Asp Arg Asp Ile Ser Gly Ile Ser Gly Ser Ala Ala Asp Arg Ser Ser
   405 410 415
   Lys Asn His Ser Gln Ile Ser Ala Leu Tyr Ile Glu Asp Asn Ile Glu
   420 425 430
   Pro Val Pro Gly Thr Asn Ile Ile Pro Gly Leu Arg Phe Asp Tyr Leu
   435 440 445
   Ser Asp Ser Gly Gly Asn Phe Ser Pro Ser Leu Asn Leu Ser Gln Glu
   450 455 460
   Leu Gly Asp Tyr Phe Lys Val Lys Ala Gly Val Ala Arg Thr Phe Lys
   465 470 475
   480
   Ala Pro Asn Leu Tyr Gln Ser Ser Glu Gly Tyr Leu Leu Tyr Ser
   Lys
   485 490 495
   Gly Asn Gly Cys Pro Lys Asp Ile Thr Ser Gly Gly Cys Tyr Leu
   Ile
   500 505 510
   Gly Asn Lys Asp Leu Asp Pro Glu Ile Ser Val Asn Lys Glu Ile
   Gly
   515 520 525
   Leu Glu Phe Thr Trp Glu Asp Tyr His Ala Ser Val Thr Tyr Phe
   Arg
   530 535 540
   Asn Asp Tyr Gln Asn Lys Ile Val Ala Gly Asp Asn Val Ile Gly
   Gln
   545 550 555
   560
   Thr Ala Ser Gly Ala Tyr Ile Leu Lys Trp Gln Asn Gly Gly Lys
   Ala
   565 570 575
   Leu Val Asp Gly Ile Glu Ala Ser Met Ser Phe Pro Leu Val Lys
   Glu
   580 585 590
   Arg Leu Asn Trp Asn Thr Asn Ala Thr Trp Met Ile Thr Ser Glu
   Gln
   595 600 605
   Lys Asp Thr Gly Asn Pro Leu Ser Val Ile Pro Lys Tyr Thr Ile
   Asn
   610 615 620
   Asn Ser Leu Asn Trp Thr Ile Thr Gln Ala Phe Ser Ala Ser Phe Asn
   625 630 635 640
   Trp Thr Leu Tyr Gly Arg Gln Lys Pro Arg Thr His Ala Glu Thr Arg
   645 650 655
   Ser Glu Asp Thr Gly Gly Leu Ser Gly Lys Glu Leu Gly Ala Tyr Ser
   660 665 670
   Leu Val Gly Thr Asn Phe Asn Tyr Asp Ile Asn Lys Asn Leu Arg Leu
   675 680 685
   Asn Val Gly Val Ser Asn Ile Leu Asn Lys Gln Ile Phe Arg Ser Ser
   690 695 700
   Glu Gly Ala Asn Thr Tyr Asn Glu Pro Gly Arg Ala Tyr Tyr Ala Gly
   705 710 715 720
   Val Thr Ala Ser Phe
   725
<210> 9
   <211> 1014
   <212> PRT
   <213> Escherichia coli
<400> 9
   Met Gly Asn Gln Trp Gln Gln Lys Tyr Leu Leu Glu Tyr Asn Glu Leu
   1 5 10 15
   Val Ser Asn Phe Pro Ser Pro Glu Arg Val Val Ser Asp Tyr Ile Lys
   20 25 30
   Asn Cys Phe Lys Thr Asp Leu Pro Trp Phe Ser Arg Ile Asp Pro Asp
   35 40 45
   Asn Ala Tyr Phe Ile Cys Phe Ser Gln Asn Arg Ser Asn Ser Arg Ser
   50 55 60
   Tyr Thr Gly Trp Asp His Leu Gly Lys Tyr Lys Thr Glu Val Leu Thr
   65 70 75 80
   Leu Thr Gln Ala Ala Leu Ile Asn Ile Gly Tyr Arg Phe Asp Val Phe
   85 90 95
   Asp Asp Ala Asn Ser Ser Thr Gly Ile Tyr Lys Thr Lys Ser Ala Asp
   100 105 110
   Val Phe Asn Glu Glu Asn Glu Glu Lys Met Leu Pro Ser Glu Tyr Leu
   115 120 125
   His Phe Leu Gln Lys Cys Asp Phe Ala Gly Val Tyr Gly Lys Thr Leu
   130 135 140
   Ser Asp Tyr Trp Ser Lys Tyr Tyr Asp Lys Phe Lys Leu Leu Leu Lys
   145 150 155 160
   Asn Tyr Tyr Ile Ser Ser Ala Leu Tyr Leu Tyr Lys Asn Gly Glu Leu
   165 170 175
   Asp Glu Arg Glu Tyr Asn Phe Ser Met Asn Ala Leu Asn Arg Ser Asp
   180 185 190
   Asn Ile Ser Leu Leu Phe Phe Asp Ile Tyr Gly Tyr Tyr Ala Ser Asp
   195 200 205
   Ile Phe Val Ala Lys Asn Asn Asp Lys Val Met Leu Phe Ile Pro Gly
   210 215 220
   Ala Lys Lys Pro Phe Leu Phe Lys Lys Asn Ile Ala Asp Leu Arg Leu
   225 230 235 240
   Thr Leu Lys Glu Leu Ile Lys Asp Ser Asp Asn Lys Gln Leu Leu Ser
   245 250 255
   Gln His Phe Ser Leu Tyr Ser Arg Gln Asp Gly Val Ser Tyr Ala Gly
   260 265 270
   Val Asn Ser Val Leu His Ala Ile Glu Asn Asp Gly Asn Phe Asn Glu
   275 280 285
   Ser Tyr Phe Leu Tyr Ser Asn Lys Thr Leu Ser Asn Lys Asp Val Phe
   290 295 300
   Asp Ala Ile Ala Ile Ser Val Lys Lys Arg Ser Phe Ser Asp Gly Asp
   305 310 315 320
   Ile Val Ile Lys Ser Asn Ser Glu Ala Gln Arg Asp Tyr Ala Leu Thr
   325 330 335
   Ile Leu Gln Thr Ile Leu Ser Met Thr Pro Ile Phe Asp Ile Val Val
   340 345 350
   Pro Glu Val Ser Val Pro Leu Gly Leu Gly Ile Ile Thr Ser Ser
   Met
   355 360 365
   Gly Ile Ser Phe Asp Gln Leu Ile Asn Gly Asp Thr Tyr Glu Glu
   Arg
   370 375 380
   Arg Ser Ala Ile Pro Gly Leu Ala Thr Asn Ala Val Leu Leu Gly
   Leu
   385 390 395
   400
   Ser Phe Ala Ile Pro Leu Leu Ile Ser Lys Ala Gly Ile Asn Gln
   Glu
   405 410 415
   Val Leu Ser Ser Val Ile Asn Asn Glu Gly Arg Thr Leu Asn Glu
   Thr
   420 425 430
   Asn Ile Asp Ile Phe Leu Lys Glu Tyr Gly Ile Ala Glu Asp Ser
   Ile
   435 440 445
   Ser Ser Thr Asn Leu Leu Asp Val Lys Leu Lys Ser Ser Gly Gln
   His
   450 455 460
   Val Asn Ile Val Lys Leu Ser Asp Glu Asp Asn Gln Ile Val Ala
   Val
   465 470 475
   480
   Lys Gly Ser Ser Leu Ser Gly Ile Tyr Tyr Glu Val Asp Ile Glu
   Thr
   485 490 495
   Gly Tyr Glu Ile Leu Ser Arg Arg Ile Tyr Arg Thr Glu Tyr Asn
   Asn
   500 505 510
   Glu Ile Leu Trp Thr Arg Gly Gly Gly Leu Lys Gly Gly Gln Pro Phe
   515 520 525
   Asp Phe Glu Ser Leu Asn Ile Pro Val Phe Phe Lys Asp Glu Pro Tyr
   530 535 540
   Ser Ala Val Thr Gly Ser Pro Leu Ser Phe Ile Asn Asp Asp Ser Ser
   545 550 555 560
   Leu Leu Tyr Pro Asp Thr Asn Pro Lys Leu Pro Gln Pro Thr Ser Glu
   565 570 575
   Met Asp Ile Val Asn Tyr Val Lys Gly Ser Gly Ser Phe Gly Asp Arg
   580 585 590
   Phe Val Thr Leu Met Arg Gly Ala Thr Glu Glu Glu Ala Trp Asn Ile
   595 600 605
   Ala Ser Tyr His Thr Ala Gly Gly Ser Thr Glu Glu Leu His Glu Ile
   610 615 620
   Leu Leu Gly Gln Gly Pro Gln Ser Ser Leu Gly Phe Thr Glu Tyr Thr
   625 630 635 640
   Ser Asn Val Asn Ser Ala Asp Ala Ala Ser Arg Arg His Phe Leu Val
   645 650 655
   Val Ile Lys Val His Val Lys Tyr Ile Thr Asn Asn Asn Val Ser Tyr
   660 665 670
   Val Asn His Trp Ala Ile Pro Asp Glu Ala Pro Val Glu Val Leu Ala
   675 680 685
   Val Val Asp Arg Arg Phe Asn Phe Pro Glu Pro Ser Thr Pro Pro Asp
   690 695 700
   Ile Ser Thr Ile Arg Lys Leu Leu Ser Leu Arg Tyr Phe Lys Glu Ser
   705 710 715 720
   Ile Glu Ser Thr Ser Lys Ser Asn Phe Gln Lys Leu Ser Arg Gly Asn
   725 730 735
   Ile Asp Val Leu Lys Gly Arg Gly Ser Ile Ser Ser Thr Arg Gln Arg
   740 745 750
   Ala Ile Tyr Pro Tyr Phe Glu Ala Ala Asn Ala Asp Glu Gln Gln Pro
   755 760 765
   Leu Phe Phe Tyr Ile Lys Lys Asp Arg Phe Asp Asn His Gly Tyr Asp
   770 775 780
   Gln Tyr Phe Tyr Asp Asn Thr Val Gly Leu Asn Gly Ile Pro Thr Leu
   785 790 795 800
   Asn Thr Tyr Thr Gly Glu Ile Pro Ser Asp Ser Ser Ser Leu Gly Ser
   805 810 815
   Thr Tyr Trp Lys Lys Tyr Asn Leu Thr Asn Glu Thr Ser Ile Ile
   Arg
   820 825 830
   Val Ser Asn Ser Ala Arg Gly Ala Asn Gly Ile Lys Ile Ala Leu
   Glu
   835 840 845
   Glu Val Gln Glu Gly Lys Pro Val Ile Ile Thr Ser Gly Asn Leu
   Ser
   850 855 860
   Gly Cys Thr Thr Ile Val Ala Arg Lys Glu Gly Tyr Ile Tyr Lys
   Val
   865 870 875
   880
   His Thr Gly Thr Thr Lys Ser Leu Ala Gly Phe Thr Ser Thr Thr
   Gly
   885 890 895
   Val Lys Lys Ala Val Glu Val Leu Glu Leu Leu Thr Lys Glu Pro
   Ile
   900 905 910
   Pro Arg Val Glu Gly Ile Met Ser Asn Asp Phe Leu Val Asp Tyr
   Leu
   915 920 925
   Ser Glu Asn Phe Glu Asp Ser Leu Ile Thr Tyr Ser Ser Ser Glu
   Lys
   930 935 940
   Lys Pro Asp Ser Gln Ile Thr Ile Ile Arg Asp Asn Val Ser Val
   Phe
   945 950 955
   960
   Pro Tyr Phe Leu Asp Asn Ile Pro Glu His Gly Phe Gly Thr Ser
   Ala
   965 970 975
   Thr Val Leu Val Arg Val Asp Gly Asn Val Val Val Arg Ser Leu Ser
   980 985 990
   Glu Ser Tyr Ser Leu Asn Ala Asp Ala Ser Glu Ile Ser Val Leu Lys
   995 1000 1005
   Val Phe Ser Lys Lys Phe
   1010
<210> 10
   <211> 454
   <212> PRT
   <213> Escherichia coli
<400> 10
   Met Val Asp Met Ile Asn Glu Ser Ala Arg Gln Thr Pro Val Ile Ala
   1 5 10 15
   Gln Thr Asp Val Leu Val Ile Gly Gly Gly Pro Ala Gly Leu Ser Ala
   20 25 30
   Ala Ile Ala Ala Gly Arg Leu Gly Ala Arg Thr Met Ile Val Glu Arg
   35 40 45
   Tyr Gly Ser Leu Gly Gly Val Leu Thr Gln Val Gly Val Glu Ser Phe
   50 55 60
   Ala Trp Tyr Arg His Pro Gly Thr Glu Asp Cys Glu Gly Ile Cys Arg
   65 70 75 80
   Glu Tyr Glu Gly Arg Ala Arg Ala Leu Gly Phe Thr Arg Pro Glu Pro
   85 90 95
   Gln Ser Ile Ser Glu Val Ile Asp Thr Glu Gly Phe Lys Val Val
   Ala
   100 105 110
   Asp Gln Met Ile Thr Glu Ser Gly Val Glu Pro Leu Tyr His Ser
   Trp
   115 120 125
   Val Val Asp Val Ile Lys Asp Gly Asp Thr Leu Cys Gly Val Ile
   Val
   130 135 140
   Glu Asn Lys Ser Gly Arg Gly Ala Ile Leu Ala Lys Arg Ile Val
   Asp
   145 150 155
   160
   Cys Thr Gly Asp Ala Asp Ile Ala Ala Arg Ala Gly Ala Pro Trp
   Thr
   165 170 175
   Lys Arg Ser Lys Asp Gln Leu Met Gly Val Thr Val Met Phe Ser
   Cys
   180 185 190
   Ala Gly Val Asp Val Ala Arg Phe Asn Arg Phe Val Ala Glu Glu
   Leu
   195 200 205
   Lys Pro Thr Tyr Ala Asp Trp Gly Lys Asn Trp Thr Ile Gln Thr
   Thr
   210 215 220
   Gly Lys Glu Asp Pro Met Phe Ser Pro Tyr Met Glu Asp Ile Phe
   Thr
   225 230 235
   240
   Arg Ala Gln Gln Asp Gly Val Ile Pro Gly Asp Ala Gln Ala Ile
   Ala
   245 250 255
   Gly Thr Trp Ser Thr Phe Ser Glu Ser Gly Glu Ala Phe Gln Met
   Asn
   260 265 270
   Met Val Tyr Ala Phe Gly Phe Asp Cys Thr Asp Val Phe Asp Leu
   Thr
   275 280 285
   Lys Ala Glu Ile Ala Gly Arg Gln Gln Ala Leu Trp Ala Ile Asp
   Ala
   290 295 300
   Leu Arg His Tyr Val Pro Gly Phe Glu Asn Val Arg Leu Arg Asn
   Phe
   305 310 315
   320
   Gly Ala Thr Leu Gly Thr Arg Glu Ser Arg Leu Ile Glu Gly Glu
   Ile
   325 330 335
   Arg Ile Ala Asp Asp Tyr Val Leu Asn Gln Gly Arg Cys Ser Asp
   Ser
   340 345 350
   Val Gly Ile Phe Pro Glu Phe Ile Asp Gly Ser Gly Tyr Leu Ile
   Leu
   355 360 365
   Pro Thr Thr Gly Arg Phe Phe Gln Ile Pro Tyr Gly Cys Leu Val
   Pro
   370 375 380
   Gln Lys Val Glu Asn Leu Leu Val Ala Gly Arg Cys Ile Ser Ala
   Gly
   385 390 395
   400
   Val Val Ala His Thr Ser Met Arg Asn Met Met Cys Cys Ala Val
   Thr
   405 410 415
   Gly Glu Ala Ala Gly Thr Ala Ala Val Val Ser Leu Gln Gln Asn Cys
   420 425 430
   Thr Val Arg Gln Val Ala Ile Pro Asp Leu Gln Asn Thr Leu Gln Gln
   435 440 445
   Gln Gly Val Arg Leu Ala
   450
<210> 11
   <211> 253
   <212> PRT
   <213> Escherichia coli
<400> 11
   Met Ser Ala Lys Arg Arg Leu Leu Ile Ala Cys Thr Leu Ile Thr Ala
   1 5 10 15
   Ile Tyr His Phe Pro Ala Tyr Ser Ser Leu Glu Tyr Lys Gly Thr Phe
   20 25 30
   Gly Ser Ile Asn Ala Gly Tyr Ala Asp Trp Asn Ser Gly Phe Val Asn
   35 40 45
   Thr His Arg Gly Glu Val Trp Lys Val Thr Ala Asp Phe Gly Val Asn
   50 55 60
   Phe Lys Glu Ala Glu Phe Tyr Ser Phe Tyr Glu Ser Asn Val Leu Asn
   65 70 75 80
   His Ala Val Ala Gly Arg Asn His Thr Val Ser Ala Met Thr His Val
   85 90 95
   Arg Leu Phe Asp Ser Asp Met Thr Phe Phe Gly Lys Ile Tyr Gly
   Gln
   100 105 110
   Trp Asp Asn Ser Trp Gly Asp Asp Leu Asp Met Phe Tyr Gly Phe
   Gly
   115 120 125
   Tyr Leu Gly Trp Asn Gly Glu Trp Gly Phe Phe Lys Pro Tyr Ile
   Gly
   130 135 140
   Leu His Asn Gln Ser Gly Asp Tyr Val Ser Ala Lys Tyr Gly Gln
   Thr
   145 150 155
   160
   Asn Gly Trp Asn Gly Tyr Val Val Gly Trp Thr Ala Val Leu Pro
   Phe
   165 170 175
   Thr Leu Phe Asp Glu Lys Phe Val Leu Ser Asn Trp Asn Glu Ile
   Glu
   180 185 190
   Leu Asp Arg Asn Asp Ala Tyr Thr Glu Gln Gln Phe Gly Arg Asn
   Gly
   195 200 205
   Leu Asn Gly Gly Leu Thr Ile Ala Trp Lys Phe Tyr Pro Arg Trp
   Lys
   210 215 220
   Ala Ser Val Thr Trp Arg Tyr Phe Asp Asn Lys Leu Gly Tyr Asp
   Gly
   225 230 235
   240
   Phe Gly Asp Gln Met Ile Tyr Met Leu Gly Tyr Asp Phe
   245 250
<210> 12
   <211> 492
   <212> PRT
   <213> Escherichia coli
<400> 12
   Met Ala Ser Leu Ile Gly Leu Ala Val Cys Thr Gly Asn Ala Phe Ser
   1 5 10 15
   Pro Ala Leu Ala Ala Glu Ala Lys Gln Pro Asn Leu Val Ile Ile Met
   20 25 30
   Ala Asp Asp Leu Gly Tyr Gly Asp Leu Ala Thr Tyr Gly His Gln Ile
   35 40 45
   Val Lys Thr Pro Asn Ile Asp Arg Leu Ala Gln Glu Gly Val Lys Phe
   50 55 60
   Thr Asp Tyr Tyr Ala Pro Ala Pro Leu Ser Ser Pro Ser Arg Ala Gly
   65 70 75 80
   Leu Leu Thr Gly Arg Met Pro Phe Arg Thr Gly Ile Arg Ser Trp Ile
   85 90 95
   Pro Ser Gly Lys Asp Val Ala Leu Gly Arg Asn Glu Leu Thr Ile Ala
   100 105 110
   Asn Leu Leu Lys Ala Gln Gly Tyr Asp Thr Ala Met Met Gly Lys Leu
   115 120 125
   His Leu Asn Ala Gly Gly Asp Arg Thr Asp Gln Pro Gln Ala Gln Asp
   130 135 140
   Met Gly Phe Asp Tyr Ser Leu Ala Asn Thr Ala Gly Phe Val Thr Asp
   145 150 155 160
   Ala Thr Leu Asp Asn Ala Lys Glu Arg Pro Arg Tyr Gly Met Val Tyr
   165 170 175
   Pro Thr Gly Trp Leu Arg Asn Gly Gln Pro Thr Pro Arg Ala Asp Lys
   180 185 190
   Met Ser Gly Glu Tyr Val Ser Ser Glu Val Val Asn Trp Leu Asp Asn
   195 200 205
   Lys Lys Asp Ser Lys Pro Phe Phe Leu Tyr Val Ala Phe Thr Glu Val
   210 215 220
   His Ser Pro Leu Ala Ser Pro Lys Lys Tyr Leu Asp Met Tyr Ser Gln
   225 230 235 240
   Tyr Met Ser Ala Tyr Gln Lys Gln His Pro Asp Leu Phe Tyr Gly Asp
   245 250 255
   Trp Ala Asp Lys Pro Trp Arg Gly Val Gly Glu Tyr Tyr Ala Asn Ile
   260 265 270
   Ser Tyr Leu Asp Ala Gln Val Gly Lys Val Leu Asp Lys Ile Lys Ala
   275 280 285
   Met Gly Glu Glu Asp Asn Thr Ile Val Ile Phe Thr Ser Asp Asn Gly
   290 295 300
   Pro Val Thr Arg Glu Ala Arg Lys Val Tyr Glu Leu Asn Leu Ala Gly
   305 310 315 320
   Glu Thr Asp Gly Leu Arg Gly Arg Lys Asp Asn Leu Trp Glu Gly Gly
   325 330 335
   Ile Arg Val Pro Ala Ile Ile Lys Tyr Gly Lys His Leu Pro Gln Gly
   340 345 350
   Met Val Ser Asp Thr Pro Val Tyr Gly Leu Asp Trp Met Pro Thr Leu
   355 360 365
   Ala Lys Met Met Asn Phe Lys Leu Pro Thr Asp Arg Thr Phe Asp Gly
   370 375 380
   Glu Ser Leu Val Pro Val Leu Glu Gln Lys Ala Leu Lys Arg Glu Lys
   385 390 395 400
   Pro Leu Ile Phe Gly Ile Asp Met Pro Phe Gln Asp Asp Pro Thr Asp
   405 410 415
   Glu Trp Ala Ile Arg Asp Gly Asp Trp Lys Met Ile Ile Asp Arg Asn
   420 425 430
   Asn Lys Pro Lys Tyr Leu Tyr Asn Leu Lys Ser Asp Arg Tyr Glu Thr
   435 440 445
   Leu Asn Leu Ile Gly Lys Lys Pro Asp Ile Glu Lys Gln Met Tyr Gly
   450 455 460
   Lys Phe Leu Lys Tyr Lys Thr Asp Ile Asp Asn Asp Ser Leu Met Lys
   465 470 475 480
   Ala Arg Gly Asp Lys Pro Glu Ala Val Thr Trp Gly
   485 490
<210> 13
   <211> 345
   <212> PRT
   <213> Escherichia coli
<400> 13
   Leu Ile Ser Leu Ser Phe Ile Pro Val Met Ser Ala Leu Pro Gly Pro
   1 5 10 15
   Ile Ala Lys Gly Phe Arg Asn Glu Arg Gly Phe Val Thr Thr Thr Ile
   20 25 30
   Cys Ala Met Gly Glu Leu Leu Ala Glu Phe Leu Ser Arg Asn Pro His
   35 40 45
   Gln Lys Phe Thr Gln Pro Gly Glu Phe Ile Gly Pro Phe Pro Ser Gly
   50 55 60
   Ala Pro Ala Ile Phe Ala Ala Gln Val Ala Lys Leu Ser His Arg Ala
   65 70 75 80
   Ile Phe Phe Gly Cys Val Gly Asn Asp Asp Phe Ala Arg Leu Ile Ile
   85 90 95
   Glu Arg Leu Arg His Glu Gly Val Ile Thr Asp Gly Ile His Val
   Met
   100 105 110
   Asn Asn Ala Val Thr Gly Thr Ala Phe Val Ser Tyr Gln Asn Pro
   Gln
   115 120 125
   Gln Arg Asp Phe Val Phe Asn Ile Pro Asn Ser Ala Cys Gly Leu
   Phe
   130 135 140
   Thr Ala Glu His Ile Asp Lys Asp Leu Leu Lys Gln Cys Asn His
   Leu
   145 150 155
   160
   His Ile Val Gly Ser Ser Leu Phe Ser Phe Arg Met Ile Asp Val
   Met
   165 170 175
   Arg Lys Ala Ile Thr Thr Ile Lys Ser Ala Gly Gly Thr Val Ser
   Phe
   180 185 190
   Asp Pro Asn Ile Arg Lys Glu Met Leu Ser Ile Pro Glu Met Ala
   Gln
   195 200 205
   Ala Leu Asp Tyr Leu Ile Glu Tyr Thr Asp Ile Phe Ile Pro Ser
   Glu
   210 215 220
   Ser Glu Leu Pro Phe Phe Ala Arg His Lys Asn Leu Ser Glu Glu
   Gln
   225 230 235
   240
   Ile Val Ser Asp Leu Leu His Gly Gly Val Lys His Val Ala Ile
   Lys
   245 250 255
   Arg Ala Gln Arg Gly Ala Ser Tyr Tyr Lys Leu Lys Asn Gly Thr Leu
   260 265 270
   His Ala Gln His Val Ala Gly His Asp Ile Glu Ile Ile Asp Pro Thr
   275 280 285
   Gly Ala Gly Asp Cys Phe Gly Ala Thr Phe Ile Thr Leu Phe Leu Ser
   290 295 300
   Gly Phe Pro Ala His Lys Ala Leu Gln Tyr Ala Asn Ala Ser Gly Ala
   305 310 315 320
   Leu Ala Val Met Arg Gln Gly Pro Met Glu Gly Ile Ser Ser Leu Ala
   325 330 335
   Asp Ile Glu Asp Phe Leu Gln Gln His
   340 345
<210> 14
   <211> 192
   <212> PRT
   <213> Escherichia coli
<400> 14
   Met Tyr Met Pro Gly Lys Gln Met Leu Cys Cys Ile Leu Ile Ser Ile
   1 5 10 15
   Ile Ser Glu Gly Asp Met Lys Ile Phe Ile Ser Leu Phe Leu Phe Ile
   20 25 30
   Ile Ser Thr Asn Ser Phe Ala Asp Asp Ile Thr His Ala Gly Val Val
   35 40 45
   Arg Ile Glu Gly Leu Ile Thr Glu Lys Thr Cys Ile Ile Ser Asp Glu
   50 55 60
   Ser Lys Asn Phe Thr Val Asn Met Pro Asp Val Pro Ser Ser Ser Val
   65 70 75 80
   Arg Ser Ala Gly Asp Val Thr Glu Lys Val Tyr Phe Ser Ile Thr Leu
   85 90 95
   Thr Arg Cys Gly Ser Asp Val Gly Asn Ala Tyr Ile Lys Phe Thr Gly
   100 105 110
   Asn Thr Val Ser Glu Asp Ala Ser Leu Tyr Lys Leu Glu Asp Gly Ser
   115 120 125
   Val Glu Gly Leu Ala Leu Thr Ile Phe Asp Lys Asn Lys Gly Ser Ile
   130 135 140
   Ser Asn Asp Val Lys Ser Met Val Phe Ser Leu Thr Ser Ser Val Asp
   145 150 155 160
   Asn Ile Leu His Phe Phe Ala Ala Tyr Lys Ala Leu Lys Asn Asn Val
   165 170 175
   Gln Pro Gly Asp Ala Asn Ala Ser Val Ser Phe Ile Val Thr Tyr Asp
   180 185 190
<210> 15
   <211> 201
   <212> PRT
   <213> Escherichia coli
<400> 15
   Met Ile Lys Phe Arg Leu Tyr Ile Pro Pro Val Ile Leu Gly Phe Val
   1 5 10 15
   Ile Val Pro Leu Leu Val Trp Pro Thr Val Ile Ala Leu Ala Val Leu
   20 25 30
   Ile Phe Thr Leu Thr Phe Leu Ala Glu Ile Ile Phe Ser Phe Pro Leu
   35 40 45
   Leu Val Val Arg Ile Ser Leu Gln Glu Leu Gln Leu Glu Leu Leu Val
   50 55 60
   Val Tyr Ala Leu Phe Phe Ser Val Met Gly Gly Ile Gly Trp Gln Phe
   65 70 75 80
   Ser Arg Arg Thr Pro Pro Glu Leu Lys Asn Arg Leu His Cys Trp Leu
   85 90 95
   Val Phe Ser Pro Val Tyr Phe Trp Leu Ile Leu Ser Asn Phe Ile Leu
   100 105 110
   Tyr Ile Ser Pro Glu Lys Ser Ala Leu Leu Glu Asn Ile Arg Asn Phe
   115 120 125
   Phe Leu Thr Phe Val Trp Leu Pro Leu Asn Phe Ser Pro Phe Trp Pro
   130 135 140
   Gln Pro Trp Thr Asp Phe Val Gly Pro Ile Ser Ala Gln Leu Gly Phe
   145 150 155 160
   Ala Leu Gly Tyr Tyr Cys Gln Trp Arg Ser Lys Asn Arg Ser His Arg
   165 170 175
   Lys Lys Trp Gly Asp Trp Val Thr Cys Leu Ser Leu Ala Ile Leu Ala
   180 185 190
   Leu Gly Pro Leu Phe Asn Tyr Leu Gln
   195 200
<210> 16
   <211> 234
   <212> PRT
   <213> Escherichia coli
<400> 16
   Met Lys Phe Asn Leu Ser Asn Leu Ser Ala Val Leu Leu Ala Ser Gly
   1 5 10 15
   Met Leu Met Ser Thr Ala Val Thr Ala Ala Pro Gly Asp Ala Thr Gln
   20 25 30
   Phe Gly Gly Ala Asp Thr Asp Trp Ser Thr Val Asp Tyr Pro Arg Leu
   35 40 45
   Thr Asp Met Asp Asp Asn Val Asp Ser Met Gly Gly Lys Ile Arg Phe
   50 55 60
   Thr Gly Arg Val Val Lys Ala Thr Cys Lys Val Ala Thr Asp Ser Lys
   65 70 75 80
   Gln Ile Glu Val Val Leu Pro Val Val Pro Ser Asn Leu Phe Thr Gly
   85 90 95
   Ile Asp Val Glu Ala Gln Gly Ala Ser Asn Gln Thr Asp Phe Asn Ile
   100 105 110
   Asn Leu Thr Glu Cys Ser Asn Thr Asp Asp Gln Lys Ile Glu Phe Arg
   115 120 125
   Phe Thr Gly Thr Ala Asp Ser Ala Asn Lys Thr Leu Ala Asn Glu Val
   130 135 140
   Glu Gly Ser Thr Asp Ala Asp Asn Ser Gly Asn Ala Gly Ala Thr Gly
   145 150 155 160
   Val Gly Ile Arg Ile Tyr Ser Lys Gly Thr Thr Asn Asn Gly Leu Ile
   165 170 175
   Asn Leu Asn Thr Thr Ala Ala Glu Gly Ser Ala Ser Thr Ala Ala Tyr
   180 185 190
   Thr Ile Pro Gly Asn Ala Thr Thr His Asp Phe Ser Ala Ala Phe Thr
   195 200 205
   Ala Gly Tyr Ala Gln Asn Gly Ser Thr Val Ala Pro Gly Val Val Lys
   210 215 220
   Ser Thr Ala Ser Phe Val Val Leu Tyr Glu
   225 230
<210> 17
   <211> 336
   <212> PRT
   <213> Escherichia coli
<400> 17
   Met Arg Ile His Thr Tyr Trp Tyr Arg Arg Tyr Phe Ile Leu Leu Ile
   1 5 10 15
   Ile Ile Phe Ser Asn Val Leu Ser Ser Ile Ala Asn Ala Glu Asp Met
   20 25 30
   Gly Arg Glu Arg Ala Tyr Cys Tyr Pro Gly Ser Pro Ser Asn Asn Thr
   35 40 45
   Thr Pro Ala Ser Phe Ser Tyr Asn Phe Gly Thr Ile Val Val Ser Asp
   50 55 60
   Val Asn Lys Asn Ala Pro Gly Thr Val Leu Pro Ser Gln Ile Trp Lys
   65 70 75 80
   Val Gly Thr Tyr Lys Ala Tyr Cys Asn Ser Leu Asp Asp Tyr Glu Ile
   85 90 95
   Tyr Phe Ser Ala Val Ser Gly Ile Asp Pro Ser Gly Ala Ser Gly Asp
   100 105 110
   His Gln Gly Ser Asp Val Phe Ile Pro Leu Thr His Glu Ile Ser Val
   115 120 125
   Ser Thr His Ile Lys Leu Tyr Asn Gln Asn Gly Thr Met Thr Asp Lys
   130 135 140
   Ile Val Pro Phe Glu Asn Tyr Asn Thr Asn Tyr Pro Gly Asp Arg Ser
   145 150 155 160
   Lys Pro Ser Asn Trp Ala Ser Gly Thr Glu Gly Tyr Ile Lys Ile Arg
   165 170 175
   Ile Asp Lys Lys Ile Ile Ser Asp Val Ser Leu Ser Asn Val Leu Leu
   180 185 190
   Val Ser Leu Tyr Val Ser Gln Ile Pro Thr Glu His Gly Pro Ile Pro
   195 200 205
   Val Phe Asn Ala Tyr Ile Gly Asn Leu Asn Ile Gln Val Pro Gln Gly
   210 215 220
   Cys Thr Ile Asn Glu Gly Thr Ser Phe Thr Val Asn Met Pro Asp Val
   225 230 235 240
   Trp Ala Ser Glu Leu Ser Arg Ala Gly Ala Gly Ala Lys Pro Ala Gly
   245 250 255
   Val Thr Pro Val Ala Thr Thr Ile Pro Ile Asn Cys Thr Asn Lys Asp
   260 265 270
   Thr Asp Ala Val Met Thr Leu Val Phe Asp Gly Asn Ile Ser Ala Thr
   275 280 285
   Arg Asp Thr Asn Gly Lys Gln Ser Ile Ile Gln Ala Gln Asp Asn Pro
   290 295 300
   Asp Val Gly Ile Met Ile Met Asp Ser Gln Gln Asn Ser Val Asp Leu
   305 310 315 320
   Asn Ala Leu Ala Thr Ser Val Gly Val Pro Phe Arg Leu Val Glu Asn
   325 330 335
<210> 18
   <211> 864
   <212> PRT
   <213> Escherichia coli
<400> 18
   Met Asn Leu Lys Leu Lys Arg Cys Glu Tyr Trp Met Ala Ala Gln
   Lys
   1 5 10 15
   Gln Met Lys Arg Val Val Pro Leu Leu Leu Val Ile Met Pro Ala
   Cys
   20 25 30
   Ser Ile Ala Gly Met Arg Phe Asn Pro Ala Phe Leu Ser Gly Asp
   Thr
   35 40 45
   Glu Ala Val Ala Asp Leu Ser Arg Phe Glu Lys Gly Met Thr Tyr
   Leu
   50 55 60
   Pro Gly Ser Tyr Glu Val Glu Val Trp Val Asn Asp Ser Pro Leu
   Leu
   65 70 75 80
   Ser Arg Thr Val Thr Phe Lys Ala Asp Asp Glu Asn Gln Leu Ile
   Pro
   85 90 95
   Cys Leu Ser Leu Ala Asp Leu Leu Ser Leu Gly Ile Asn Lys Asn
   Ala
   100 105 110
   Leu Pro Glu Gln Ala Leu Ala Ser Ser Glu Asn Ser Cys Leu Asp
   Leu
   115 120 125
   Arg Ile Trp Phe Pro Asp Val His Tyr Met Pro Glu Leu Asp Ala Gln
   130 135 140
   Arg Leu Lys Leu Thr Phe Pro Gln Ala Ile Ile Lys Arg Asp Ala Arg
   145 150 155 160
   Gly Tyr Ile Pro Pro Glu Gln Trp Asp Asn Gly Ile Thr Ala Phe Leu
   165 170 175
   Leu Asn Tyr Asp Phe Ser Gly Asn Asn Asp Arg Gly Asp Tyr Ser Ser
   180 185 190
   Asn Asn Tyr Tyr Leu Asn Leu Arg Ala Gly Ile Asn Ile Gly Ala Trp
   195 200 205
   Arg Phe Arg Asp Tyr Ser Thr Trp Ser Arg Gly Ser Asn Ser Ala Gly
   210 215 220
   Lys Leu Glu His Ile Ser Ser Thr Leu Gln Arg Val Ile Ile Pro Phe
   225 230 235 240
   Arg Ser Glu Leu Thr Leu Gly Asp Thr Trp Ser Ser Ser Asp Val Phe
   245 250 255
   Asp Ser Val Ser Ile Arg Gly Ile Lys Leu Glu Ser Asp Glu Asn Met
   260 265 270
   Leu Pro Asp Ser Gln Ser Gly Phe Ala Pro Thr Val Arg Gly Ile Ala
   275 280 285
   Lys Ser Arg Ala Gln Val Thr Ile Lys Gln Asn Gly Tyr Val Ile Tyr
   290 295 300
   Gln Thr Tyr Met Pro Pro Gly Pro Phe Glu Ile Ser Asp Leu Asn Pro
   305 310 315 320
   Thr Ser Ser Ala Gly Asp Leu Glu Val Thr Ile Lys Glu Ser Asp Asn
   325 330 335
   Ser Glu Thr Val Tyr Thr Val Pro Tyr Ala Ala Val Pro Ile Leu Gln
   340 345 350
   Arg Glu Gly His Leu Lys Tyr Ser Thr Thr Val Gly Gln Tyr Arg Ser
   355 360 365
   Asn Ser Tyr Asn Gln Lys Ser Pro Tyr Val Phe Gln Gly Glu Leu Ile
   370 375 380
   Trp Gly Leu Pro Trp Asp Ile Thr Ala Tyr Gly Gly Ala Gln Phe Ser
   385 390 395 400
   Glu Asp Tyr Arg Ala Leu Ala Leu Gly Leu Gly Leu Asn Leu Gly Val
   405 410 415
   Phe Gly Ala Thr Ser Phe Asp Val Thr Gln Ala Asn Ser Ser Leu Val
   420 425 430
   Asp Gly Ser Lys His Gln Gly Gln Ser Tyr Arg Phe Leu Tyr Ser Lys
   435 440 445
   Ser Leu Val Gln Thr Gly Thr Ala Phe His Ile Ile Gly Tyr Arg Tyr
   450 455 460
   Ser Thr Gln Gly Phe Tyr Thr Leu Ser Asp Thr Thr Tyr Gln Gln Met
   465 470 475 480
   Ser Gly Thr Val Val Asp Pro Lys Thr Leu Asp Asp Lys Asp Tyr Val
   485 490 495
   Tyr Asn Trp Asn Asp Phe Tyr Asn Leu Arg Tyr Ser Lys Arg Gly Lys
   500 505 510
   Phe Gln Ala Ser Val Ser Gln Pro Phe Gly Asn Tyr Gly Ser Met Tyr
   515 520 525
   Leu Ser Ala Ser Gln Gln Thr Tyr Trp Asn Thr Asp Lys Lys Asp Ser
   530 535 540
   Leu Tyr Gln Val Gly Tyr Asn Thr Ser Ile Lys Gly Ile Tyr Leu Asn
   545 550 555 560
   Val Ala Trp Asn Tyr Ser Lys Ser Pro Gly Thr Asn Ala Asp Lys Ile
   565 570 575
   Val Ser Leu Asn Val Ser Leu Pro Ile Ser Asn Trp Leu Ser Ser Thr
   580 585 590
   Asn Asp Gly Arg Ser Ser Ser Asn Ala Met Thr Ala Thr Tyr Gly Tyr
   595 600 605
   Ser Gln Asp Asn His Gly Gln Val Asn Gln Tyr Thr Gly Val Ser Gly
   610 615 620
   Ser Leu Leu Glu Gln His Asn Leu Ser Tyr Asn Ile Gln His Gly Phe
   625 630 635 640
   Ala Asn Gln Asp Asn Ser Ser Ser Gly Ser Val Gly Val Asn Tyr Arg
   645 650 655
   Gly Ala Tyr Gly Ser Leu Asn Ser Ala Tyr Ser Tyr Asp Asn Glu Gly
   660 665 670
   Asn Gln Gln Ile Asn Tyr Gly Ile Ser Gly Ala Leu Val Val His Glu
   675 680 685
   Asn Gly Leu Thr Leu Ser Gln Pro Leu Gly Glu Thr Asn Val Leu Ile
   690 695 700
   Lys Ala Pro Gly Ala Asn Asn Val Asp Val Gln Arg Gly Thr Gly Ile
   705 710 715 720
   Ser Thr Asp Trp Arg Gly Tyr Ala Val Val Pro Tyr Ala Thr Glu Tyr
   725 730 735
   Arg Arg Asn Asn Ile Ser Leu Asp Pro Met Ser Met Asn Met His Thr
   740 745 750
   Glu Leu Asp Ile Thr Ser Thr Glu Val Ile Pro Gly Lys Gly Ala
   Leu
           755                 760                 765
   Val Arg Ala Glu Phe Ala Ala His Ile Gly Ile Arg Gly Leu Phe
   Thr
       770                 775                 780
   Val Arg Tyr Arg Asn Lys Ser Val Pro Phe Gly Ala Thr Ala Ser
   Ala
   785                 790                 795
   800
   Gln Ile Lys Asn Ser Ser Gln Ile Thr Gly Ile Val Gly Asp Asn
   Gly
                   805                 810                 815
   Gln Leu Tyr Leu Ser Gly Leu Pro Leu Glu Gly Val Ile Asn Ile
   Gln
               820                 825                 830
   Trp Gly Asp Gly Val Gln Gln Lys Cys Gln Ala Asn Tyr Lys Leu
   Pro
           835                 840                 845
   Glu Thr Glu Leu Asp Asn Pro Val Ser Tyr Ala Thr Leu Glu Cys
   Arg
       850                 855                 860
<210> 19 <211> 169 <212> PRT <213> Escherichia coli <400> 19
<210> 20
   <211> 713
   <212> PRT
   <213> Escherichia coli
<400> 20
   Met Ala Met Phe Thr Pro Ser Phe Ser Gly Leu Lys Gly Arg Ala Leu
   1 5 10 15
   Phe Ser Leu Leu Phe Ala Ala Pro Met Ile His Ala Thr Asp Ser Val
   20 25 30
   Thr Thr Lys Asp Gly Glu Thr Ile Thr Val Thr Ala Asp Ala Asn Thr
   35 40 45
   Ala Thr Glu Ala Thr Asp Gly Tyr Gln Pro Leu Ser Thr Ser Thr Ala
   50 55 60
   Thr Leu Thr Asp Met Pro Met Leu Asp Ile Pro Gln Val Val Asn Thr
   65 70 75 80
   Val Ser Asp Gln Val Leu Glu Asn Gln Asn Ala Thr Thr Leu Asp Glu
   85 90 95
   Ala Leu Tyr Asn Val Ser Asn Val Val Gln Thr Asn Thr Leu Gly Gly
   100 105 110
   Thr Gln Asp Ala Phe Val Arg Arg Gly Phe Gly Ala Asn Arg Asp Gly
   115 120 125
   Ser Ile Met Thr Asn Gly Leu Arg Thr Val Leu Pro Arg Ser Phe Asn
   130 135 140
   Ala Ala Thr Glu Arg Val Glu Val Leu Lys Gly Pro Ala Ser Thr Leu
   145 150 155 160
   Tyr Gly Ile Leu Asp Pro Gly Gly Leu Ile Asn Val Val Thr Lys Arg
   165 170 175
   Pro Glu Lys Thr Phe His Gly Ser Val Ser Ala Thr Ser Ser Ser Phe
   180 185 190
   Gly Gly Gly Thr Gly Gln Leu Asp Ile Thr Gly Pro Ile Glu Gly Thr
   195 200 205
   Gln Leu Ala Tyr Arg Leu Thr Gly Glu Val Gln Asp Glu Asp Tyr Trp
   210 215 220
   Arg Asn Phe Gly Lys Glu Arg Ser Thr Phe Ile Ala Pro Ser Leu Thr
   225 230 235 240
   Trp Phe Gly Asp Asn Ala Thr Val Thr Met Leu Tyr Ser His Arg Asp
   245 250 255
   Tyr Lys Thr Pro Phe Asp Arg Gly Thr Ile Phe Asp Leu Thr Thr Lys
   260 265 270
   Gln Pro Val Asn Val Asp Arg Lys Ile Arg Phe Asp Glu Pro Phe Asn
   275 280 285
   Ile Thr Asp Gly Gln Ser Asp Leu Ala Gln Leu Asn Ala Glu Tyr His
   290 295 300
   Leu Asn Ser Gln Trp Thr Ala Arg Phe Asp Tyr Ser Tyr Ser Gln Asp
   305 310 315 320
   Lys Tyr Ser Asp Asn Gln Ala Arg Val Thr Ala Tyr Asp Ala Thr
   Thr
   325 330 335
   Gly Thr Leu Thr Arg Arg Val Asp Ala Thr Gln Gly Ser Thr Gln
   Arg
   340 345 350
   Met His Ala Thr Arg Ala Asp Leu Gln Gly Asn Val Asp Ile Ala
   Gly
   355 360 365
   Phe Tyr Asn Glu Ile Leu Gly Gly Val Ser Tyr Glu Tyr Tyr Asp
   Leu
   370 375 380
   Leu Arg Thr Asp Met Ile Arg Cys Lys Lys Ala Lys Asp Phe Asn
   Ile
   385 390 395
   400
   Tyr Asn Pro Val Tyr Gly Asn Thr Ser Lys Cys Thr Thr Val Ser
   Ala
   405 410 415
   Ser Asp Ser Asp Gln Thr Ile Lys Gln Glu Asn Tyr Ser Ala Tyr
   Ala
   420 425 430
   Gln Asp Ala Leu Tyr Leu Thr Asp Asn Trp Ile Ala Val Ala Gly
   Ile
   435 440 445
   Arg Tyr Gln Tyr Tyr Thr Gln Tyr Ala Gly Lys Gly Arg Pro Phe
   Asn
   450 455 460
   Val Asn Thr Asp Ser Arg Asp Glu Gln Trp Thr Pro Lys Leu Gly
   Leu
   465 470 475
   480
   Val Tyr Lys Leu Thr Pro Ser Val Ser Leu Phe Ala Asn Tyr Ser Gln
   485 490 495
   Thr Phe Met Pro Gln Ser Ser Ile Ala Ser Tyr Ile Gly Asp Leu Pro
   500 505 510
   Pro Glu Ser Ser Asn Ala Tyr Glu Val Gly Ala Lys Phe Glu Leu Phe
   515 520 525
   Asp Gly Ile Thr Ala Asp Ile Ala Leu Phe Asp Ile His Lys Arg Asn
   530 535 540
   Val Leu Tyr Thr Glu Ser Ile Gly Asp Glu Thr Ile Ala Lys Thr Ala
   545 550 555 560
   Gly Arg Val Arg Ser Arg Gly Val Glu Val Asp Leu Ala Gly Ala Leu
   565 570 575
   Thr Glu Asn Ile Asn Ile Ile Ala Ser Tyr Gly Tyr Thr Asp Ala Lys
   580 585 590
   Val Leu Glu Asp Pro Asp Tyr Ala Gly Lys Pro Leu Pro Asn Val Pro
   595 600 605
   Arg His Thr Gly Ser Leu Phe Leu Thr Tyr Asp Ile His Asn Met Pro
   610 615 620
   Gly Asn Asn Thr Leu Thr Phe Gly Gly Gly Gly His Gly Val Ser Arg
   625 630 635 640
   Arg Ser Ala Thr Asn Gly Ala Asp Tyr Tyr Leu Pro Gly Tyr Phe Val
   645 650 655
   Ala Asp Ala Phe Ala Ala Tyr Lys Met Lys Leu Gln Tyr Pro Val Thr
   660 665 670
   Leu Gln Leu Asn Val Lys Asn Leu Phe Asp Lys Thr Tyr Tyr Thr Ser
   675 680 685
   Ser Ile Ala Thr Asn Asn Leu Gly Asn Gln Ile Gly Asp Pro Arg Glu
   690 695 700
   Val Gln Phe Thr Val Lys Met Glu Phe
   705 710
<210> 21 <211> 606 <212> PRT <213> Escherichia coli <400> 21
<210> 22 <211> 101 <212> PRT <213> Escherichia coli <400> 22
<210> 23 <211> 263 <212> PRT <213> Escherichia coli <400> 23
<210> 24 <211> 378 <212> PRT <213> Escherichia coli <400> 24
<210> 25 <211> 654 <212> PRT <213> Escherichia coli <400> 25
<210> 26 <211> 1376 <212> PRT <213> Escherichia coli <400> 26
<210> 27 <211> 349 <212> PRT <213> Escherichia coli <400> 27
<210> 28 <211> 840 <212> PRT <213> Escherichia coli <400> 28
<210> 29 <211> 169 <212> PRT <213> Escherichia coli <400> 29
<210> 30 <211> 311 <212> PRT <213> Escherichia coli <400> 30
<210> 31 <211> 722 <212> PRT <213> Escherichia coli <400> 31
<210> 32 <211> 319 <212> PRT <213> Escherichia coli <400> 32
<210> 33 <211> 629 <212> PRT <213> Escherichia coli <400> 33
<210> 34 <211> 1778 <212> PRT <213> Escherichia coli
<400> 34
   Met Asn Lys Ile Phe Lys Val Ile Trp Asn Pro Ala Thr Gly Ser Tyr
   1               5                   10                  15
   Thr Val Ala Ser Glu Thr Ala Lys Ser Arg Gly Lys Lys Ser Gly Arg
   20 25 30
   Ser Lys Leu Leu Ile Ser Ala Leu Val Ala Gly Gly Leu Leu Ser Ser
   35 40 45
   Phe Gly Ala Ser Ala Asp Asn Tyr Thr Gly Gln Pro Thr Asp Tyr Gly
   50 55 60
   Asp Gly Ser Ala Gly Asp Gly Trp Val Ala Ile Gly Lys Gly Ala Lys
   65 70 75 80
   Ala Asn Thr Phe Met Asn Thr Ser Gly Ala Ser Thr Ala Leu Gly Tyr
   85 90 95
   Asp Ala Ile Ala Glu Gly Glu Tyr Ser Ser Ala Ile Gly Ser Lys Thr
   100 105 110
   Leu Ala Thr Gly Gly Ala Ser Met Ala Phe Gly Val Ser Ala Lys Ala
   115 120 125
   Met Gly Asp Arg Ser Val Ala Leu Gly Ala Ser Ser Val Ala Asn Gly
   130 135 140
   Asp Arg Ser Met Ala Phe Gly Arg Tyr Ala Lys Thr Asn Gly Phe Thr
   145 150 155 160
   Ser Leu Ala Ile Gly Asp Ser Ser Leu Ala Asp Gly Glu Lys Thr Ile
   165 170 175
   Ala Leu Gly Asn Thr Ala Lys Ala Tyr Glu Ile Met Ser Ile Ala Leu
   180 185 190
   Gly Asp Asn Ala Asn Ala Ser Lys Glu Tyr Ala Met Ala Leu Gly Ala
   195 200 205
   Ser Ser Lys Ala Gly Gly Ala Asp Ser Leu Ala Phe Gly Arg Lys Ser
   210 215 220
   Thr Ala Asn Ser Thr Gly Ser Leu Ala Ile Gly Ala Asp Ser Ser Ser
   225 230 235 240
   Ser Asn Asp Asn Ala Ile Ala Ile Gly Asn Lys Thr Gln Ala Leu Gly
   245 250 255
   Val Asn Ser Met Ala Leu Gly Asn Ala Ser Gln Ala Ser Gly Glu Ser
   260 265 270
   Ser Ile Ala Leu Gly Asn Thr Ser Glu Ala Ser Glu Gln Asn Ala Ile
   275 280 285
   Ala Leu Gly Gln Gly Ser Ile Ala Ser Lys Val Asn Ser Ile Ala Leu
   290 295 300
   Gly Ser Asn Ser Leu Ser Ser Gly Glu Asn Ala Ile Ala Leu Gly Glu
   305 310 315 320
   Gly Ser Ala Ala Gly Gly Ser Asn Ser Leu Ala Phe Gly Ser Gln Ser
   325 330 335
   Arg Ala Asn Gly Asn Asp Ser Val Ala Ile Gly Val Gly Ala Ala Ala
   340 345 350
   Ala Thr Asp Asn Ser Val Ala Ile Gly Ala Gly Ser Thr Thr Asp Ala
   355 360 365
   Ser Asn Thr Val Ser Val Gly Asn Ser Ala Thr Lys Arg Lys Ile Val
   370 375 380
   Asn Met Ala Ala Gly Ala Ile Ser Asn Thr Ser Thr Asp Ala Ile Asn
   385 390 395 400
   Gly Ser Gln Leu Tyr Thr Ile Ser Asp Ser Val Ala Lys Arg Leu Gly
   405 410 415
   Gly Gly Ala Thr Val Gly Ser Asp Gly Thr Val Thr Ala Val Ser Tyr
   420 425 430
   Ala Leu Arg Ser Gly Thr Tyr Asn Asn Val Gly Asp Ala Leu Ser Gly
   435 440 445
   Ile Asp Asn Asn Thr Leu Gln Trp Asn Lys Thr Ala Gly Ala Phe Ser
   450 455 460
   Ala Asn His Gly Ala Asn Ala Thr Asn Lys Ile Thr Asn Val Ala Lys
   465 470 475 480
   Gly Thr Val Ser Ala Thr Ser Thr Asp Val Val Asn Gly Ser Gln Leu
   485 490 495
   Tyr Asp Leu Gln Gln Asp Ala Leu Leu Trp Asn Gly Thr Ala Phe Ser
   500 505 510
   Ala Ala His Gly Thr Glu Ala Thr Ser Lys Ile Thr Asn Val Thr Ala
   515 520 525
   Gly Asn Leu Thr Ala Gly Ser Thr Asp Ala Val Asn Gly Ser Gln Leu
   530 535 540
   Lys Thr Thr Asn Asp Asn Val Thr Thr Asn Thr Thr Asn Ile Ala Thr
   545 550 555 560
   Asn Thr Thr Asn Ile Thr Asn Leu Thr Asp Ala Val Asn Gly Leu Gly
   565 570 575
   Asp Asp Ser Leu Leu Trp Asn Lys Ala Ala Gly Ala Phe Ser Ala Ala
   580 585 590
   His Gly Thr Glu Ala Thr Ser Lys Ile Thr Asn Val Thr Ala Gly Asn
   595 600 605
   Leu Thr Ala Gly Ser Thr Asp Ala Val Asn Gly Ser Gln Leu Lys Thr
   610 615 620
   Thr Asn Asp Asn Val Thr Thr Asn Thr Thr Asn Ile Ala Thr Asn Thr
   625 630 635 640
   Thr Asn Ile Thr Asn Leu Thr Asp Ala Val Asn Gly Leu Gly Asp Asp
   645 650 655
   Ser Leu Leu Trp Asn Lys Thr Ala Gly Ala Phe Ser Ala Ala His Gly
   660 665 670
   Thr Asp Ala Thr Ser Lys Ile Thr Asn Val Thr Ala Gly Asn Leu Thr
   675 680 685
   Ala Gly Ser Thr Asp Ala Val Asn Gly Ser Gln Leu Lys Thr Thr Asn
   690 695 700
   Asp Asn Val Thr Thr Asn Thr Thr Asn Ile Ala Thr Asn Thr Thr Asn
   705 710 715 720
   Ile Thr Asn Leu Thr Asp Ala Val Asn Gly Leu Gly Asp Asp Ser Leu
   725 730 735
   Leu Trp Asn Lys Thr Ala Gly Ala Phe Ser Ala Ala His Gly Thr Asp
   740 745 750
   Ala Thr Ser Lys Ile Thr Asn Val Lys Ala Gly Asp Leu Thr Ala Gly
   755 760 765
   Ser Thr Asp Ala Val Asn Gly Ser Gln Leu Lys Thr Thr Asn Asp Asn
   770 775 780
   Val Ser Thr Asn Thr Thr Asn Ile Thr Asn Leu Thr Asp Ala Val Asn
   785 790 795 800
   Gly Leu Gly Asp Asp Ser Leu Leu Trp Asn Lys Thr Ala Gly Ala Phe
   805 810 815
   Ser Ala Ala His Gly Thr Asp Ala Thr Ser Lys Ile Thr Asn Val Lys
   820 825 830
   Ala Gly Asp Leu Thr Ala Gly Ser Thr Asp Ala Val Asn Gly Ser Gln
   835 840 845
   Leu Lys Thr Thr Asn Asp Asn Val Ser Thr Asn Thr Thr Asn Ile Thr
   850 855 860
   Asn Leu Thr Asp Ser Val Gly Asp Leu Lys Asp Asp Ser Leu Leu Trp
   865 870 875 880
   Asn Lys Ala Ala Gly Ala Phe Ser Ala Ala His Gly Thr Glu Ala Thr
   885 890 895
   Ser Lys Ile Thr Asn Leu Leu Ala Gly Lys Ile Ser Ser Asn Ser Thr
   900 905 910
   Asp Ala Ile Asn Gly Ser Gln Leu Tyr Gly Val Ala Asp Ser Phe Thr
   915 920 925
   Ser Tyr Leu Gly Gly Gly Ala Asp Ile Ser Asp Thr Gly Val Leu Ser
   930 935 940
   Gly Pro Thr Tyr Thr Ile Gly Gly Thr Asp Tyr Thr Asn Val Gly Asp
   945 950 955 960
   Ala Leu Ala Ala Ile Asn Thr Ser Phe Ser Thr Ser Leu Gly Asp
   Ala
   965 970 975
   Leu Leu Trp Asp Ala Thr Ala Gly Lys Phe Ser Ala Lys His Gly
   Ile
   980 985 990
   Asn Asn Ala Pro Ser Val Ile Thr Asp Val Ala Asn Gly Ala Val
   Ser
   995 1000 1005
   Ser Thr Ser Ser Asp Ala Ile Asn Gly Ser Gln Leu Tyr Gly Val
   1010 1015 1020
   Ser Asp Tyr Ile Ala Asp Ala Leu Gly Gly Asn Ala Val Val Asn
   1025 1030 1035
   Thr Asp Gly Ser Ile Thr Thr Pro Thr Tyr Ala Ile Ala Gly Gly
   1040 1045 1050
   Ser Tyr Asn Asn Val Gly Asp Ala Leu Glu Ala Ile Asp Thr Thr
   1055 1060 1065
   Leu Asp Asp Ala Leu Leu Trp Asp Thr Thr Ala Asn Gly Gly Asn
   1070 1075 1080
   Gly Ala Phe Ser Ala Ala His Gly Lys Asp Lys Thr Ala Ser Val
   1085 1090 1095
   Ile Thr Asn Val Ala Asn Gly Ala Val Ser Ala Thr Ser Asn Asp
   1100 1105 1110
   Ala Ile Asn Gly Ser Gln Leu Tyr Ser Thr Asn Lys Tyr Ile Ala
   1115 1120 1125
   Asp Ala Leu Gly Gly Asp Ala Glu Val Asn Ala Asp Gly Thr Ile
   1130 1135 1140
   Thr Ala Pro Thr Tyr Thr Ile Ala Asn Thr Asp Tyr Asn Asn Val
   1145 1150 1155
   Gly Glu Ala Leu Asp Ala Leu Asp Asn Asn Ala Leu Leu Trp Asp
   1160 1165 1170
   Glu Asp Ala Gly Ala Tyr Asn Ala Ser His Asp Gly Asn Ala Ser
   1175 1180 1185
   Lys Ile Thr Asn Val Ala Ala Gly Asp Leu Ser Thr Thr Ser Thr
   1190 1195 1200
   Asp Ala Val Asn Gly Ser Gln Leu Asn Ala Thr Asn Ile Leu Val
   1205 1210 1215
   Thr Gln Asn Ser Gln Met Ile Asn Gln Leu Ala Gly Asn Thr Ser
   1220 1225 1230
   Glu Thr Tyr Ile Glu Glu Asn Gly Ala Gly Ile Asn Tyr Val Arg
   1235 1240 1245
   Thr Asn Asp Ser Gly Leu Ala Phe Asn Asp Ala Ser Ala Ser Gly
   1250 1255 1260
   Ile Gly Ala Thr Ala Val Gly Tyr Asn Ala Val Ala Ser His Ala
   1265 1270 1275
   Ser Ser Val Ala Ile Gly Gln Asp Ser Ile Ser Glu Val Asp Thr
   1280 1285 1290
   Gly Ile Ala Leu Gly Ser Ser Ser Val Ser Ser Arg Val Ile Val
   1295 1300 1305
   Lys Gly Thr Arg Asn Thr Ser Val Ser Glu Glu Gly Val Val Ile
   1310 1315 1320
   Gly Tyr Asp Thr Thr Asp Gly Glu Leu Leu Gly Ala Leu Ser Ile
   1325 1330 1335
   Gly Asp Asp Gly Lys Tyr Arg Gln Ile Ile Asn Val Ala Asp Gly
   1340 1345 1350
   Ser Glu Ala His Asp Ala Val Thr Val Arg Gln Leu Gln Asn Ala
   1355 1360 1365
   Ile Gly Ala Val Ala Thr Thr Pro Thr Lys Tyr Tyr His Ala Asn
   1370 1375 1380
   Ser Thr Ala Glu Asp Ser Leu Ala Val Gly Glu Asp Ser Leu Ala
   1385 1390 1395
   Met Gly Ala Lys Thr Ile Val Asn Gly Asn Ala Gly Ile Gly Ile
   1400 1405 1410
   Gly Leu Asn Thr Leu Val Leu Ala Asp Ala Ile Asn Gly Ile Ala
   1415 1420 1425
   Ile Gly Ser Asn Ala Arg Ala Asn His Ala Asp Ser Ile Ala Met
   1430 1435 1440
   Gly Asn Gly Ser Gln Thr Thr Arg Gly Ala Gln Thr Asn Tyr Thr
   1445 1450 1455
   Ala Tyr Asn Met Asp Ala Pro Gln Asn Ser Val Gly Glu Phe Ser
   1460 1465 1470
   Val Gly Ser Glu Asp Gly Gln Arg Gln Ile Thr Asn Val Ala Ala
   1475 1480 1485
   Gly Ser Ala Asp Thr Asp Ala Val Asn Val Gly Gln Leu Lys Val
   1490 1495 1500
   Thr Asp Ala Gln Val Ser Gln Asn Thr Gln Ser Ile Thr Asn Leu
   1505 1510 1515
   Asn Thr Gln Val Thr Asn Leu Asp Thr Arg Val Thr Asn Ile Glu
   1520 1525 1530
   Asn Gly Ile Gly Asp Ile Val Thr Thr Gly Ser Thr Lys Tyr Phe
   1535 1540 1545
   Lys Thr Asn Thr Asp Gly Ala Asp Ala Asn Ala Gln Gly Lys Asp
   1550 1555 1560
   Ser Val Ala Ile Gly Ser Gly Ser Ile Ala Ala Ala Asp Asn Ser
   1565 1570 1575
   Val Ala Leu Gly Thr Gly Ser Val Ala Asp Glu Glu Asn Thr Ile
   1580 1585 1590
   Ser Val Gly Ser Ser Thr Asn Gln Arg Arg Ile Thr Asn Val Ala
   1595 1600 1605
   Ala Gly Val Asn Ala Thr Asp Ala Val Asn Val Ser Gln Leu Lys
   1610 1615 1620
   Ser Ser Glu Ala Gly Gly Val Arg Tyr Asp Thr Lys Ala Asp Gly
   1625 1630 1635
   Ser Ile Asp Tyr Ser Asn Ile Thr Leu Gly Gly Gly Asn Ser Gly
   1640 1645 1650
   Thr Thr Arg Ile Ser Asn Val Ser Ala Gly Val Asn Asn Asn Asp
   1655 1660 1665
   Ala Val Asn Tyr Ala Gln Leu Lys Gln Ser Val Gln Glu Thr Lys
   1670 1675 1680
   Gln Tyr Thr Asp Gln Arg Met Val Glu Met Asp Asn Lys Leu Ser
   1685 1690 1695
   Lys Thr Glu Ser Lys Leu Ser Gly Gly Ile Ala Ser Ala Met Ala
   1700 1705 1710
   Met Thr Gly Leu Pro Gln Ala Tyr Thr Pro Gly Ala Ser Met Ala
   1715 1720 1725
   Ser Ile Gly Gly Gly Thr Tyr Asn Gly Glu Ser Ala Val Ala Leu
   1730 1735 1740
   Gly Val Ser Met Val Ser Ala Asn Gly Arg Trp Val Tyr Lys Leu
   1745 1750 1755
   Gln Gly Ser Thr Asn Ser Gln Gly Glu Tyr Ser Ala Ala Leu Gly
   1760 1765 1770
   Ala Gly Ile Gln Trp
   1775
<210> 35 <211> 227 <212> PRT <213> Escherichia coli <400> 35
<210> 36 <211> 1109 <212> PRT <213> Escherichia coli <400> 36
<210> 38 <211> 280 <212> PRT <213> Escherichia coli <400> 38
<210> 39 <211> 501 <212> PRT <213> Escherichia coli <400> 39
<210> 40 <211> 682 <212> PRT <213> Escherichia coli <400> 40
<210> 41 <211> 164 <212> PRT <213> Escherichia coli <400> 41
<210> 42 <211> 218 <212> PRT <213> Escherichia coli <400> 42
<210> 43 <211> 2732 <212> PRT <213> Escherichia coli
<400> 43
   Met His Gln Pro Pro Val Arg Phe Thr Tyr Arg Leu Leu Ser Tyr
   Leu
   1 5 10 15
   Val Ser Ala Ile Ile Ala Gly Gln Pro Leu Leu Pro Ala Val Gly
   Ala
   20 25 30
   Val Ile Thr Pro Gln Asn Gly Ala Gly Met Asp Lys Ala Ala Asn
   Gly
   35 40 45
   Val Pro Val Val Asn Ile Ala Thr Pro Asn Gly Ala Gly Ile Ser
   His
   50 55 60
   Asn Arg Phe Thr Asp Tyr Asn Val Gly Lys Glu Gly Leu Ile Leu
   Asn
   65 70 75 80
   Asn Ala Thr Gly Lys Leu Asn Pro Thr Gln Leu Gly Gly Leu Ile
   Gln
   85 90 95
   Asn Asn Pro Asn Leu Lys Ala Gly Gly Glu Ala Lys Gly Ile Ile
   Asn
   100 105 110
   Glu Val Thr Gly Gly Lys Arg Ser Leu Leu Gln Gly Tyr Thr Glu
   Val
   115 120 125
   Ala Gly Lys Ala Ala Asn Val Met Val Ala Asn Pro Tyr Gly Ile
   Thr
   130 135 140
   Cys Asp Gly Cys Gly Phe Ile Asn Thr Pro His Ala Thr Leu Thr
   Thr
   145 150 155
   160
   Gly Lys Pro Val Met Asn Ala Asp Gly Ser Leu Gln Ala Leu Glu
   Val
   165 170 175
   Thr Glu Gly Ser Ile Thr Ile Asn Gly Ala Gly Leu Asp Gly Thr
   Arg
   180 185 190
   Ser Asp Ala Val Ser Ile Ile Ala Arg Ala Thr Glu Val Asn Ala
   Ala
   195 200 205
   Leu His Ala Lys Asp Leu Thr Val Thr Ala Gly Ala Asn Arg Val
   Thr
   210 215 220
   Ala Asp Gly Arg Val Arg Ala Leu Lys Gly Glu Gly Asp Val Pro
   Lys
   225 230 235
   240
   Val Ala Val Asp Thr Gly Ala Leu Gly Gly Met Tyr Ala Arg Arg
   Ile
   245 250 255
   His Leu Thr Ser Thr Glu Ser Gly Val Gly Val Asn Leu Gly Asn Leu
   260 265 270
   Tyr Ala Arg Asp Gly Asp Ile Thr Leu Asp Ala Ser Gly Arg Leu Thr
   275 280 285
   Val Asn Asn Ser Leu Ala Thr Gly Ala Val Thr Ala Lys Gly Gln Gly
   290 295 300
   Val Thr Leu Thr Gly Asp His Lys Ala Gly Gly Asn Leu Ser Val Ser
   305 310 315 320
   Ser Arg Arg Asp Ile Val Leu Ser Asn Gly Thr Leu Asn Ser Asp Lys
   325 330 335
   Asp Leu Ser Leu Thr Ala Gly Gly Arg Ile Thr Gln Gln Asn Glu Lys
   340 345 350
   Leu Thr Ala Gly Arg Asp Val Thr Leu Ala Ala Lys Asn Ile Thr Gln
   355 360 365
   Asp Thr Ala Ser Gln Ile Asn Ala Ala Arg Asp Ile Val Thr Val Ala
   370 375 380
   Ser Asp Thr Leu Thr Thr Gln Gly Gln Ile Thr Ala Gly Gln Asn Leu
   385 390 395 400
   Thr Ala Ser Ala Thr Thr Leu Thr Gln Asp Gly Ile Leu Leu Ala Lys
   405 410 415
   Ser His Ala Gly Leu Asn Ala Gly Thr Leu Asn Asn Ser Gly Ala Val
   420 425 430
   Gln Gly Ala Thr Leu Thr Leu Gly Ser Thr Thr Leu Ser Asn Ser Gly
   435 440 445
   Ser Leu Leu Ser Gly Gly Pro Leu Thr Met Asn Thr Arg Asp Phe Thr
   450 455 460
   Gln Ser Gly Arg Thr Gly Ala Lys Gly Lys Val Asp Ile Met Ala Ser
   465 470 475
   480
   Gly Lys Leu Thr Ser Thr Gly Leu Leu Val Thr Met His Leu Val Leu
   485 490 495
   Lys Ala Gln Asp Val Thr Gln Asn Gly Val Leu Ser Gly Gly Lys Gly
   500 505 510
   Leu Thr Val Ser Ala Thr Ser Ser Gly Lys Lys Ser Val Thr His Ser
   515 520 525
   Asp Ala Ala Met Thr Leu Asn Val Thr Thr Val Ala Leu Asp Gly Glu
   530 535 540
   Thr Ser Ala Gly Asp Thr Leu Arg Val Gln Ala Asp Lys Leu Ser Thr
   545 550 555
   560
   Ala Ala Gly Ala Gln Leu Gln Ser Gly Lys Asn Leu Ser Ile Asn Ala
   565 570 575
   Arg Asp Ala Arg Leu Ala Gly Thr Gln Ala Ala Gln Gln Thr Met
   Val
   580 585 590
   Val Asn Ala Ser Glu Lys Leu Thr His Ser Gly Lys Ser Ser Ala
   Pro
   595 600 605
   Ser Leu Ser Leu Ser Ala Pro Glu Leu Thr Ser Ser Gly Val Leu
   Val
   610 615 620
   Gly Ser Ala Leu Asn Thr Gln Ser Gln Thr Leu Thr Asn Ser Gly
   Leu
   625 630 635
   640
   Leu Gln Gly Glu Ala Ser Leu Thr Val Asn Thr Gln Arg Leu Asp
   Asn
   645 650 655
   Gln Gln Asn Gly Thr Leu Tyr Ser Ala Ala Asp Leu Thr Leu Asp
   Ile
   660 665 670
   Pro Asp Ile Arg Asn Ser Gly Leu Ile Thr Gly Asp Asn Gly Leu
   Met
   675 680 685
   Leu Asn Ala Val Ser Leu Ser Asn Pro Gly Lys Ile Ile Ala Asp
   Thr
   690 695 700
   Leu Ser Val Arg Ala Thr Thr Leu Asp Gly Asp Gly Leu Leu Gln
   Gly
   705 710 715
   720
   Ala Gly Ala Leu Ala Leu Ala Gly Asp Thr Leu Ser Gln Gly Ser
   His
   725 730 735
   Gly Arg Trp Leu Thr Ala Asp Asp Leu Ser Leu Arg Gly Lys Thr Leu
   740 745 750
   Asn Thr Ala Gly Thr Thr Gln Gly Gln Asn Ile Thr Val Gln Ala Asp
   755 760 765
   Arg Trp Ala Asn Ser Gly Ser Val Leu Ala Thr Gly Asn Leu Thr Ala
   770 775 780
   Ser Ala Thr Gly Gln Leu Thr Ser Thr Gly Asp Ile Met Ser Gln Gly
   785 790 795 800
   Asp Thr Thr Leu Lys Ala Ala Thr Thr Asp Asn Arg Gly Ser Leu Leu
   805 810 815
   Ser Ala Gly Thr Leu Ser Leu Asp Gly Asn Ser Leu Asp Asn Arg Gly
   820 825 830
   Thr Val Gln Gly Asn His Val Thr Ile Arg Gln Asn Ser Val Thr Asn
   835 840 845
   Ser Gly Thr Leu Thr Gly Ile Ala Ala Leu Thr Leu Ala Ala Arg Met
   850 855 860
   Ala Ser Pro Gln Pro Ala Leu Met Asn Asn Gly Gly Ser Leu Leu Thr
   865 870 875 880
   Ser Gly Asp Leu Thr Ile Thr Ala Gly Ser Ile Thr Ser Ser Gly His
   885 890 895
   Trp Gln Gly Lys Arg Val Leu Ile Thr Ala Asp Ser Leu Ala Asn Ser
   900 905 910
   Gly Ala Ile Gln Ala Ala Asp Ser Leu Thr Ala Arg Leu Thr Gly Glu
   915 920 925
   Leu Val Ser Thr Ala Gly Ser Lys Val Thr Ser Asn Gly Glu Met Ala
   930 935 940
   Leu Ser Ala Leu Asn Leu Ser Asn Ser Gly Gln Trp Ile Ala Lys Asn
   945 950 955 960
   Leu Thr Leu Lys Ala Asn Ser Leu Thr Ser Ala Gly Asp Ile Thr Gly
   965 970 975
   Val Asp Thr Leu Thr Leu Thr Val Asn Gln Thr Leu Asn Asn Gln Ala
   980 985 990
   Asn Gly Lys Leu Leu Ser Ala Gly Val Leu Thr Leu Lys Ala Asp Ser
   995 1000 1005
   Val Thr Asn Asp Gly Gln Leu Gln Gly Asn Val Thr Thr Ile Thr
   1010 1015 1020
   Ala Gly Gln Leu Thr Asn Gly Gly His Leu Gln Gly Glu Thr Leu
   1025 1030 1035
   Thr Leu Thr Ala Ser Gly Gly Val Asn Asn Arg Ser Gly Gly Val
   1040 1045 1050
   Leu Met Ser Arg Asn Ala Leu Asn Val Ser Thr Ala Thr Leu Ser
   1055 1060 1065
   Asn Gln Ser Thr Ile Gln Gly Gly Gly Gly Val Ser Leu Asn Ala
   1070 1075 1080
   Thr Asp Arg Leu Gln Asn Asp Gly Lys Ile Leu Ser Gly Ser Asn
   1085 1090 1095
   Leu Thr Leu Thr Ala Gln Val Leu Ala Asn Thr Gly Ser Gly Leu
   1100 1105 1110
   Val Gln Ala Ala Thr Leu Leu Leu Asp Val Val Asn Thr Val Asn
   1115 1120 1125
   Gly Gly Arg Val Leu Ala Thr Gly Ser Asp Val Lys Gly Thr Thr
   1130 1135 1140
   Leu Asn Asn Thr Gly Thr Leu Gln Gly Ala Thr Leu Val Asn Tyr
   1145 1150 1155
   His Thr Phe Ser Ser Gly Thr Leu Leu Gly Thr Ser Gly Leu Gly
   1160 1165 1170
   Val Lys Gly Ser Ser Leu Leu Gln Asn Gly Thr Gly Arg Leu Tyr
   1175 1180 1185
   Ser Ala Gly Asn Leu Leu Leu Asp Ala Gln Asp Phe Ser Gly Gln
   1190 1195 1200
   Gly Gln Val Val Ala Thr Gly Asp Val Thr Leu Lys Leu Ile Ala
   1205 1210 1215
   Ala Leu Thr Asn His Gly Thr Leu Ala Ala Gly Lys Thr Leu Ser
   1220 1225 1230
   Val Thr Ser Gln Asn Ala Ile Thr Asn Gly Gly Val Met Gln Gly
   1235 1240 1245
   Asp Ala Met Val Leu Gly Ala Gly Glu Ala Phe Thr Asn Asn Gly
   1250 1255 1260
   Leu Thr Ala Gly Lys Gly Asn Ser Val Phe Ser Ala Gln Arg Leu
   1265 1270 1275
   Phe Leu Asn Ala Pro Gly Ser Leu Gln Gly Gly Gly Asp Val Ser
   1280 1285 1290
   Leu Asn Ser Arg Ser Asp Ile Thr Ile Ser Gly Phe Thr Gly Thr
   1295 1300 1305
   Ala Gly Ser Leu Thr Met Asn Val Ala Gly Thr Leu Leu Asn Ser
   1310 1315 1320
   Ala Leu Ile Tyr Ala Gly Asn Asn Leu Lys Leu Phe Thr Asp Arg
   1325 1330 1335
   Leu His Asn Gln His Gly Asp Ile Leu Ala Gly Asn Ser Leu Trp
   1340 1345 1350
   Val Gln Lys Asp Ala Ser Gly Gly Ala Asn Thr Glu Ile Ile Asn
   1355 1360 1365
   Asn Ser Gly Asn Ile Glu Thr His Gln Gly Asp Ile Val Val Arg
   1370 1375 1380
   Thr Gly His Leu Leu Asn Gln Arg Glu Gly Phe Ser Ala Thr Thr
   1385 1390 1395
   Thr Thr Arg Thr Asn Pro Ser Ser Ile Gln Gly Met Gly Asn Ala
   1400 1405 1410
   Leu Val Asp Ile Pro Leu Ser Leu Leu Pro Asp Gly Ser Tyr Gly
   1415 1420 1425
   Tyr Phe Thr Arg Glu Val Glu Asn Gln His Gly Thr Pro Cys Asn
   1430 1435 1440
   Gly His Gly Ala Cys Asn Ile Thr Met Asp Thr Leu Tyr Tyr Tyr
   1445 1450 1455
   Ala Pro Phe Ala Asp Ser Ala Thr Gln Arg Phe Leu Ser Ser Gln
   1460 1465 1470
   Asn Ile Thr Thr Val Thr Gly Ala Asp Asn Pro Ala Gly Arg Ile
   1475 1480 1485
   Ala Ser Gly Arg Asn Leu Ser Ala Glu Ala Glu Arg Leu Glu Asn
   1490 1495 1500
   Arg Ala Ser Phe Ile Leu Ala Asn Gly Asp Ile Ala Leu Ser Gly
   1505 1510 1515
   Arg Glu Leu Ser Asn Gln Ser Trp Gln Thr Gly Thr Glu Asn Glu
   1520 1525 1530
   Tyr Leu Val Tyr Arg Tyr Asp Pro Lys Thr Phe Tyr Gly Ser Tyr
   1535 1540 1545
   Ala Thr Gly Ser Leu Asp Lys Leu Pro Leu Leu Ser Pro Glu Phe
   1550 1555 1560
   Glu Asn Asn Thr Ile Arg Phe Ser Leu Asp Gly Arg Glu Lys Asp
   1565 1570 1575
   Tyr Thr Pro Gly Lys Thr Tyr Tyr Ser Val Ile Gln Ala Gly Gly
   1580 1585 1590
   Asp Val Lys Thr Arg Phe Thr Ser Ser Ile Asn Asn Gly Thr Thr
   1595 1600 1605
   Thr Ala His Ala Gly Ser Val Ser Pro Val Val Ser Ala Pro Val
   1610 1615 1620
   Leu Asn Thr Leu Ser Gln Gln Thr Gly Gly Asp Ser Leu Thr Gln
   1625 1630 1635
   Thr Ala Leu Gln Gln Tyr Glu Pro Val Val Val Gly Ser Pro Gln
   1640 1645 1650
   Trp His Asp Glu Leu Ala Gly Ala Leu Lys Asn Ile Ala Gly Gly
   1655 1660 1665
   Ser Pro Leu Thr Gly Gln Thr Gly Ile Ser Asp Asp Trp Pro Leu
   1670 1675 1680
   Pro Ser Gly Asn Asn Gly Tyr Leu Val Pro Ser Thr Asp Pro Asp
   1685 1690 1695
   Ser Pro Tyr Leu Ile Thr Val Asn Pro Lys Leu Asp Gly Leu Gly
   1700 1705 1710
   Gln Val Asp Ser His Leu Phe Ala Gly Leu Tyr Glu Leu Leu Gly
   1715 1720 1725
   Ala Lys Pro Gly Gln Ala Pro Arg Glu Thr Ala Pro Ser Tyr Thr
   1730 1735 1740
   Asp Glu Lys Gln Phe Leu Gly Ser Ser Tyr Phe Leu Asp Arg Leu
   1745 1750 1755
   Gly Leu Lys Pro Glu Lys Asp Tyr Arg Phe Leu Gly Asp Ala Val
   1760 1765 1770
   Phe Asp Thr Arg Tyr Val Ser Asn Ala Val Leu Ser Arg Thr Gly
   1775 1780 1785
   Ser Arg Tyr Leu Asn Gly Leu Gly Ser Asp Thr Glu Gln Met Arg
   1790 1795 1800
   Tyr Leu Met Asp Asn Ala Ala Arg Gln Gln Lys Gly Leu Gly Leu
   1805 1810 1815
   Glu Phe Gly Val Ala Leu Thr Ala Glu Gln Ile Ala Gln Leu Asp
   1820 1825 1830
   Gly Ser Ile Leu Trp Trp Glu Ser Val Thr Ile Asn Gly Gln Thr
   1835 1840 1845
   Val Met Val Pro Lys Leu Tyr Leu Ser Pro Glu Asp Ile Thr Leu
   1850 1855 1860
   His Asn Gly Ser Val Ile Ser Gly Asn Asn Val Gln Leu Ala Gly
   1865 1870 1875
   Gly Asn Ile Thr Asn Ser Gly Gly Ser Ile Asn Ala Gln Asn Asp
   1880 1885 1890
   Leu Ser Leu Asp Ser Ser Gly Tyr Ile Asp Asn Leu Asn Ala Gly
   1895 1900 1905
   Leu Ile Ser Ala Gly Gly Ser Leu Asp Leu Ser Ala Ile Gly Asp
   1910 1915 1920
   Ile Ser Asn Ile Ser Ser Val Ile Ser Gly Lys Thr Val Gln Leu
   1925 1930 1935
   Glu Ser Val Ser Gly Asn Ile Ser Asn Ile Thr Arg Arg Gln Gln
   1940 1945 1950
   Trp Asn Ala Gly Ser Asp Ser Gln Tyr Gly Gly Val His Leu Ser
   1955 1960 1965
   Gly Thr Asp Thr Gly Pro Val Ala Thr Ile Lys Gly Thr Asp Ser
   1970 1975 1980
   Leu Ser Leu Asp Ala Gly Lys Asn Ile Asp Ile Thr Gly Ala Thr
   1985 1990 1995
   Val Ser Ser Gly Gly Asp Leu Gly Met Ser Ala Gly Asn Asp Ile
   2000 2005 2010
   Asn Ile Ala Ala Asn Leu Ile Ser Gly Ser Lys Ser Gln Ser Gly
   2015 2020 2025
   Phe Trp His Thr Asp Asp Asn Ser Ser Ser Ser Thr Thr Ser Gln
   2030 2035 2040
   Gly Ser Ser Ile Ser Ala Gly Gly Asn Leu Ala Met Ala Ala Gly
   2045 2050 2055
   His Asn Leu Asp Val Thr Ala Ser Ser Val Ser Ala Gly His Ser
   2060 2065 2070
   Ala Leu Leu Ser Cys Arg Ser Arg Pro Ser Leu Glu Cys Ser Gln
   2075 2080 2085
   Gly Lys Ala Lys Thr Ser Arg Asn Gly Arg Ser Glu Ser His Glu
   2090 2095 2100
   Ser His Ala Ala Val Ser Thr Val Thr Ala Gly Asp Asn Phe Leu
   2105 2110 2115
   Leu Val Ala Gly Arg Asp Ile Ala Ser Gln Ala Ala Gly Met Ala
   2120 2125 2130
   Ala Glu Asn Asn Val Val Ile Arg Gly Gly Arg Asp Val Asn Leu
   2135 2140 2145
   Val Ala Glu Ser Ala Gly Ala Gly Asp Ser Tyr Thr Ser Lys Lys
   2150 2155 2160
   Lys Lys Glu Ile Asn Glu Thr Val Arg Gln Gln Gly Thr Glu Ile
   2165 2170 2175
   Ala Ser Gly Gly Asp Thr Thr Val Asn Ala Gly Arg Asp Ile Thr
   2180 2185 2190
   Ala Val Ala Ser Ser Val Thr Ala Thr Gly Asn Ile Ser Val Asn
   2195 2200 2205
   Ala Gly Arg Asp Val Ala Leu Thr Thr Ala Thr Glu Ser Asp Tyr
   2210 2215 2220
   His Tyr Leu Glu Thr Lys Lys Lys Ser Gly Gly Phe Leu Ser Lys
   2225 2230 2235
   Lys Thr Thr Arg Thr Ile Ser Glu Asp Ser Ala Thr Arg Glu Ala
   2240 2245 2250
   Gly Ser Leu Leu Ser Gly Asn Arg Val Thr Val Asn Ala Gly Asp
   2255 2260 2265
   Asn Leu Thr Val Glu Gly Ser Asp Val Val Ala Asp Arg Asp Val
   2270 2275 2280
   Ser Leu Ala Ala Gly Asn His Val Asp Val Leu Ala Ala Thr Ser
   2285 2290 2295
   Thr Asp Thr Ser Trp Arg Phe Lys Glu Thr Lys Lys Ser Gly Leu
   2300 2305 2310
   Met Gly Thr Gly Gly Ile Gly Phe Thr Ile Gly Ser Ser Lys Thr
   2315 2320 2325
   Thr His Asp Arg Arg Glu Ala Gly Thr Thr Gln Ser Gln Ser Ala
   2330 2335 2340
   Ser Thr Ile Gly Ser Thr Ala Gly Asn Val Ser Ile Thr Ala Gly
   2345 2350 2355
   Lys Gln Ala His Ile Ser Gly Ser Asp Val Ile Ala Asn Arg Asp
   2360 2365 2370
   Ile Ser Ile Thr Gly Asp Ser Val Val Val Asp Pro Gly His Asp
   2375 2380 2385
   Arg Arg Thr Val Asp Glu Lys Phe Glu Gln Lys Lys Ser Gly Leu
   2390 2395 2400
   Thr Val Ala Leu Ser Gly Thr Val Gly Ser Ala Ile Asn Asn Ala
   2405 2410 2415
   Val Thr Ser Ala Gln Glu Thr Lys Glu Ser Ser Asp Ser Arg Leu
   2420 2425 2430
   Lys Ala Leu Gln Ala Thr Lys Thr Ala Leu Ser Gly Val Gln Ala
   2435 2440 2445
   Gly Gln Ala Ala Thr Met Ala Ser Ala Thr Gly Asp Pro Asn Ala
   2450 2455 2460
   Gly Val Ser Leu Ser Leu Thr Thr Gln Lys Ser Lys Ser Gln Gln
   2465 2470 2475
   His Ser Glu Ser Asp Thr Val Ser Gly Ser Thr Leu Asn Ala Gly
   2480 2485 2490
   Asn Asn Leu Ser Val Val Ala Thr Gly Lys Asn Arg Gly Asp Asn
   2495 2500 2505
   Arg Gly Asp Ile Val Ile Ala Gly Ser Gln Leu Lys Ala Gly Gly
   2510 2515 2520
   Asn Thr Ser Leu Asp Ala Ala Asn Asp Ile Leu Leu Ser Gly Ala
   2525 2530 2535
   Ala Asn Thr Gln Lys Thr Thr Gly Arg Asn Ser Ser Ser Gly Gly
   2540 2545 2550
   Gly Val Gly Val Ser Ile Gly Ala Gly Lys Gly Ala Gly Ile Ser
   2555 2560 2565
   Ala Phe Ala Ser Val Asn Ala Ala Lys Gly Arg Glu Lys Gly Asn
   2570 2575 2580
   Gly Thr Thr Thr Asp Lys Thr Val Thr Ile Asn Ser Gly Arg Asp
   2585 2590 2595
   Thr Val Leu Asn Gly Ala Gln Val Asn Gly Asn Arg Ile Ile Ala
   2600 2605 2610
   Asp Val Gly His Asp Leu Leu Ile Ser Ser Gln Gln Asp Thr Ser
   2615 2620 2625
   Lys Tyr Asp Ser Lys Gln Thr Ser Val Ala Ala Gly Gly Ser Phe
   2630 2635 2640
   Thr Phe Gly Ser Met Thr Gly Ser Gly Tyr Ile Ala Ala Ser Arg
   2645 2650 2655
   Asp Lys Met Lys Ser Arg Phe Asp Ser Val Ala Glu Gln Thr Gly
   2660 2665 2670
   Met Phe Ala Arg Val Met Val Ala Ser Thr Ser Gln Trp Val Asn
   2675 2680 2685
   Ile Pro Asn Trp Met Val Arg Ser Leu Pro His Cys His Thr Gly
   2690 2695 2700
   Glu Lys Pro Pro Gly Tyr Arg Thr Leu Gly Leu Val Thr Leu Gln
   2705 2710 2715
   Arg Ser Gly Ile Ile Lys Ser Ser His Arg Trp Asn Gln Ser
   2720 2725 2730
<210> 44 <211> 321 <212> PRT <212> Escherichia coli <400> 44
<210> 45 <211> 587 <212> PRT <213> Escherichia coli <400> 45
<210> 46 <211> 744 <212> PRT <213> Escherichia coli <400> 46
<210> 47 <211> 136 <212> PRT <213> Escherichia coli <400> 47
<210> 48 <211> 225 <212> PRT <213> Escherichia coli <400> 48
<210> 49 <211> 721 <212> PRT <213> Escherichia coli <400> 49
<210> 50 <211> 669 <212> PRT <213> Escherichia coli <400> 50
<210> 51 <211> 753 <212> PRT <213> Escherichia coli <400> 51
<210> 52 <211> 133 <212> PRT <213> Escherichia coli <400> 52
<210> 53 <211> 286 <212> PRT <213> Escherichia coli <400> 53
<210> 54 <211> 172 <212> PRT <213> Escherichia coli <400> 54
<210> 55 <211> 182 <212> PRT <213> Escherichia coli <400> 55
<210> 56 <211> 359 <212> PRT <213> Escherichia coli <400> 56
<210> 57 <211> 844 <212> PRT <213> Escherichia coli <400> 57
<210> 58 <211> 277 <212> PRT <213> Escherichia coli <400> 58
<210> 59 <211> 366 <212> PRT <213> Escherichia coli <400> 59
<210> 60 <211> 260 <212> PRT <213> Escherichia coli <400> 60
<210> 61 <211> 385 <212> PRT <213> Escherichia coli <400> 61
<210> 62 <211> 105 <212> PRT <213> Escherichia coli <400> 62
<210> 63 <211> 147 <212> PRT <213> Escherichia coli <400> 63
<210> 64 <211> 178 <212> PRT <213> Escherichia coli <400> 64
<210> 65 <211> 209 <212> PRT <213> Escherichia coli <400> 65
<210> 66 <211> 424 <212> PRT <213> Escherichia coli <400> 66
<210> 67 <211> 489 <212> DNA <213> Escherichia coli <400> 67
<210> 68 <211> 2019 <212> DNA <213> Escherichia coli
<400> 68
<210> 69 <211> 738 <212> DNA <213> Escherichia coli <400> 69
<210> 70 <211> 498 <212> DNA <213> Escherichia coli <400> 70
<210> 71 <211> 3885 <212> DNA <213> Escherichia coli
<400> 71
   atgaataaaa tatactccct taaatatagt gctgccactg gcggactcat tgctgtttct 60
   gaattagcga aaagagtttc tggtaaaaca aaccgaaaac ttgtagcaac aatgttgtct 120
   ctggctgttg ccggtacagt aaatgcagca aatattgata tatcaaatgt atgggcgaga 180
   gactatcttg atcttgcaca aaataaaggt attttccagc ccggagcaac agacgtaaca 240
   atcactttaa aaaacggaga taaattctct ttccataatc tctcaattcc ggatttttct 300
   ggtgcagcag cgagtggcgc agctaccgca ataggaggtt cttatagtgt tactgttgca 360
   cataacaaaa agaaccctca ggccgcagaa acccaggttt acgctcagtc ttcttacagg 420
   gttgttgaca gaagaaattc caatgatttt gagattcaga ggttaaataa atttgttgtg 480
   gaaacagtag gtgccacccc ggcagagacc aaccctacaa catattctga tgcattagaa 540
   cgctacggta tagtcacttc tgacggttca aaaaaaatca taggttttcg tgctggctct 600
   ggaggaacat catttattaa tggtgaatcc aaaatctcaa caaattcagc atatagccat 660
   gatctgttaa gtgctagtct atttgaggtc acccaatggg actcatacgg catgatgatt 720
   tataaaaatg ataaaacatt tcgtaatctt gaaatattcg gagacagcgg ctctggagca 780
   tacttatatg ataacaaact agaaaaatgg gtattagtcg gaacaaccca tggtattgcc 840
   agcgttaatg gtgaccaact gacatggata acaaaataca atgataaact ggttagtgag 900
   ttaaaagata cctatagtca taaaataaat ctgaatggca ataatgtaac cattaaaaac 960
   acagatataa cattacacca aaacaatgca gataccactg gtactcaaga aaaaataact 1020
   aaagacaaag atattgtgtt cacaaatggg ggagatgtcc tgtttaagga taatttggat 1080
   tttggtagcg gtggtattat ctttgacgaa ggccatgaat ataacataaa cggtcaggga 1140
   tttacattta aaggagcagg aattgatatc ggaaaagaaa gcattgtaaa ctggaatgca 1200
   ttgtattcca gtgatgatgt tttacacaaa ataggccccg gtactctgaa tgttcaaaaa 1260
   aaacaggggg caaatataaa gataggtgaa ggaaatgtta ttcttaatga agaaggaaca 1320
   tttaacaata tataccttgc aagcggaaat ggtaaggtaa tactaaataa agataattcc 1380
   cttggcaatg atcaatatgc ggggatattt tttactaaac gtggtggtac gctagattta 1440
   aatggacaca atcagacttt tactagaatt gccgccactg acgatggaac aacaataact 1500
   aactcagata caacgaaaga agccgttctg gcaatcaata acgaagactc ctacatatat 1560
   catgggaaca taaatggcaa tataaaacta acgcacaata ttaattctca ggataagaaa 1620
   actaatgcaa aattaattct ggatggtagt gtcaacacaa aaaatgatgt tgaagtcagt 1680
   aatgccagtc ttaccatgca aggccatgca acagagcatg caatattcag aagctcagcg 1740
   aatcattgct ccctggtatt tctttgtgga acggactggg tcaccgtttt gaaagaaaca 1800
   gagagttcat ataataaaaa attcaattct gattacaaaa gtaataatca gcagacctca 1860
   tttgatcagc ctgactggaa aaccggggtg tttaaatttg atacattaca cctgaacaat 1920
   gctgactttt caatatcacg caatgccaat gttgaaggaa atatatcagc aaataaatca 1980
   gctatcacaa tcggcgataa aaatgtttac attgataatc ttgcagggaa aaatattact 2040
   aataatggtt ttgacttcaa acaaactatc agtactaatc tatccatagg agaaactaaa 2100
   tttacaggtg gcatcactgc acataacagc caaatagcca taggtgatca agctgtagtt 2160
   acacttaatg gtgcaacctt tctggataat actcctataa gtatagataa aggagcaaaa 2220
   gttatagcac aaaattccat gttcacaaca aaaggtattg atatctccgg tgaactgact 2280
   atgatgggaa tccctgaaca gaatagtaaa actgtaacgc cgggtctcca ctacgctgct 2340
   gatggattca ggctgagtgg tggaaatgca aatttcattg ccagaaatat ggcatctgtc 2400
   accggaaata tttatgctga tgatgcagca accattactc tgggacagcc tgaaactgaa 2460
   acaccgacta tatcgtctgc ttatcaggca tgggcagaga ctcttttgta tggctttgat 2520
   accgcttatc gaggcgcaat aacagccccc aaagctacag ttagcatgaa taatgcgatc 2580
   tggcatctaa atagccagtc atcaattaat cgtctagaaa caaaagacag tatggtgcgt 2640
   tttactggtg ataatgggaa gtttacaacc cttacagtga acaaccttac tatagatgac 2700
   agtgcatttg tgctgcgtgc aaatctggcc caagcagatc agcttgttgt caataaatcg 2760
   ttgtctggta aaaacaacct tctgttagtc gacttcattg agaaaaatgg aaacagcaac 2820
   ggactgaata tcgatctggt cagcgcacca aaaggaactg cagtagatgt ctttaaagct 2880
   acgactcgga gtattggctt cagtgatgta acaccggtta tcgagcaaaa gaacgataca 2940
   gacaaagcaa catggactct gatcggctat aaatctgtgg ccaacgccga tgcggctaaa 3000
   aaggcaacat tactgatgtc aggcggctat aaagccttcc ttgctgaggt caacaacctt 3060
   aacaaacgta tgggtgatct gcgtgacatt aacggtgagt ccggtgcatg ggcccgaatc 3120
   attagcggaa ccgggtctgc cggcggtgga ttcagtgaca actacaccca cgttcaggtc 3180
   ggtgcggata acaaacatga actcgatggc cttgacctct tcaccggggt gaccatgacc 3240
   tataccgaca gccatgcagg cagtgatgcc ttcagtggtg aaacgaagtc tgtgggtgcc 3300
   ggtctctatg cctctgccat gtttgagtcc ggagcatata tcgacctcat cggtaagtac 3360
   gttcaccatg acaacgagta taccgcaact ttcgccggcc ttggcaccag agactacagc 3420
   tcccactcct ggtatgccgg tgcggaagtc ggttaccgtt accatgtaac tgactctgca 3480
   tggattgagc cgcaggcgga acttgtttac ggtgctgtat ccgggaaaca gttctcctgg 3540
   aaggaccagg gaatgaacct caccatgaag gataaggact ttaatccgct gattgggcgt 3600
   accggtgttg atgtgggtaa atccttctcc ggtaaggact ggaaagtcac agcccgcgcc 3660
   ggccttggct accagtttga cctgtttgcc aacggtgaaa ccgtactgcg tgatgcgtcc 3720
   ggtgagaaac gtatcaaagg tgaaaaagac ggtcgtatgc tcatgaatgt tggtctcaac 3780
   gccgaaattc gcgataatct tcgcttcggt cttgagtttg agaaatcggc atttggtaaa 3840
   tacaacgtgg ataacgcgat caacgccaac ttccgttact ctttc 3885
<210> 72 <211> 426 <212> DNA <213> Escherichia coli <400> 72
<210> 73 <211> 954 <212> DNA <213> Escherichia coli <400> 73
<210> 74 <211> 2175 <212> DNA <213> Escherichia coli
<400> 74
<210> 75 <211> 3042 <212> DNA <213> Escherichia coli
<400> 75
<210> 76 <211> 1362 <212> DNA <213> Escherichia coli
<400> 76
   atggtggaca tgattaatga aagtgcacgg caaacgccag tcattgcaca aacggacgtt 60
   ctggttatcg ggggcggtcc ggcaggatta tccgctgcca ttgcggcagg gcggttaggt     120
   gccagaacca tgattgttga gcgctacggg tcgctaggcg gcgtattgac gcaggtcggg 180
   gtagaaagtt ttgcctggta tcgtcatccg gggacggaag attgtgaagg gatctgtcgt 240
   gagtatgaag gccgcgcacg agcgctgggt ttcacacgac cagaacctca gtcaattagc 300
   gaagttatag atactgaagg atttaaagtt gtcgccgatc agatgattac ggaatctggc 360
   gttgagccgt tatatcactc ctgggttgtg gacgtgatca aggacgggga tacgttatgc 420
   ggtgttatcg tcgagaataa atcaggtcga ggggcaattc tggcgaaaag aatcgtcgat 480
   tgcacggggg atgctgatat tgccgctcgt gcaggcgcgc cctggacgaa acggagcaag 540
   gaccaactga tgggcgtcac cgtgatgttc agttgcgcag gtgttgatgt ggcacgcttt 600
   aaccgttttg ttgcggaaga acttaagccg acctacgcgg attggggcaa aaactggacg 660
   attcaaacca cgggtaaaga agacccgatg tttagcccgt atatggagga tatttttacc 720
   cgcgcgcaac aggatggtgt gattccaggt gacgcccagg cgattgccgg aacctggtcg 780
   accttttctg aaagcggtga ggctttccag atgaatatgg tgtacgcctt tggttttgac 840
   tgtaccgatg tcttcgattt aaccaaagct gagattgccg gaaggcagca agcattatgg 900
   gcaattgacg cactacgcca ctatgttccg ggctttgaaa atgtacggtt acgcaatttt 960
   ggtgccacgc tggggacgcg tgaatcacgg cttattgagg gggaaatacg tattgctgat 1020
   gattacgtcc ttaatcaggg gcgttgttcg gacagtgtag ggattttccc ggaatttatt 1080
   gatggttccg gttatctcat tttgccaacg accgggcgtt tctttcagat cccttatggt 1140
   tgtctggtgc cgcaaaaagt ggagaacctt ttggtcgccg gtcgctgtat ttccgcaggc 1200
   gtagttgcac atacttctat gcgtaacatg atgtgttgtg ccgttaccgg tgaggccgca 1260
   ggtactgccg ccgtggtttc gctacagcaa aattgcaccg tgcgtcaggt tgctatccct 1320
   gatttgcaaa acacgctgca acagcagggc gttcgtctgg ca 1362
<210> 77 <211> 759 <212> DNA <213> Escherichia coli
<400> 77
<210> 78 <211> 1476 <212> DNA <213> Escherichia coli <400> 78
<210> 79 <211> 954 <212> DNA <213> Escherichia coli <400> 79
<210> 80 <211> 513 <212> DNA <213> Escherichia coli <400> 80
<210> 81 <211> 603 <212> DNA <213> Escherichia coli <400> 81
<210> 82 <211> 702 <212> DNA <213> Escherichia coli <400> 82
<210> 83 <211> 1008 <212> DNA <213> Escherichia coli
<400> 83
   atgcgtatac atacttattg gtatagaaga tatttcattt tattgattat tatattttca 60
   aatgttcttt cttctattgc taatgctgaa gatatggggc gagaacgtgc atattgttat 120
   ccgggttcac cgagtaataa tactacgcct gcatcctttt cttataattt tggtactata 180
   gtggtttctg atgtcaacaa aaatgcgcct ggcactgtat tgccatcaca aatctggaag 240
   gttggaacct ataaggctta ttgtaattct cttgatgatt atgaaattta cttcagtgct 300
   gtctctggaa tagatccgtc tggtgccagt ggtgatcatc aagggagtga tgtatttatt 360
   ccactcaccc atgaaatatc tgtctctact catataaaac tttataatca aaatggcaca 420
   atgacagata aaattgtgcc attcgaaaat tataatacca attatccggg ggacagaagc 480
   aaaccatcta attgggcatc aggtactgaa ggatatatta aaatcaggat tgataaaaaa 540
   attatatctg atgtttcatt aagtaacgta ttattggtgt cattatatgt cagccagatc 600
   cctaccgaac atggtcctat ccctgtcttt aatgcctaca taggaaactt aaatattcag 660
   gttccgcaag gttgcactat taatgagggt acgagtttta ctgttaatat gccggatgtg 720
   tgggccagtg aattgagccg ggctggtgcc ggagcgaagc ccgctggtgt tactcctgta 780
   gcaacaacta ttccgattaa ttgtacgaat aaagatacag atgcggtaat gacgttggta 840
   ttcgacggta acatttccgc cacacgtgat accaatggga aacaaagtat tattcaggca 900
   caagataatc ctgatgttgg tattatgatt atggatagtc agcaaaactc cgtagattta 960
   aatgccctgg caacatcagt aggcgttccg ttcagattgg tggaaaac 1008
<210> 84 <211> 2592 <212> DNA <213> Escherichia coli
<400> 84
<210> 85 <211> 507 <212> DNA <213> Escherichia coli <400> 85
<210> 86 <211> 2139 <212> DNA <213> Escherichia coli <400> 86
<210> 87 <211> 1818 <212> DNA <213> Escherichia coli
<400> 87
   atgaaaatat cgtggaatta tatatttaag aacaaatggc gatttcacat tacaagcatt 60
   tcactttttc ttatcatgct cgcggtttca atcgcttttt tgcacttgcg ttttaatacc 120
   ttgtccagta ccgataaaat gcggcttgaa atgtataagt ccacattata ttccaccatc 180
   gagcaatttt atgttttacc ctatatgctc tcaacagacc atatcatccg tcaggcggta 240
   attacgcctg acgatatgac gtccagcgaa ctcaatcaac gaattgcaca tttcaatact 300
   caactcaaaa ccgcagcaat atttattctg gatacccaag gtaaggccat cgcttctagc 360
   aactggcagg accccggcag ctatgtaggg caaaattata gctatcgccc ctattataaa 420
   cacgccatgt ctggcttaaa tggacgcttt tacggtattg gtagcactac gaatacaccg 480
   ggattcttcc tctctacaag tataaaagat aaaggaaaaa ttgtcggtgt tgtagtagta 540
   aaaataagtc ttaatgaaat tgaaaaagca tgggccgaag gtcctgaaaa tattatcgtg 600
   aatgatgaac atgggattat atttttaagt tcaaaatcgc catggcgaat gcgaacactg 660
   caaccgttac ctgttcaggc aaaacaaaaa ctacaatcta cccgccaata tagtctcgac 720
   aatcttttac cggcggatta ttatccctgt tataccgtga gcaattttac tttcctgaaa 780
   gataaaaaag aacaactctg tttattcccg caatattata cgcaacaaat agccattcca 840
   gaatttaact ggaaaatgac aattatggtc cccttagata acctgtactg gtcatgggct 900
   atttcgttag tcattacact aattatttac ctgctgtttt tgttatttat taaatactgg 960
   agaatgcgat ctcatgcaca acaattatta acacttgcga atgaaacatt agaaaaacag 1020
   gttaaagagc gtacatctgc cctggaattg atcaatcaaa aattaataca ggagataaaa 1080
   gagcgcagtc aagctgaaca agtattacaa attacgcgta gtgaactggc agagtccagc 1140
   aaactggcgg cgcttggaca gatggcaacc gaaattgccc atgaacaaaa tcaaccgtta 1200
   gccgccattc acgcacttac tgataacgcg cgtactatgc taaaaaaaga gatgtatccg 1260
   caggttgaac agaatctgaa acatattatt tcagtgattg agcggatgac gcagctcatt 1320
   tccgaactta aagcatttgc ctcgcgccat cgcgtaccta aaggttctgc cgatgtcatc 1380
   aaagtgatgt atagcgccgt ggcgttactt aatcacagca tggagaaaaa taacattgag 1440
   cgacgaataa aagccccatc catgccgtta tttgtcaatt gcgatgagct cggtcttgaa 1500
   cagatattca gtaatttaat tagcaacgcc ttagattcta tggaaggtag ctcttacaaa 1560
   cgactggata tcgccattcg ccaggcaaat aacaaagtta ttattaccat taaagacagc 1620
   ggtggcggtt ttgcacctga agttgtcgat cgcatatttg aaccattttt taccactaaa 1680
   cgtagaggaa tggggttggg actggcaata gtcagcgaaa ttgtccgaaa ttcgaacggc 1740
   gcactccacg ccagtaatca tcctgaaggc ggcgcagtaa tgacattaac ctggcctgaa 1800
   tggggagaag aacatgaa 1818
<210> 88 <211> 303 <212> DNA <213> Escherichia coli <400> 88
<210> 89 <211> 789 <212> DNA <213> Escherichia coli <400> 89
<210> 90 <211> 1134 <212> DNA <213> Escherichia coli
<400> 90
   atgggaaaaa taaaatattg gctaatagta ggatttatta tactttttgc gattttttac 60
   attgctatta gtgacaggga ttctacgctt tctaggttga aatcagcagg tgaaaacgga 120
   gatgtagaag ctcagtatgc tttggggctc atgtatttgt atggagaaat tctggatgtt 180
   gattatcagc aggcaaagat ttggtatgaa aaagccgctg accaaaatga tccgcgtgcg 240
   caggccaaac tcggtgtgat gtatgcaaat ggtctcgggg taaatcagga ttatcagcaa 300
   tcaaaattat ggtatgaaaa ggcggctgcg caaaatgatg ttgatgcgca atttttgctt 360
   ggggagatgt atgacgatgg tctcggggta agccaagact accagcatgc aaagatgtgg 420
   tatgaaaaag cggctgctca aaatgatgag cgtgctcagg tcaatctcgc tgttctatac 480
   gcaaagggta atggtgttga acaggattat cgacaggcca aaagctggta tgaaaaggct 540
   gcagctcaaa atagtcctga tgcgcagttc gctcttggaa ttctgtatgc caatgctaat 600
   ggtgtagagc aggactatca gcaggcaaaa gactggtatg agaaagcagc agaacaaaat 660
   ttcgccaatg ctcagtttaa tcttggtatg ctctattaca aaggtgaggg tgttaaacaa 720
   aactttcggc aagccagaga atggtttgaa aaagccgcat ctcaaaatca gccgaatgcc 780
   caatataatt taggtcagat ttattactac ggtcagggtg tgactcagag ctatcgacag 840
   gcgaaagact ggtttgaaaa agcggcagag aaaggtcatg tcgatgctca atataatctc 900
   ggtgtaatat acgaaaatgg tgaaggtgtg agtcagaact atcaacaggc aaaggcttgg 960
   tatgaaaagg cagcctcaca aaatgatgcg caggcgcagt tcgaacttgg cgttatgaat 1020
   gaactgggtc agggtgaaag catagacctg aaacaagcaa gacattacta tgagcggtca 1080
   tgtaataatg ggcttaagaa aggttgtgaa cggttaaaag agttattata caaa 1134
<210> 91 <211> 1962 <212> DNA <213> Escherichia coli
<400> 91
   atgaatgtaa tcagaactgt catttgtaca ttaattatac ttccggtggg attacaggca 60
   gcgaccagtc attcttctat ggttaaagat acaatcacca ttgtcgcgac aggaaatcag 120
   aacacggtat ttgaaacgcc gtcgatggtc agtgtcgtca cgaatgacac accgtggagt 180
   cagaatgcgg ttacatcggc cggcatgctg aaaggtgttg ccggtctcag ccagactggt 240
   gcaggacgga ccaatgggca gacctttaat ttacgcggct atgacaaaag cggggtactt 300
   gttcttgttg acggcgttcg ccaactcagt gacatggcaa aaagcagtgg cacttatctg 360
   gatccggcac tcgtcaaacg tatcgaagtt gtccgcgggc caaactccag tctgtacggc 420
   agtggcgggc tgggaggtgt agtggacttc agaactgccg atgcagcaga ttttcttccc 480
   cccggagaga caaacggttt aagtctgtgg ggaaatatcg ccagtggtga ccacagcaca 540
   ggctcggggc tcacctggtt tggtaaaact ggaaaaacag atgcgctcct ttctgtcatt 600
   atgcgtaaaa gaggtaatat ctatcaaagt gatggtgagc acgcacctaa caaggaaaaa 660
   cctgcagccc tgtttgcgaa aggctctgtc ggtataacag acagtaacaa agcaggtgcc 720
   agcttgcgtc tctaccggaa taacaccact gaaccgggca attccactca gacacatggt 780
   gacagcggcc tgcgtgacag aaaaacagta caaaatgacg tacagttctg gtaccagtac 840
   gctcctgtgg ataacagcct catcaatgta aagtcaacgt tatatctcag tgatatcact 900
   atcaagacaa acggtcacaa caaaacggca gaatggagaa acaacagaac ctccggtgtt 960
   aatgttgtca acaggagtca tactctgatt tttccgggag cccatcagtt aagttatggc 1020
   gctgaatatt accgtcagca gcagaagcca gaaggctctg ccacactata tccggaagga 1080
   aacattgact ttacatcgtt gtatttccag gatgaaatga caatgaaaag ctacccggtt 1140
   aacattatcg tcggttcccg ctatgaccgg tacaagagct tcaatccccg tgccggagaa 1200
   ctgaaagccg aacgcctgtc cccaagggcg gcgatttcag tctcaccgac agactggctg 1260
   atgatgtacg gctccatatc ctctgcattc cgagcgccca caatggcaga aatgtacagg 1320
   gatgatgtac atttttaccg caagggtaaa cccaattact gggttcctaa ccttaatctg 1380
   aaaccagaaa ataacatcac ccgtgagatt ggcgcaggta ttcaactgga tggcctgctt 1440
   acagacaatg accggctgca gttaaaaggc ggatatttcg gaacggatgc cagaaactat 1500
   attgccacac gcgtggatat gaaacggatg cgttcttatt cttataatgt atcccgggcc 1560
   cgtatctggg gatgggatat gcagggtaat taccagtctg attatgttga ctggatgctt 1620
   tcttataacc ggacggaaag tatggatgcc agcagcaggg aatggctggg ctccggcaat 1680
   cctgacacac ttatcagtga catcagcata cctgttggtc atagaggcgt ttatgccgga 1740
   tggcgtgctg aactttcagc atcagccacg catgtgaaaa aaggcgatcc ccatcaggct 1800
   ggttatacca tacattcctt ttcactgtct tataagcctg taagtgttaa aggctttgag 1860
   gcgtcagtaa ctctggataa tgccttcaac aagcttgcca tgaatggcaa aggtgtgccg 1920
   ctttcaggca gaactgtcag tctttatacc cgttatcagt gg 1962
<210> 92 <211> 4128 <212> DNA <213> Escherichia coli
<400> 92
<210> 93 <211> 1047 <212> DNA <213> Escherichia coli
<400> 93
   atgattacac tttttcgact actggcgatt ctttgccttt tttttaacgt ttcagctttt 60
   gctgttgatt gctatcagga tgggtacaga ggaacaaccc tcataaatgg agatttacca 120
   acgttcaaaa ttccagagaa tgcgcaacct gggcaaaaaa tttgggagag cggagatatt 180
   aatatcacag tttattgtga caatgcacca ggatggtcaa gtaataaccc atcagaaaat 240
   gtctatgcct ggatcaaatt gccccaaata aatagtgccg atatgttgaa taatccgtat 300
   ttaacatttg gcgtgactta taatggtgta gattatgaag ggacaaatga aaaaattgat 360
   actcatgcgt gcctggataa atatgaacaa tactataatg ggtattatca tgaccctgta 420
   tgcaatggca gcactcttca aaaaaatgta acatttaacg cccattttcg cgtctatgta 480
   aaattcaaaa gccgcccggc aggagatcag acggtaaact ttggcacagt caacgtgctg 540
   caattcgacg gtgaaggcgg ggcgaacatg gcccccaacg cgaaaaattt acgctatgcg 600
   attacggggt tagataatat ttcattcctt gactgtagtg tcgacgtccg catttccccg 660
   gaaagtcaga tagtcaattt tgggcagatc gctgcgaatt ccattgcaac tttcccaccg 720
   aaggcagcat tcagcgtttc taccataaaa gacattgcgt ctgattgtac cgaacagttt 780
   gatgttgcaa ccagtttctt tacttcagat acattatatg acaatacgca tctggaaata 840
   ggtaacggct tgctcatgcg aattactgat caaaaaacgc aagaagatat taaatttaac 900
   cagttcaaat tatttagtac ttatattccc ggtcagagtg cggcaatggc aacccgcgat 960
   taccaggccg aattaaccca aaaacctggt gaaccactcg tctatggccc atttcagaaa 1020
   gacctgatag ttaaaatcaa ctaccac 1047
<210> 94 <211> 2520 <212> DNA <213> Escherichia coli
<400> 94
<210> 95 <211> 507 <212> DNA <213> Escherichia coli <400> 95
<210> 96 <211> 933 <212> DNA <213> Escherichia coli <400> 96
<210> 97 <211> 2166 <212> DNA <213> Escherichia coli
<400> 97
<210> 98 <211> 957 <212> DNA <213> Escherichia coli <400> 98
<210> 99 <211> 1887 <212> DNA <213> Escherichia coli
<400> 99
   atgaagaaag tgctcactct ctcactactg gctctgtgtg tgtctcatag tgcagtagca 60
   gcaaactata cgttcaataa cgataatatt gccctctcgt ttgatgatac aaactcgacg 120
   attgtgctga aggaccgtag aactaaccat ccgatcacac cacaggaatt gttctttctg 180
   acactaccgg atgagacaaa aatccacacc gcagatttca aaatcaagca catcaaaaaa 240
   caggacaatg cgattgtcat cgactttacg cgcccagatt ttaacgtaac agtgcagttg 300
   aaccttgtga agggaaaata tgccagcatc gactacacta ttgccgccgt tgggcaacca 360
   cgagacgtcg ccaagattac cttcttcccg accaaaaaac agtttcaggc tccttacgta 420
   gacggcgcaa tcactagctc accgatcatt gcggactcgt tctttatcct gccgaataaa 480
   ccgatcgtga atacctacgc ctatgaagca acaaccaatc tcaacgtaga actgaaaact 540
   ccaattcagc cagagacgcc ggttagcttt accacctggt tcggtacttt cccggaaacc 600
   agccagttgc gacgcagtgt gaaccagttt attaatgccg tacgtccacg tccgtacaag 660
   ccttatttgc attacaacag ttggatggat atcggctttt tcactccgta caccgaacag 720
   gatgttctgg gacgcatgga cgaatggaac aaggaattca ttagcggccg cggagtggcg 780
   ttagacgctt ttctgctgga cgatggctgg gacgatctta ccggacgctg gttatttggc 840
   ccggcattca gcaacggttt tagcaaagta cgagagaaag ccgatagcct gcacagctcc 900
   gttgggctat ggctttcacc gtgggggggt tacaataagc cgcagcgacg ttcgcgtttc 960
   gcatgcaaaa gagtatgggt tcgaaaccgt ggacggcaag ctggcgcttt cgggagcgaa 1020
   ctacttaaaa acttcaatga gcagatcatt aatcttatca aaaatgaaca cattacctcg 1080
   tttaaactcg acggaatggg gaacgccagt tcacatataa agggtagccc gttcgcctcg 1140
   gattttgatg cgtcaatagc tctgctgcac aatatgcgca gagcaaaccc gaatctattt 1200
   atcaacctga ccaccggcac caacgccagc ccgtcctggt tgttctatgc tgattctatc 1260
   tggcgtcagg gggatgatat aaacctgtat ggccccggca cgccggtgca gcagtggata 1320
   acatatcgtg atgccgagac ataccgctct attgtacgta aaggcccgct attcccgctg 1380
   aactcgctga tgtaccacgg gatagtcagc gccgagaatg cctattacgg gttagagaag 1440
   gtgcaaacgg acagcgactt tgccgatcag gtctggagct acttcgcgac cggcacccag 1500
   ctgcaggagc tgtatattac cccgtccatg ctgaacaagg tgaagtggga tacgctggcg 1560
   aaggctgcaa aatggtcgaa ggaaaatgcc agcgtgctgg ttgataccca ctggattggc 1620
   ggcgacccaa cggcgcttgc cgtgtacggc tgggcatcct ggagcaaaga caaagccatt 1680
   ctcggtttgc gcaacccatc ggataagcca cagacctact atctggattt ggcgaaggat 1740
   ttcgaaatac cggcaggaaa cgcggcgcag tttagtctga aagcggtata cggcagcaat 1800
   aaaacagtgc ccgttgagta taaaaacgcg acggtgatta cgttgcagcc gctggaaacg 1860
   ctggtgtttg aggcggtgac cattaac 1887
<210> 100 <211> 5334 <212> DNA <213> Escherichia coli
<400> 100
<210> 101 <211> 681 <212> DNA <213> Escherichia coli
<400> 101
   atgaacctaa agaaaacact gttaagcgtg ttaatgatat tgcaactttg cttattggta 60
   gggtgtgact atattgaaaa agcgagtaag gtcgacgatc tcgttacaca gcaagagttg 120
   caaaaaagca aaattgaggc gcttgaaaaa caacaagaac tcgacaagcg caagatagaa 180
   cactttgaaa aacaacaaac taccatcata aacagtacca aaacgctcgc tggtgtggtg 240
   aaggcagtta aaaacaaaca ggacgaattt gtctttacag aatttaaccc ggcacaaacc 300
   caatacttta ttttaaataa cggctctgtt ggtttggcag ggaaaatact gtctattgac 360
   gcagtagaaa acggcagtgt tattcgtatt tcactggtta acttattaag tgttcctgta 420
   tcaaatatgg gtttctacgc aacatggggg ggagaaaaac ccaccgacat caacgcatta 480
   gcaaaatggc agcaattgct atttagtacc gcaatgaact cctccctgaa attattacca 540
   ggtcaatggc aagacattaa tttgacgcta aaaggtgtct cgcccaacaa cctcaaatat 600
   ctgaaattag ccatcaacat ggcaaatatt cagttcgacc gtcttcaacc tgctgaatct 660
   ccacagcgga aaaacaaaaa a 681
<210> 102 <211> 3327 <212> DNA <213> Escherichia coli
<400> 102
<210> 103 <211> 534 <212> DNA <213> Escherichia coli <400> . 103
<210> 104 <211> 840 <212> DNA <213> Escherichia coli
<400> 104
   atgatttcag ggggaaatat gttgaaagaa tggatgatat ttacgtgcag tttattgact 60
   ctggctgggg cgtcactgcc cctcagtggc tgtatttcca gaggccagga gtctatatcc 120
   gaaggggcgg catttggggc agggatcctg cgcgaaccgg gagcaacaaa aaaagccgac 180
   acgaaagacc tcaatgtgcc accaccggtt tatggtccgc cgcaggtgat atttcgcatt 240
   gatgacaacc gctatttcac gctagaaaat tatacccact gcgagaacgg gcagacgttt 300
   tataataata aagcaaaaaa cattcatgtt aaaatattag acgcttcagg gtatttattt 360
   aaaggccgct tattctggtt atcaacgcgt gatgattttc tggcctttcc tgccacgtta 420
   aataccagac acgcttcctg tatggggtcg aataaaggct gtatgaatgc ggtcattgtc 480
   actaccgatg gtggaaaaag acgcagtggt gtgccatacg gcagttatac ccagaatccg 540
   accggtgcca cgagggatta tgacatgctg gtgatgaatg acggcttcta cctgcttaga 600
   tatcgggggg gacagggcag atttagtccg gtgatactta gatggattct cagtactgaa 660
   gatagctctg gtgttgtgcg ttcagaagat gcttatgaat tgttccgtcc cggagaagag 720
   gtaccctcca ccggttttta taaaatcgac ctgtcacgtt tttatcccaa aaacaacgtt 780
   atggaaatgc agtgtgacag gacgctggag ccagttcaac cttcagagag taaaattcaa 840
<210> 105 <211> 1503 <212> DNA <213> Escherichia coli
<400> 105
<210> 106 <211> 2046 <212> DNA <213> Escherichia coli
<400> 106
<210> 107 <211> 492 <212> DNA <213> Escherichia coli
<400> 107
<210> 108 <211> 654 <212> DNA <213> Escherichia coli <400> 108
<210> 109 <211> 8198 <212> DNA <213> Escherichia coli
<400> 109
   atgcatcagc ctcccgttcg cttcacttac cgcctgctga gttacctggt cagtgcgatt 60
   atcgccgggc agccgttgtt accggctgtg ggggccgtca tcaccccaca aaacggggcc 120
   ggaatggata aagcggcaaa tggtgtgccg gtcgtgaaca ttgccacgcc gaacggggcc 180
   gggatttcgc ataaccggtt tacggattac aacgtcggga aggaagggct gattctcaat 240
   aatgccaccg gtaagcttaa tccgacgcag cttggtggac tgatacagaa taacccgaac 300
   ctgaaagcgg gcggggaagc gaagggtatc atcaacgaag tgaccggcgg taagcgttca 360
   ctgctgcagg gctatacgga agtggccggc aaagcggcga atgtgatggt tgccaacccg 420
   tatggtatca cctgtgacgg ctgtggcttt atcaacacgc cgcacgcgac gctcaccacg 480
   ggcaaacctg tgatgaatgc cgacggcagc ctgcaggcgc tggaggtgac tgaaggcagt 540
   atcaccatca atggcgcggg cctggacggc acccggagcg atgccgtatc cattattgcc 600
   cgtgcaacgg aagtgaatgc cgcgcttcat gcgaaggatt taactgtcac tgcaggcgct 660
   aaccgtgtaa ctgcagatgg tcgtgtcaga gccctgaagg gcgaaggtga tgtgccgaaa 720
   gttgccgttg ataccggcgc tctcggtgga atgtacgcca ggcgtattca tctgacctcc 780
   actgaaagtg gtgtcggggt taatcttggt aacctttatg cccgcgatgg cgatatcacc 840
   ctggatgcca gcggcagact gactgtcaac aacagtctcg ccacggggac cgtcactgca 900
   aaaggtcagg gcgtcacctt aaccggcgac cataaagcgg gaggtaacct gagcgtcagc 960
   agccggagag atatcgttct cagcaatgga acgcttaaca gcgacaagga cctcagcctg 1020
   accgccggcg gcagaatcac tcaacagaat gaaaaactga ctgccggccg ggatgtaacg 1080
   cttgccgcga aaaacatcac acaggatacc gccagccaga ttaacgcggc ccgcgatatc 1140
   gtgactgtcg ccagtgacac gctgacaaca cagggacaga taaccgccgg gcagaatctc 1200
   acggccagcg ccaccacgct gacgcaggac ggaatattgc tggcgaaaag tcatgcggga 1260
   ctcaatgccg gtacgctgaa taacagtggc gccgttcagg gagctaccct gacgctcggc 1320
   agtacaacgc tcagcaacag tggctccctg ctcagtggcg gtcccctgac catgaatacc 1380
   cgcgacttta cccagagcgg ccgcactggc gcgaagggca aagtggatat catggccagt 1440
   gggaaactga ccagtacagg tttgctggtg acgatgcact tggtgctgaa ggcgcaggat 1500
   gtgacacaga acggtgtgct gtccggcggc aaagggctga cggtcagtgc gacgagctcc 1560
   ggtaaaaaat cggtcaccca cagcgatgct gcgatgacgc tgaatgtgac aacagtggcg 1620
   ctggacgggg aaaccagtgc cggtgacacc ctccgggttc aggcagacaa actgagtacc 1680
   gcagcgggcg cacaacttca gagcggcaaa aatctcagca tcaacgccag agatgcacgt 1740
   cttgcaggta cgcaggcagc acaacagacc atggtggtga acgccagtga aaagctcacc 1800
   cacagcggga aaagcagtgc cccgtcgctc agcctcagtg cgccggaact gaccagcagc 1860
   ggcgtacttg ttggttccgc cctgaataca cagtcacaga ccctgaccaa cagcggtctg 1920
   ttgcaggggg aggcctcact caccgttaac acacagaggc ttgataatca gcagaacggc 1980
   acgctgtaca gtgctgcaga cctgacgctg gatataccgg acatccgcaa cagcgggctt 2040
   atcaccggtg ataatggttt aatgttaaat gctgtctccc tcagcaatcc gggaaaaatc 2100
   atcgctgaca cgctgagcgt cagggcgacc acgctggatg gtgacggcct gttgcagggc 2160
   gccggtgcac tggcgcttgc tggcgacacc ctctcacagg gtagtcacgg acgctggctg 2220
   acggcggacg acctctccct ccggggcaaa acactgaata ccgcagggac cacgcaggga 2280
   cagaatatca ccgtgcaggc ggacagatgg gcgaacagtg gttccgtgct ggcaaccggt 2340
   aaccttactg cttcggcaac cggtcagttg accagtaccg gcgatatcat gagccagggt 2400
   gacaccacgc tgaaagcagc caccacggac aaccggggca gtctgctttc ggccggcacg 2460
   ctctcccttg atggaaattc actggataac cgcggcactg tccagggtaa ccatgtcacg 2520
   attcgccaga acagtgtcac caacagtggc acgctcaccg ggatcgccgc actgacgctt 2580
   gccgcccgta tggcatcccc tcaacctgcg ctgatgaata acggaggttc attgctgacc 2640
   agcggcgatc tgacaatcac cgcaggcagt attaccagtt ccggacactg gcagggcaaa 2700
   cgggtgctga tcaccgcaga cagtctggca aacagcgggg cgatccaggc ggctgacagc 2760
   ctgactgcac gtctgacggg tgagctcgtc agcacagcgg gcagcaaagt cacctcgaac 2820
   ggtgaaatgg cgctcagtgc actgaattta agcaacagcg gacaatggat tgcaaaaaat 2880
   ctgaccctga aggcgaactc actgaccagt gcgggtgaca tcaccggtgt ggatactctc 2940
   acgctcacgg tgaatcagac gctgaacaat caggcgaacg gaaaactgct cagtgcaggt 3000
   gtgctgacgc tgaaggcaga cagtgtcaca aacgacgggc aattacaggg aaatgtcacc 3060
   accatcacgg caggacaact cacaaacggc gggcatctgc agggcgaaac gctgacgctg 3120
   acagcctccg gtggcgtgaa caaccgttcc ggtggtgttc tgatgagccg gaatgcactg 3180
   aatgtcagta ctgcgaccct gagtaaccag agcacgatac agggtggagg cggggtttcc 3240
   ctgaacgcca cagaccgtct gcagaacgac ggcaaaatcc tctccggcag taacctcacg 3300
   ctgacggcgc aggtgctggc gaacaccggc agcggactgg tacaggctgc caccctgctg 3360
   ctggatgtgg tgaatactgt caacggcgga cgcgtacttg ccaccggcag tgacgttaaa 3420
   ggaaccacgc tgaataatac cggtacgctt cagggtgcga ctctggtgaa ttaccacaca 3480
   ttcagcagcg gtaccctgct gggaacctcc gggcttggcg tcaagggcag ttcactgctg 3540
   caaaatggta cagggcggct gtacagtgca ggcaacctgc tgcttgacgc tcaggacttc 3600
   agtggtcagg ggcaggtggt ggccaccggt gatgtcacac tgaaactgat tgctgccctc 3660
   acgaatcatg gtaccctggc cgcagggaaa accctttccg tcacgtcgca aaatgccatc 3720
   accaacggcg gtgtcatgca gggtgatgcc atggtgctcg gtgccggaga ggcattcacc 3780
   aacaatggac tgactgccgg taaaggcaac agtgttttca gcgcacagcg tcttttcctt 3840
   aacgcaccgg gttcacttca gggcggtggc gatgtgagtc tgaacagccg gagtgatatc 3900
   accatcagtg gttttaccgg cacggcaggc agtctgacaa tgaatgtggc cggtaccctg 3960
   ctgaacagtg cgctgattta tgcggggaat aacctgaagc tgtttacaga ccgtctgcat 4020
   aaccagcatg gtgatatcct ggccggcaac agtctgtggg tacagaagga tgcttccggc 4080
   ggtgcaaaca cagagattat caataattcc gggaatattg agacgcatca gggcgatatt 4140
   gttgtaagaa ccgggcatct tctgaaccag cgggagggat tttctgccac aacaacaacc 4200
   cggactaacc cctcatccat tcagggaatg ggaaatgctc tggttgatat tcccctttcc 4260
   cttcttcctg acggcagcta tggctatttc acccgtgaag ttgaaaatca gcacggtacg 4320
   ccctgcaacg ggcacggggc atgcaatatc acaatggata cgctttatta ttacgcgccg 4380
   tttgctgaca gtgccacaca gcgctttctc agcagccaga acatcacaac agtaaccggt 4440
   gctgataatc cggcaggccg cattgcgtca gggcgtaatc tttctgctga ggctgaacga 4500
   ctggaaaacc gggcgtcatt tatcctggcg aatggggata tcgcactctc gggcagagag 4560
   ttaagcaatc agagctggca gacggggaca gagaatgaat atctggtata ccgctacgac 4620
   ccgaaaacgt tttacggtag ctatgcaaca ggctctctgg ataaactgcc cctgctgtca 4680
   ccggaatttg aaaacaatac catcagattt tcactggatg gccgggaaaa agattacacg 4740
   cccggtaaga cgtattattc cgttattcag gcgggcgggg atgttaagac ccgttttacc 4800
   agcagtatca ataacggaac aaccactgca catgcaggta gtgtcagtcc ggtggtctct 4860
   gcacctgtac tgaatacgtt aagtcagcag accggcggag acagtctgac acagacagcg 4920
   ctgcagcagt atgagccggt ggtggttggc tctccgcaat ggcacgatga actggcaggt 4980
   gccctgaaaa atattgccgg aggttcgcca ctgaccggtc agaccggtat cagtgatgac 5040
   tggccactgc cttccggcaa caatggatac ctggttccgt ccacggaccc ggacagtccg 5100
   tatctgatta cggtgaaccc gaaactggat ggtctcggac aggtggacag ccatttgttt 5160
   gccggactgt atgagcttct tggagcgaag ccgggtcagg cgccacgtga aacggctccg 5220
   tcgtataccg atgaaaaaca gtttctgggc tcatcgtatt ttcttgaccg cctcgggctg 5280
   aaaccggaaa aagattatcg tttcctgggg gatgcggtct ttgatacccg gtatgtcagt 5340
   aacgcggtgc tgagccggac gggttcacgt tatctcaacg gactgggttc agacacggaa 5400
   cagatgcggt atctgatgga taacgcggcc agacaacaga aaggactggg attagagttt 5460
   ggtgtggcgc tgacagctga acagattgct cagcttgacg gcagcatact gtggtgggag 5520
   tcagtcacca tcaacggaca aacagtcatg gtcccgaaac tgtatctgtc gccggaagat 5580
   atcaccctgc ataacggcag cgttatcagc gggaacaacg tgcaacttgc gggcggcaat 5640
   atcaccaaca gcggcggcag catcaacgca cagaacgacc tctcgctcga cagttccggc 5700
   tatatcgaca acctgaatgc ggggctgata agcgcgggcg gtagcctgga cctgagcgcc 5760
   atcggggata tcagcaatat cagctcagtc atcagcggta aaaccgtaca actggaaagc 5820
   gtgagtggca acatcagcaa tatcacccgg cgtcagcaat ggaatgcggg cagtgacagc 5880
   caatatggtg gtgtgcatct cagcggtacg gacaccggtc cggttgcgac cattaaaggc 5940
   actgattcac tttcgctgga tgcagggaaa aacattgata ttaccggggc aacggtctcg 6000
   tccggtggag accttggaat gtctgcgggt aatgatatca acattgccgc aaacctgata 6060
   agtgggagca aaagtcagtc cggtttctgg cacactgatg acaacagttc atcatccacc 6120
   acctcacagg gcagcagcat cagcgccggc ggtaacctgg cgatggctgc aggccataat 6180
   ctggatgtca cggcatcctc tgtttctgcc gggcacagcg ccctgctttc ttgcaggtca 6240
   cgacctagtc ttgaatgcag tcagggaaaa gcaaaaacaa gtcgcaacgg caggtcagaa 6300
   agtcatgaaa gccacgcagc tgtgtccacg gtgacagcgg gcgataactt cctccttgtt 6360
   gccggtcgtg atattgccag tcaggctgcc ggtatggctg cggaaaataa cgtggtcatc 6420
   cggggcggac gtgatgtgaa cctggtggca gagtctgccg gcgcaggcga cagctatacg 6480
   tcgaagaaaa agaaagagat taacgagaca gtccgtcagc agggaacgga aatcgccagc 6540
   ggtggtgaca ccaccgtcaa cgcaggacgg gatatcaccg ctgttgcgtc atccgttacc 6600
   gcaaccggca atatcagcgt gaatgccggt cgtgatgttg ccctgaccac ggcgacagaa 6660
   agtgactatc actatctgga aacgaagaaa aaaagcggag gttttctcag taagaaaacc 6720
   acccgcacca tcagtgagga cagtgccacc cgtgaagcag gctccctgct gtcggggaac 6780
   cgcgtgaccg ttaacgccgg tgataacctg acggtagagg gttcggatgt ggtggctgac 6840
   cgggatgtgt cactggcggc gggtaaccat gttgatgttc ttgctgccac cagtacagat 6900
   acgtcctggc gctttaagga aacgaagaaa tccggtctga tgggtaccgg cggtattggt 6960
   ttcaccattg gcagcagtaa gacaacgcac gaccggcgcg aggccgggac aacgcagagt 7020
   cagagtgcca gcaccatcgg ctccactgcc ggtaatgtca gtattaccgc gggcaaacag 7080
   gctcatatca gcggttcgga tgtgattgcg aaccgggata tcagcattac cggtgacagt 7140
   gtggtggttg acccggggca tgaccgtcgt actgtggacg aaaaatttga gcagaagaaa 7200
   agcgggctga cggttgccct ttccggcacg gtgggcagtg ccatcaataa tgcggttacc 7260
   agtgcacagg agacgaagga gagcagtgac agccgtctga aagccctgca ggccacaaag 7320
   acagcgctgt ctggtgtgca ggccggacag gctgcgacaa tggcctccgc aaccggtgac 7380
   ccgaatgcgg gagtcagcct gtcgctcacc acccagaaat cgaaatcaca acaacattct 7440
   gaaagtgaca cagtatccgg cagtacgctg aatgccggga ataatctgtc tgttgtcgca 7500
   accggcaaaa acaggggcga taaccgcgga gatattgtga ttgcaggaag ccagcttaag 7560
   gccggtggta acacaagcct ggatgccgcg aatgatattc tgttgagtgg cgccgcaaac 7620
   acacaaaaaa caacgggcag gaacagcagc agtggcggtg gcgtgggtgt cagtatcggt 7680
   gcaggtaaag gtgccggtat cagcgccttt gccagcgtta atgcggcaaa aggcagggag 7740
   aaaggtaacg gtactactac cgacaaaacc gtcaccatca acagtggtcg ggatacggta 7800
   ctgaacggtg ctcaggtcaa cggcaacagg attatcgccg atgtgggcca cgacctgctg 7860
   ataagcagcc agcaggacac cagtaagtac gacagtaaac agaccagcgt ggctgccggc 7920
   ggcagtttta cctttggctc catgaccggc tcaggttaca tcgctgcctc ccgggataag 7980
   atgaagagcc gctttgactc cgttgctgaa caaaccggaa tgtttgcccg ggtgatggtg 8040
   gcttcgacat cacagtgggt aaacataccc aactggatgg tgcggtcatt gcctcactgc 8100
   cacaccggag aaaaaccacc tggataccgg acgctgggtt tagtgacttt acaacgaagc 8160
   gggattataa agtcaagtca caggtggaat cagtctga 8198
<210> 110 <211> 963 <212> DNA <213> Escherichia coli
<400> 110
   atgatgttga agaaaacgat atttatatta acgttattct ctggcaacgt aattgctgca 60
   actgtagaat taggttttga aaatgagcaa tataattatg cttatcgttc tgcagatgtc 120
   ttcatgccgt atattaagag taatttcaac cctgttactg attctgcttt gaatgtgtca 180
   ctcacctata tgtatcagga tcaatatggg aaaaaacata aaaaaacatc tgaggacaga 240
   tttaaaacca atcgcgatcg catagagctc tatcttaaag gttatacttt aaatagggga 300
   gcatattctt tttctccttc cgcaggtttc cgttatgagt catgggatgt aaactacgat 360
   aatccgaaaa agcaggataa gtggaaactg gaactacgct tttatcctaa tatgacttat 420
   aaactcaatg accagttaag cctatatatg aatggttttg ttgcccctgt attttttaaa 480
   acacaacaag agtcgagaaa agataacaat tatgtaaagg gtaagttagg ggcgaaacgt 540
   tataacaacg attattatca ggaactccag attctgggtg tcagatataa atttaataat 600
   gataatacgc tctgggcatc agtctataat gaaagaaaat ataatcaaca ttcctcaaaa 660
   tatgatcgct ggcaattgcg tggaggctat gatttaaaag ttacagagga gtttgttttg 720
   agtccattca taagatatga cctctcttat agagaaaaaa acctcgaaag cacaagtaat 780
   aatggtttat caaaaaataa taaagaaatt cgaactggag ccagcttttc ctataaaatt 840
   atcccttctg taaaactggt aggagaaata tacaggcaaa caaccaacat tgaaaactat 900
   tatggagagc attctgaaga caaaaaccgc atgttctaca aacttggtat aaacaaaaca 960
   ttt 963
<210> 111 <211> 1761 <212> DNA <213> Escherichia coli
<400> 111
<210> 112 <211> 2220 <212> DNA <213> Escherichia coli
<400> 112
<210> 113 <211> 408 <212> DNA <213> Escherichia coli
<400> 113
   atgaaaactt ttatcaaaac tttactcgtt gctgtaacta ttctgttctc tgtcttcgct 60
   acggcgaaac aagtaaaact gccaaacaac atcaaatacg ttaatactac agaggcgttt 120
   tcctgtactg agattgacgg tatgaattgc cagacgaaga atccgtttaa ctataaagat 180
   aacagctatg ttttcgtgct tgaacgtggt ggtgcctggt gttacgacta cactgtctcg 240
   gtacttaacc tgaaaaccgg gaaagcacag atgctcgaat acaaagacaa ccagctgtgc 300
   tcaggtagca acaaaccgtt cttcgaaatc aaaaatggcg taccgacggt aggagtcatc 360
   gacacatccg gaaaacctgt cgttgtggct ctggacaaac ttaaaacc 408
<210> 114 <211> 675 <212> DNA <213> Escherichia coli <400> 114
<210> 115 <211> 2163 <212> DNA <213> Escherichia coli
<400> 115
   atgaaaactc aaataacttt cgctgcgctt ttgccagcat tagcgtcttt cataccgctt 60
   catgctcatg cctcgtctac ttctgaagat gaaatgattg tcacgggcaa caccgccgcc 120
   gacaccaccg attctgccgc cggtgccggt ttcaaaacga acgatataga tgtcggcccg 180
   ctgggaacga aatcctggat cgaaacacca tattccagca ccactgttac taaagagatg 240
   attgaaaatc agcaggcgca aagcgtcagc gagatgctga aatactctcc cagtacgcaa 300
   atgcaggcgc gcggtggaat ggatgtcggg cgtccgcaaa gtcgggggat gcagggcagc 360
   gtggtggcca acagccgtct ggacgggctg aatatcgttt caacaaccgc gtttccggtg 420
   gaaatgcttg agcgcatgga tgtgcttaac agtttgaccg gcgcgctgta cggcccggcg 480
   agcccagcag ggcagtttaa tttcgtggcg aagcgcccaa ccgaagagac gctgcgtaaa 540
   gtgacgctgg gctatcaaag ccgcagtgcg tttaccggcc atgccgatct gggtggccat 600
   tttgatgaaa acaaacggtt tggctatcgc gtgaacctgc ttgatcagga aggggaaggc 660
   aatgtggatg acagcacgct gcgtcgcaaa ctcgtttccg ttgcgctcga ctggaatatt 720
   cagccgggca ctcagctaca gctcgacgcc agccattacg aatttatcca gaaaggctat 780
   gtcggtagct ttaactatgg gccgaacgtc aaactgccgt ctgcgccgaa tccgaaggac 840
   aaaaatctgg cgctcagcac tgcgggcaac gacctcacta ccgataccat cagcactcgc 900
   ctgatccact actttaacga cgactggtcc atgaacgctg gcgtgggctg gcagcaggct 960
   gaccgcgcga tgcgtagtgt ttccagtaaa atactcaaca atcagggcga tatctctcgt 1020
   tcgatgaagg attccaccgc tgccggacgt tttcgcgtcc tgagcaacac cgccgggctg 1080
   aatggtcata ttgataccgg ctctatcggc cacgatctgt cactttctac cacgggatat 1140
   gtctggtcgc tttatagtgc caaaggaaca ggttccagct atagctgggg tacaacaaat 1200
   atgtatcacc cggatgcgat agatgagcag ggcgatggca aaatccgcac cggcgggccg 1260
   cgataccgct ccagcgtaaa tactcagcag agcgttacgc tcggcgatac ggtgacattt 1320
   acgccgcagt ggtcggcaat gttctatctc agccagagct ggctgcagac taaaaactac 1380
   gataagcacg gtaatcaaac gaaccaggtt gatgaaaatg gtttaagtcc gaacgccgcg 1440
   ctgatgtata aaattacccc taacacaatg gcctacgtta gctatgccga ttcgctggag 1500
   cagggcggta ccgcaccgac ggatgagagc gtaaaaaatg ccggtcaaac gctaaacccg 1560
   tatcgcagca agcagtatga agtggggcta aaatcggaca tcggcgagat gaatctaggc 1620
   gccgcgctgt tccgactgga acgtccgttt gcctatcttg atacggataa cgtgtataaa 1680
   gagcagggta accaggttaa caacggcctt gagttaaccg ctgccgggaa tgtgtggcag 1740
   gggctgaata tttacagcgg cgtgaccttc ctcgacccga aactgaaaga tacggcgaat 1800
   gcctcaacca gcaataaaca ggttgtcggc gtgccgaaag tgcaggccaa tctgttggcg 1860
   gaatacagtt tgccgtccat accggaatgg gtttacagcg ctaacgtcca ttatacgggc 1920
   aaacgcgcgg cgaacgatac caacacctct tacgccagca gctataccac atgggatttg 1980
   ggaacgcgtt acaccacgaa agtgagcaac gtcccaacca ctttccgcgt ggtggtaaac 2040
   aacgtgtttg ataaacatta ctgggcttct atcttcccat cgggtaccga tggcgataac 2100
   ggttccccaa gtgcgtttat cggcggcggc cgcgaagtgc gtgcatccgt caccttcgat 2160
   ttc 2163
<210> 116 <211> 2007 <212> DNA <213> Escherichia coli
<400> 116
<210> 117 <211> 2259 <212> DNA <213> Escherichia coli
<400> 117
<210> 118 <211> 399 <212> DNA <213> Escherichia coli
<400> 118
   atgtcttcga aaacaaaatg ctggctatgg atgttactgg tcatcctttc tgaaacctct 60
   gcaacatcca cacttaaaat gttcgataac agtgagggga tgacaaaaac gctgctgctg 120
   gccctaatcg tcgtactgta ttgcatttgt tactactcgc tttcacgggc agtaaaagat 180
   atccccgttg gtctggctta cgccacatgg tccggtactg gcattttgat ggtttcaacc 240
   cttgggattt tattttacgg tcaacacccg gataccgccg ccattattgg tatggtcatc 300
   atagccagcg gtattatcat tatgaatctg ttctcaaaaa tgggcagtga agaggcggaa 360
   gaaactccag ttaccaacct cgataaaaaa atcgctaac 399
<210> 119 <211> 858 <212> DNA <213> Escherichia coli
<400> 119
   atgtatataa aaaagcactg gatagcttta tccattctat taataccttg cattggaaac 60
   gctcaggaaa ttaaaattga tgaaagctgg ttacatcaaa gcttgaatgt cattggtcgc 120
   acagactctc gctttggccc aagactgact aacgacctct accctgaata tactgtagca 180
   ggaagaaaag actggtttga tttttatggt tatgttgatc taccgaaatt ctttggcgtc 240
   ggcagtcact atgatgttgg gatctgggat gagggctcac cactatttac ggaaatagaa 300
   cctcggtttt ccattgacaa attgaccgga ttaaatcttg cgttcggccc atttaaagaa 360
   tggttcattg caaacaacta tgtctatgat atgggtgaca accagtcatc ccggcaaagt 420
   acatggtata tggggcttgg tacagatatc gacacgggtc taccaattaa gctttctgcc 480
   aatatatacg ccaagtatca gtggcaaaac tatggtgccg ctaatgaaaa tgaatgggac 540
   ggatatcgat tcaaaataaa atatagcatc cctcttacaa atttattcgg aggacgattg 600
   gtatacaata gttttactaa ctttgatttt ggctccgatc ttgcggacaa gtcacacaat 660
   aataaacgaa ccagtaatgc tattgcttca agccatatcc tttcccttct atatgaacac 720
   tggaaatttg catttacact acgttatttt cacaacggtg gacaatggaa tgcgggagag 780
   aaggttaact tcggagatgg tccatttgaa ttaaaaaata caggatgggg aacctatact 840
   actattggtt atcaattt
   858
<210> 120 <211> 516 <212> DNA <213> Escherichia coli
<400> 120
   atgagaatcg caccgcgtac cttctttgct atttccgccc tggcgtttat tgtcgcctcc 60
   ggatttagtt tctggcggtt gtcccctgct gaaaatacag ggattatgag ttgttcaaca 120
   aaaggcatca tgcgttttga gaatatggaa aaggagaacg ttaacggtaa tattcacttt 180
   aactttggca gccagggtaa aggttcgatg gtgctcgaag gctacacgga ctctgccgct 240
   ggctggctgt acctgcaacg ctatgtcaaa tttacctata ccagtaaacg tgtttccgcc 300
   acggaacgcc attaccgcat cagccagtgg gaatccagcg cctcatcgat agatgaatca 360
   ccagatgtga tttttgacta ctttatgcgt gaaatgtctg acagccatga cgggctgttc 420
   ctcaacgccc agaagctgaa cgataaagcg attttgctca gttctattaa ttcaccgctt 480
   tggatctgta cccttaaatc tggcagcaaa ttagac 516
<210> 121 <211> 546 <212> DBA <213> Escherichia coli
<400> 121
   atgaaaataa aagttatagc attggctaca tttgtttctg ctgtgtttgc tggttcagct 60
   atggcctatg acggaacaat tacgtttacc ggtaaagttg tagctcagac ctgcacagtt 120
   aatacaagcg acaaagactt agcagtaact ttacccactg ttgccacttc atctctaaaa 180
   gacaatgctg ctacgtcagg gctgacaccc tttgccattc gtttaactgg ttgtgcaact 240
   ggtatgaata gtgctcagaa tgttaaagcg tactttgagc cttcaagtaa cattgactta 300
   gctacacata atttaaaaaa tactgctact ccaactaaag cggataatgt acagattcag 360
   ttgctaaata gtaatggaac ttcaactatt cttttggggg aagcggataa tgggcaagat 420
   gtccagtctg agacaatcgg atctgatgga agtgccacat tgcgttatat ggcccagtat 480
   tatgcaacag gacaatctac cgcaggggat gtaaaagcga cggtccatta taccattgcc 540
   tacgaa
   546
<210> 122 <211> 1077 <212> DNA <213> Escherichia coli
<400> 122
   atgaaaagaa tctttttcat accattgttt ttaattttac tccctaagct ggcggtagcg 60
   ggtccggatg attatgtgcc ttcgcagata gcggttaata catccacatt gccaggtgtt 120
   gtgattggtc ctgctgatgc ccatacctat ccccgggtga taggagagct ggcgggaaca 180
   agtaaccagt atgtttttaa tggcggtgcc atcgctctga tgcgtggaaa gtttacaccc 240
   gcactgccta aaattggaag tattacggta tactttccat caaggaaaca gcgtgattca 300
   tctgattttg atatctatga tattggtgta tccggactgg gtattattat tggcatggcg 360
   ggctattggc ccgcaacgcc tctggtcccc ataaatagct caggtatata tattgaccct 420
   gtaggtgcca atacaaaccc caatacttat aacggtgcga cagcaagctt cggagctcgt 480
   ttgtttgttg cttttgtcgc aacgggaaga ttacccaatg gatatataac aatacccacc 540
   aggcagcttg gtactatttt gttggaagca aaacgtacaa gtttaaataa taaaggactg 600
   acagcacctg ttatgttaaa tggtgggcgc attcaggtac agagtcagac atgtaccatg 660
   gggcaaaaaa actatgtggt gccattaaat accgtatatc aatcacagtt cacatctttg 720
   tataaagaaa tacagggagg taaaattgac atacacctac aatgcccgga tggaattgat 780
   gtttatgcta cattgacaga tgcatcacag ccagtgaaca gaacagatat attgacctta 840
   agcagtgaat ccactgcaaa aggatttggc atcaggctat ataaagacag tgatgtaact 900
   gccatcagct atggtgaaga ctcccctgtg aaaggaaatg gcagtcaatg gcacttctcc 960
   gattacaggg gagaggtaaa tccacatatc aatttaagag ccaattatat aaaaattgct 1020
   gatgcaacta cacctggaag tgtgaaggct attgcaacta ttactttctc atatcaa 1077
<210> 123 <211> 2532 <212> DNA <213> Escherichia coli
<400> 123
   atgaacgcta ataatctgtc atgcctgatt tactgtcgtt gttctcttct gctttttgct 60
   gcattagggt taacagtaac aaaccattca tttgctgctg aagaggctga gtttgattct 120
   gagtttttgc atttggataa agggataaat gctattgata tccgccgctt tagtcatggt 180
   aaccctgtgc ctgagggcag gtattattct gatatttatg ttaataatgt atggaagggg 240
   aaggctgatt tgcagtattt acgtactgcc aataccggtg ctccgacgtt atgcctgacg 300
   cctgagctgc tttcattgat tgatttagtc aaagatacta tgtcgggaaa cacctcctgc 360
   tttccggcgt caacagggct ttcttcagcc agaattaatt ttgacttatc gactttaagg 420
   ttgaatatcg aaatccctca ggcactgctg aatacacgtc caagaggata tatttcccct 480
   gctcagtggc aaagtggtgt tcctgcagca tttataaact atgatgctaa ctattaccag 540
   tatagctctt ccgggacgag taacgaacag acttatctgg gattaaaagc tggattcaat 600
   ttgtggggat gggctttgcg ccaccgtggc agtgagagct ggaataatag ctatcctgcc 660
   ggatatcaga atatagaaac aagtataatg catgaccttg ccccattgag agcacaattc 720
   acattagggg atttttatac gaatggtgag ctaatggata gcctcagttt gcggggagtc 780
   aggttagcat cggatgaacg aatgttaccc ggctctttac gtggctatgc tcctgctgtc 840
   cggggaatag ctaacagtaa tgctaaagta accatttatc aaaatgctca tatcctctat 900
   gaaacgacgg tgccagccgg accatttgtc atcaatgatt tatatcccag tggatatgct 960
   ggtgaccttc tcgttaagat aacagagtct aatggccaga cacgaatgtt cacggttcct 1020
   tttgcggccg ttgctcaact cattcgtccc ggatttagtc gctggcaaat gtcagtggga 1080
   aagtatcgtt atgcgaataa aacatataat gatttaatag cacaaggcac ctatcaatac 1140
   ggcctgacga atgatattac tttaaacagt ggtcttacca cagcttcagg atatacagcg 1200
   gggttagctg gcctggcctt taatacccct ctgggtgcta tagcatctga cattacattg 1260
   tccagaacag cattcaggta ttccggtgta acgcgtaaag gttatagtct gcactcaagt 1320
   tatagcatca atattccagc ctcaaacaca aatataactc tggcggctta tcgttattca 1380
   tcaaaagatt tttatcatct gaaggatgcg ctatcagcta atcacaacgc gtttattgat 1440
   gatgtttctg taaaaagtac agcgttttat cgtcccagga atcaattcca gatttcaatc 1500
   aaccaggaat taggtgaaaa atggggtggg atgtatttaa caggaacaac ctataattac 1560
   tggggacata aaggaagtcg taatgaatac cagattgggt acagcaactt ctggaaacaa 1620
   ctcggctatc aaattggatt gtctcagtca agagataatg agcaacaacg ccgtgatgac 1680
   agattttata ttaattttac tctccctctg ggaggaagtg ttcaaagccc ggtgttttcc 1740
   actgttttaa attatagcaa agaagagaaa aatagtattc agacatcaat tagtggtact 1800
   ggcggggagg ataatcagtt ctcttatggt atttcaggaa acagccagga aaacgggcct 1860
   tccggttatg caatgaatgg gggttatcgt tcaccttatg taaatataac cacaacagtc 1920
   gggcatgata ctcagaataa taatcaaagg tcatttggtg cgtcgggagc ggtggtcgca 1980
   cacccctatg gagtgacatt gagtaatgac ctgagtgata cttttgccat tatccatgct 2040
   gaaggagctc agggggctgt catcaataat gcctctggta gtcgtctgga tttttgggga 2100
   aatggtgttg ttccttatgt tacaccctat gagaaaaatc aaattagcat cgatccctcc 2160
   aatttagatt tgaatgttga attatcggcg acggagcagg aaatcattcc tcgtgctaat 2220
   agcgccacgt tagtgaaatt tgacactaaa acaggaagaa gtctgttatt tgatattcgt 2280
   atgtctactg gcaatccccc tccaatggct tctgaagttc tggatgaaca tggacagttg 2340
   gccggatatg tcgctcaggc cgggaaggta tttaccaggg gactccctga aaaaggtcat 2400
   ctcagcgttg tatggggacc agataataaa gacagatgtt catttgtata tcatgttgca 2460
   cacaataaag atgatatgca atctcagctc gttcctgttc tgtgtataca gcaccctaat 2520
   caggaaaaaa ca
   2532
<210> 124 <211> 831 <212> DNA <213> Escherichia coli
<400> 124
   atggtaaaat gtcatactct gattaaccgt agaaataaat gtctgctgat tgtttttata 60
   gtccttattg gatggattat attcagacct aaagcatata cttattcact aaatgataaa 120
   gaaaaagaga tgctcataat gttatcacaa catcctgaaa ctcggtactt tggattttat 180
   tccatagaac ttccggctga ttacaaacca acaggaatgg ttatgttcat acaaggatcg 240
   gcgatgatcc ctgtagaaac aaagctacaa tattatcctc cttttctgca atatatgaca 300
   cgatatgagg cagaactaaa aaacacctca gcattagatc cactggatac gccttatttg 360
   aagcaagttc acccactaag tccacctatg aatggagtca tttttgaacg aatgaaagcg 420
   aaatacaccc cagattttgc acgagtattg gatgcatgga aatgggaaaa tggcgttacg 480
   ttttcagtaa aaatagaagc taaagatggt agagcaaccc gctatgatgg aattagtaag 540
   attgccgaat acagttatgg atataatatt ccagaaaaaa aagtacagtt acttactatt 600
   ctttcaggac tacaacctcg tgcagataac caacccccat cagaaaataa attggcgata 660
   caatatgcac aggttgacgc ttcactactt ggagagtatg aattatctgt agattataaa 720
   aatagcaata atattaaaat aagtttgcag acggataata atagttatat tgactcatta 780
   ttagatataa gatatccgag taatggaaac agagcatggt ataactctat a 831
<210> 125 <211> 1098 <212> DNA <213> Escherichia coli
<400> 125
<210> 126 <211> 780 <212> DNA <213> Escherichia coli
<400> 126
   atgttgaaca gaaaactaaa tatacggcta cgtcattccc tgaacagtca ctgcatacct 60
   tccatcatta tcaataacac cgtacgttca tttcagaggt cagtcatgaa taccagagct 120
   ctttttcccc tgctgttcac tgtggcatca ttctccgcct ccgccggcaa ctgggctgtc 180
   aaaaacggct ggtgtcagac catgacggaa gatggtcagg cgctggtaat gctgaaaaat 240
   ggcacgattg gtattaccgg cctgatgcag ggatgcccga atggtgtaca gacgctcctg 300
   ggcagccgta tcagtattaa cggtaacctg atccccacat cacaaatgtg taatcagcag 360
   acgggattca gggctgttga ggtggaaatc ggacaggcgc cggaaatggt caaaaaagcc 420
   gttcactcca tagcagagcg tgatgtgtcc gttttacagg catttggtgt acgaatggaa 480
   ttcacccgcg gtgatatgct gaaggtctgt ccgaaatttg tcacatcact tgccggtttt 540
   tccccgaaac agacgaccac tattaataaa gattccgtcc tgcaggctgc ccggcaggca 600
   tacgcccggg aatatgacga ggaaacaaca gaaaccgctg attttggctc ttacgaagta 660
   aaaggcaata aggttgagtt tgaagtattc aatcctgaag accgtgcgta cgacaaagtg 720
   accgtcacgg ttggtgctga cggtaatgcc accggcgcca gcgttgaatt tatcggaaaa 780
<210> 127 <211> 1155 <212> DNA <213> Escherichia coli
<400> 127
   gtggtaatta tcaatagcac gatactgagc ggcgcaggcg ctatcccttc cctgacgtcg 60
   ctcttacccg acatcagaaa aatgctgctg gtcactgacc gtaatattgc gcagctcgac 120
   ggtgtgcagc agattcgcgc cttactggaa aagcactgcc cgcaggttaa cgttatcgat 180
   aatgtgcccg cagagcccac gcatcatgat gtgcgccagc taatggatgc ccctggcgat 240
   gcctcttttg atgtggtggt cgggatcggc ggtggcagcg tgttggatgt ggcgaagctg 300
   ctatcggtgc tttgccatcc acaatcaccg gggctggatg cgctgcttgc gggtgaaaaa 360
   ccgactcagc gggtgcaatc atggttgatt cctacaaccg ccggaaccgg ctcagaagcc 420
   acgccgaatg cgattctggc aatccctgag caaagcacga aggtgggtat tatttcccag 480
   gtgctgttac cagactatgt ggcgcttttc ccggaactga ccaccagcat gcccgcgcat 540
   attgcggcgt ccacgggcat tgatgctctt tgccacttac tggagtgttt taccgcgacc 600
   gtggcaaatc cggtcagcga taacgcggcg ctgactgggt taagtaaact tttccggcac 660
   attcaacccg ccgtgaacga tcctcaggat ctgcgcgcaa aactggaaat gctgtgggcg 720
   tcttactatg gcggcgtagc gataacccat gcgggcacgc atctcgttca tgcgctctcc 780
   tacccgttag gtggcaaata tcatctgccg catggcgtcg cgaatgccat cttgctggcg 840
   ccgtgcatgg cgtttgttcg cccctgggcg gtcgagaaat ttgcccgggt ctgggattgc 900
   attcccgatg cggaaaccgc cctgagcgcg gaagaaaaat ctcatgccct ggtgacctgg 960
   ttacaggcat tagtcaatca actcaagcta cccaacaatc tcgcggctct cggcgtaccg 1020
   ccagaggata ttgcctctct gagcgaggcg gcactgaacg tgaagcgcct tatgaacaat 1080
   gtgccgtgcc aaattgatct acaggacgta caggccattt accaaacact gtttccgcaa 1140
   catccattta aggag 1155
<210> 128 <211> 315 <212> DNA <213> Escherichia coli
<400> 128
   atgaatatca gaaaactgtt ttgtccggga aacacccccc ggattttatt gtttttattc 60
   ttttttgttg tttctgcaat aaccacaatt gcatgcggat acactgagaa gaatgcaaca 120
   ggaaatgtgc tgcttctgtt tctccttctg ctccttgcac acagaaatac cctcacatcc 180
   attacagcgc tgttatttct gttctgttgt gcactgtatg cgcctgccgg tatgacgtac 240
   ggtaaaatca acaacagttt tattgtcgcg ttgttgcaga ccacaactga tgaggcagcg 300
   gagtttaccg ggatg 315
<210> 129 <211> 441 <212> DNA <213> Escherichia coli
<400> 129
   atgaatattc aggcaataaa agaaatggta aatttaattt gtagtttttt atttatattc 60
   tttctgtcct cggcttttgt ttcttttggg tgttatgcta tttatgaatt gtttttatgg 120
   aatgatatta ttgtatatag ctggggatat atattaattg tctttttacc tttcacatta 180
   tatgtaatgt cgtttgagat tttgtttttt gctattagtg ggcgacgatt gtctaaagta 240
   acaatggtgc gcctttggtt gataattaaa attattattg ctttctctat ttgcgcagtg 300
   ttgatttttt cttcaattta caaaaaagaa ttattatcta gaaattatat tgcttgtagt 360
   ggtatcccgt ctgggtggat gccgggtctg gcaacgaaat acgttaaaga aaaatcatta 420
   tgcgaaaaaa atggcaataa t 441
<210> 130 <211> 534 <212> DNA <213> Escherichia coli <400> 130
<210> 131 <211> 627 <212> DNA <213> Escherichia coli
<400> 131
<210> 132 <211> 1272 <212> DNA <213> Escherichia coli
<400> 132
   atggatattt ggcggggaca ttcgtttctg atgacaattt ccgctaggtt cagacaatac 60
   gttttctctc ttatgtcaat tttattgcag gaacgaaaaa tgaatatttt cactttatcc 120
   aaagcaccgc tatacctgtt aatttcacta tttttaccca cgatggccat ggctatcgat 180
   ccacctgaac gcgaactttc gcgatttgcc ctgaaaacga attaccttca gtcccctgat 240
   gaaggcgtct atgaactggc gtttgataat gccagtaaaa aggtgtttgc agcagtcacc 300
   gatcgtgtaa atcgtgaagc caataaaggc tatctgtatt cgtttaattc agattcgctg 360
   aaagtcgaaa ataaatacac gatgccatac cgggcatttt cgctggcgat aaatcaggat 420
   aaacatcagc tctatatcgg acacacccag tcagcgtccc tgcgtatcag tatgtttgac 480
   accccaaccg gcaaactggt aagaaccagc gacaggttaa gttttaaagc ggcaaacgct 540
   gcagattcgc gttttgagca ttttcgccat atggtttaca gccaggattc cgataccctg 600
   tttgtgagtt atagcaatat gctgaaaacg gccgagggca tgaagcctct gcataagctg 660
   ttaatgctcg acgggacgac gcttgcctta aaaggcgagg ttaaggatgc ttacaaaggt 720
   acagcgtatg gtctgacgat ggatgaaaaa acacagaaaa tctacgttgg cggaagagat 780
   tacatcaacg aaattgatgc gaaaaatcag acgctgctgc gtaccatccc gttgaaagat 840
   ccgagaccac aaatcacaag tgtgcagaat ctggcggtgg actccgcttc tgaccgtgcc 900
   tttgtggtgg tattcgacca tgacgatcgt tccggtacaa aagatggact ctatattttt 960
   gacttacgcg acggtaaaca gcttggctat gtgcacacag gagccggagc taacgcggtg 1020
   aaatacaatc cgaaatataa cgaactgtat gtcaccaact tcactagcgg caccatcagc 1080
   gtagtggatg ccaccaaata cagcatcacc cgtgaattta acatgccggt ctacccaaac 1140
   cagatggtgt tgtcggacga tatggatacc ctttacattg gcatcaaaga aggctttaac 1200
   cgcgattggg atcctgatgt gtttgtggaa ggagctaaag aacgtattct gagcattgat 1260
   ttgaaaaagt cg 1272
<210> 133 <211> 163 <212> PRT <213> Escherichia coli
<400> 133
   Met Ala Ile Pro Ala Tyr Leu Trp Leu Lys Asp Asp Gly Gly Ala Asp
   1 5 10 15
   Ile Lys Gly Ser Val Asp Val Gln Gly Arg Glu Gly Ser Ile Glu Val
   20 25 30
   Val Ala Leu Asp His Asp Val Tyr Ile Pro Thr Asp Asn Asn Thr Gly
   35 40 45
   Lys Leu Thr Gly Thr Arg Thr His Lys Pro Phe Thr Phe Thr Lys Glu
   50 55 60
   Ile Asp Ala Ser Ser Pro Tyr Leu Tyr Lys Ala Val Thr Thr Gly Gln
   65 70 75 80
   Thr Leu Lys Thr Ala Glu Phe Lys Phe Tyr Arg Ile Asn Asp Ala Gly
   85 90 95
   Gln Glu Val Glu Tyr Phe Asn Ile Thr Leu Asp Asn Val Lys Leu Val
   100 105 110
   Arg Val Ala Pro Leu Met His Asp Ile Lys Asp Pro Ser Arg Glu Lys
   115 120 125
   His Asn His Leu Glu Arg Ile Glu Phe Arg Tyr Glu Lys Ile Thr Trp
   130 135 140
   Thr Tyr Lys Asp Gly Asn Ile Ile His Ser Asp Ser Trp Asn Glu Arg
   145 150 155 160
   Pro Ser Ala
<210> 134 <211> 550 <212> PRT <213> Escherichia coli
<400> 134
   Val Arg Asn Thr Leu Lys Gln Ala Ile Val Leu Trp Gly Met Val Leu
   1 5 10 15
   Leu Leu Val Leu Trp Ser Val Phe Ile Ser Pro Ser Gly Val Leu Arg
   20 25 30
   Trp Ala Gly Ala Ala Ala Ile Val Leu Ala Val Ala Ala Leu Leu Ile
   35 40 45
   Tyr Arg Arg Arg Gln Ala Trp Thr Glu Met Thr Gly Asp Ala Gly Leu
   50 55 60
   Ser Ser Leu Pro Pro Glu Thr Tyr Arg Gln Pro Val Val Leu Val Cys
   65 70 75 80
   Gly Gly Leu Ser Ala His Leu Ser Thr Asp Ser Pro Val Arg Gln Val
   85 90 95
   Ser Glu Gly Leu Tyr Leu His Val Pro Asp Glu Glu Gln Leu Val Ala
   100 105 110
   Gln Val Glu Arg Leu Leu Thr Leu Arg Pro Ala Trp Ala Ser Gln Leu
   115 120 125
   Ala Val Ala Tyr Thr Ile Met Pro Gly Ile His Arg Asp Val Ala Val
   130 135 140
   Leu Ala Gly Arg Leu Arg Arg Phe Ala His Ser Met Ala Thr Val Arg
   145 150 155 160
   Arg Arg Ala Gly Val Asn Val Pro Trp Leu Leu Trp Ser Gly Leu Ser
   165 170 175
   Gly Ser Pro Leu Pro Glu Arg Ala Ser Ser Pro Trp Phe Ile Cys Thr
   180 185 190
   Gly Gly Glu Val Gln Val Ala Thr Ser Thr Glu Thr Thr Met Pro Ala
   195 200 205
   Gln Trp Ile Ala Gln Ser Gly Val Gln Glu Arg Ser Gln Arg Leu Cys
   210 215 220
   Tyr Leu Leu Lys Ala Glu Ser Leu Met Gln Trp Leu Asn Leu Asn Val
   225 230 235
   240
   Leu Thr Ala Leu Asn Gly Pro Glu Ala Lys Cys Pro Pro Leu Ala Met
   245 250 255
   Thr Val Gly Leu Val Pro Ser Leu Pro Ala Val Asp Asn Asn Leu Trp
   260 265 270
   Gln Leu Trp Ile Thr Ala Arg Thr Gly Leu Thr Pro Asp Ile Ala Asp
   275 280 285
   Thr Gly Thr Asp Asp Ala Leu Pro Phe Pro Asp Ala Leu Leu Arg Gln
   290 295 300
   Leu Pro Arg Gln Ser Gly Phe Thr Pro Leu Arg Arg Ala Cys Val Thr
   305 310 315
   320
   Met Leu Gly Val Thr Thr Val Ala Gly Ile Ala Ala Leu Cys Leu
   Ser
   325 330 335
   Ala Thr Ala Asn Arg Gln Leu Leu Arg Gln Val Gly Asp Asp Leu
   His
   340 345 350
   Arg Phe Tyr Ala Val Pro Val Glu Glu Phe Ile Thr Lys Ala Arg
   His
   355 360 365
   Leu Ser Val Leu Lys Asp Asp Ala Thr Met Leu Asp Gly Tyr Tyr
   Arg
   370 375 380
   Glu Gly Glu Pro Leu Arg Leu Gly Leu Gly Leu Tyr Pro Gly Glu
   Arg
   385 390 395
   400
   Ile Arg Gln Pro Val Leu Arg Ala Ile Arg Asp Trp Arg Pro Pro
   Glu
   405 410 415
   Gln Lys Met Glu Val Thr Ala Ser Leu Gln Val Gln Thr Val Arg
   Leu
   420 425 430
   Asp Ser Met Ser Leu Phe Asp Val Gly Gln Ala Arg Leu Lys Asp
   Gly
   435 440 445
   Ser Thr Lys Val Leu Val Asp Ala Leu Val Asn Ile Arg Ala Lys
   Pro
   450 455 460
   Gly Trp Leu Ile Leu Val Ala Gly Tyr Thr Asp Ala Thr Gly Asp
   Glu
   465 470 475
   480
   Lys Ser Asn Gln Gln Leu Ser Leu Arg Arg Ala Glu Ala Val Arg Asn
   485 490 495
   Trp Met Leu Gln Thr Ser Asp Ile Pro Ala Thr Cys Phe Ala Val Gln
   500 505 510
   Gly Leu Gly Glu Ser Gln Pro Ala Ala Thr Asn Asp Thr Pro Gln Gly
   515 520 525
   Arg Ala Val Asn Arg Arg Val Glu Ile Ser Leu Val Pro Arg Ser Asp
   530 535 540
   Ala Cys Gln Asp Val Lys
   545 550
<210> 135 <211> 194 <212> PRT <212> Escherichia coli
<400> 135
   Met Ile Lys Ser Thr Phe Trp Arg Ala Leu Ala Leu Thr Ala Thr Leu
   1 5 10 15
   Ile Leu Thr Gly Cys Ser His Ser Gln Pro Glu Gln Glu Gly Arg Pro
   20 25 30
   Gln Ala Trp Leu Gln Pro Gly Thr Leu Ile Thr Leu Pro Ala Pro Gly
   35 40 45
   Ile Ser Pro Ala Val Asn Ser Gln Gln Leu Leu Thr Gly Ser Phe Asn
   50 55 60
   Gly Lys Thr Gln Ser Leu Leu Val Met Leu Asn Ala Glu Asp Gln Lys
   65 70 75 80
   Ile Thr Leu Ala Gly Leu Ser Ser Val Gly Ile Arg Leu Phe Leu Val
   85 90 95
   Thr Tyr Asp Ala Lys Gly Leu Arg Ala Glu Gln Ser Ile Val Val Pro
   100 105 110
   Gln Leu Pro Pro Ala Ser Gln Val Leu Ala Asp Val Met Leu Ser His
   115 120 125
   Trp Pro Ile Ser Ala Trp Gln Pro Gln Leu Pro Thr Gly Trp Thr Leu
   130 135 140
   Arg Asp Asn Gly Asp Lys Arg Glu Leu Arg Asn Ala Ser Gly Lys Leu
   145 150 155 160
   Val Thr Glu Ile Thr Tyr Leu Asn Arg Gln Gly Lys Arg Val Pro Ile
   165 170 175
   Ser Ile Glu Gln His Val Phe Lys Tyr His Ile Thr Ile Gln Tyr Leu
   180 185 190
   Gly Asp
<210> 136 <211> 129 <212> PRT <213> Escherichia coli
<400 136
<210> 137 <211> 415 <212> PRT <213> Escherichia coli
<400> 137
   Met Lys Arg Lys His Leu Leu Leu Leu Leu Leu Phe Ser Phe Ser Thr
   1 5 10 15
   Asn Ser Ala Pro Leu Tyr Ser Leu Ile Arg Glu Ala Val Met His Asp
   20 25 30
   Pro Ile Val Met Glu Ala Arg Ala Glu Leu Thr Ser Ala Gln Ser
   Arg
   35 40 45
   Ile Glu Gln Ala Ser Ser Ala His Trp Pro Val Val Thr Ala Thr
   Gly
   50 55 60
   Ser Lys Leu Leu Ser Gln Ser His Arg Tyr Ser Tyr Asp Tyr Asp
   Thr
   65 70 75 80
   Glu Asp Ile Leu Pro Gly Ile Arg Gly Glu Val Asn Ile Phe Ala
   Ser
   85 90 95
   Gly Ala Ile Glu Ala Asp Val Arg Arg Ser Glu Ser Glu Ala Glu
   Tyr
   100 105 110
   Tyr His Tyr Lys Met Glu Glu Thr Lys Glu Glu Thr Ile His Ser
   Phe
   115 120 125
   Val Ser Leu Tyr Leu Asp Ala Leu Arg Glu Lys Gln Ser Ile Ala
   Val
   130 135 140
   Leu Glu Gln Ser Leu Ser Arg His Asn Ala Ile Leu Asn Asp Leu
   Asn
   145 150 155
   160
   Thr Ile Ser Ile His Asp Thr Gly Arg Glu Ser Glu Leu Val Gln
   Ala
   165 170 175
   Glu Ala Arg Arg Leu Met Val Arg Gln Gln Ile Asn Ser Arg Ser
   Arg
   180 185 190
   Val Leu Lys Thr Thr Leu Gly Lys Leu Ser Thr Trp Thr Lys Asn
   Pro
   195 200 205
   Val Thr Glu Ala Asp Leu Glu Asn Pro Phe Ser Arg Met Thr Glu
   Ala
   210 215 220
   Lys Leu Leu Thr Asp Phe Thr Gln Ala Pro Gln Lys Gly Asn Pro
   Ser
   225 230 235
   240
   Trp Leu Ala Ser Gln Ala Asp Val Glu Ser Lys Lys Ala Ala Leu
   Lys
   245 250 255
   Ala Gln Glu Leu Ala Arg Tyr Pro Arg Val Asp Leu Thr Gly Ser
   Val
   260 265 270
   Thr Arg Asp Asp Gln Gln Ile Gly Val Asn Leu Ser Trp Asp Leu
   Phe
   275 280 285
   Asn Arg Asn Ala Ser Tyr Gly Val Thr Glu Lys Ala Ala Gln Ile
   Val
   290 295 300
   Ala Ala Thr Gly Arg Leu Asp Ser Val Ala Arg Met Ile Asp Glu
   Thr
   305 310 315
   320
   Gly Arg Leu Ser Leu Ile Thr Val Arg Gln Ser Arg Gly Glu Met
   Glu
   325 330 335
   Thr Leu Arg Arg Gln Glu Gln Ala Ser Ala Arg Val Val Asp Phe
   Tyr
   340 345 350
   Arg Leu Gln Phe Gln Val Ala Arg Lys Thr Leu Ile Glu Leu Leu Asn
   355 360 365
   Ala Glu Asn Glu Leu Tyr Ser Val Gly Leu Ser Arg Val Gln Thr Glu
   370 375 380
   Asp Gln Met Leu His Gly Met Leu Asp Tyr Leu Tyr Ser Gln Gly Met
   385 390 395 400
   Leu Leu Lys Trp Ser Gly Val Asn Leu Ser Gly Glu Glu Glu Lys
   405 410 415
<210> 138 <211> 201 <212> PRT <213> Escherichia coli
<400> 138
   Met Lys Phe Leu Pro Leu Leu Ala Leu Leu Ile Ser Pro Phe Val Ser
   1 5 10 15
   Ala Leu Thr Leu Asp Asp Leu Gln Gln Arg Phe Thr Glu Gln Pro Val
   20 25 30
   Ile Arg Ala His Phe Asp Gln Thr Arg Thr Ile Lys Asp Leu Pro Gln
   35 40 45
   Pro Leu Arg Ser Gln Gly Gln Met Leu Ile Ala Arg Asp Gln Gly Leu
   50 55 60
   Leu Trp Asp Gln Thr Ser Pro Phe Pro Met Gln Leu Leu Leu Asp Asp
   65 70 75 80
   Lys Arg Met Val Gln Val Ile Asn Gly Gln Pro Pro Gln Ile Ile Thr
   85 90 95
   Ala Glu Asn Asn Pro Gln Met Phe Gln Phe Asn His Leu Leu Arg Ala
   100 105 110
   Leu Phe Gln Ala Asp Arg Lys Val Leu Glu Gln Asn Phe Arg Val Glu
   115 120 125
   Phe Ala Asp Lys Gly Glu Gly Arg Trp Thr Leu Arg Leu Thr Pro Thr
   130 135 140
   Thr Thr Pro Leu Asp Lys Ile Phe Asn Thr Ile Asp Leu Ala Gly Lys
   145 150 155 160
   Thr Tyr Leu Glu Ser Ile Gln Leu Asn Asp Lys Gln Gly Asp Arg Thr
   165 170 175
   Asp Ile Ala Leu Thr Gln His Gln Leu Thr Pro Ala Gln Leu Thr Asp
   180 185 190
   Asp Glu His Gln Arg Phe Ala Ala Gln
   195 200
<210> 139 <211> 770 <212> PRT <213> Escherichia coli
<400> 139
   Met Glu Asn Phe Phe Met Lys Asn Ser Lys Val Phe Tyr Arg Ser Ala
   1 5 10 15
   Leu Ala Thr Ala Ile Val Met Ala Leu Ser Ala Pro Ala Phe Ala Thr
   20 25 30
   Asp Ser Thr Val Ser Thr Asp Pro Val Thr Leu Asn Thr Glu Lys Thr
   35 40 45
   Thr Leu Asp Gln Asp Val Val Ile Asn Gly Asp Asn Lys Ile Thr Ala
   50 55 60
   Val Thr Ile Glu Thr Ser Asp Ser Asp Lys Asp Leu Asn Val Thr Phe
   65 70 75 80
   Gly Gly His Asp Ile Thr Ala Ala Ser Thr Val Asn Gln Asp Phe Val
   85 90 95
   Glu Gly Val Lys Val Ser Gly Asn Lys Asn Val Val Ile Asn Ala Thr
   100 105 110
   Asp Ser Thr Ile Thr Ala Gln Gly Glu Gly Thr Tyr Val Arg Thr Ala
   115 120 125
   Met Val Ile Asp Ser Thr Gly Asp Val Val Val Asn Gly Gly Asn Phe
   130 135 140
   Val Ala Lys Asn Glu Lys Gly Ser Ala Thr Gly Ile Ser Leu Glu Ala
   145 150 155 160
   Thr Thr Gly Asn Asn Leu Thr Leu Asn Gly Thr Thr Ile Asn Ala Gln
   165 170 175
   Gly Asn Lys Ser Tyr Ser Asn Gly Ser Thr Ala Ile Phe Ala Gln Lys
   180 185 190
   Gly Asn Leu Leu Gln Gly Phe Asp Gly Asp Ala Thr Asp Asn Ile Thr
   195 200 205
   Leu Ala Asp Ser Asn Ile Ile Asn Gly Gly Ile Glu Thr Ile Val Thr
   210 215 220
   Ala Gly Asn Lys Thr Gly Ile His Thr Val Asn Leu Asn Ile Lys Asp
   225 230 235 240
   Gly Ser Val Ile Gly Ala Ala Asn Asn Lys Gln Thr Ile Tyr Ala Ser
   245 250 255
   Ala Ser Ala Gln Gly Ala Gly Ser Ala Thr Gln Asn Leu Asn Leu Ser
   260 265 270
   Val Ala Asp Ser Thr Ile Tyr Ser Asp Val Leu Ala Leu Ser Glu Ser
   275 280 285
   Glu Asn Ser Ala Ser Thr Thr Thr Asn Val Asn Met Asn Val Ala Arg
   290 295 300
   Ser Tyr Trp Glu Gly Asn Ala Tyr Thr Phe Asn Ser Gly Asp Lys Ala
   305 310 315 320
   Gly Ser Asp Leu Asp Ile Asn Leu Ser Asp Ser Ser Val Trp Lys Gly
   325 330 335
   Lys Val Ser Gly Ala Gly Asp Ala Ser Val Ser Leu Gln Asn Gly Ser
   340 345 350
   Val Trp Asn Val Thr Gly Ser Ser Thr Val Asp Ala Leu Ala Val Lys
   355 360 365
   Asp Ser Thr Val Asn Ile Thr Lys Ala Thr Val Asn Thr Gly Thr Phe
   370 375 380
   Ala Ser Gln Asn Gly Thr Leu Ile Val Asp Ala Ser Ser Glu Asn Thr
   385 390 395 400
   Leu Asp Ile Ser Gly Lys Ala Ser Gly Asp Leu Arg Val Tyr Ser Ala
   405 410 415
   Gly Ser Leu Asp Leu Ile Asn Glu Gln Thr Ala Phe Ile Ser Thr Gly
   420 425 430
   Lys Asp Ser Thr Leu Lys Ala Thr Gly Thr Thr Glu Gly Gly Leu Tyr
   435 440 445
   Gln Tyr Asp Leu Thr Gln Gly Ala Asp Gly Asn Phe Tyr Phe Val Lys
   450 455 460
   Asn Thr His Lys Ala Ser Asn Ala Ser Ser Val Ile Gln Ala Met Ala
   465 470 475 480
   Ala Ala Pro Ala Asn Val Ala Asn Leu Gln Ala Asp Thr Leu Ser Ala
   485 490 495
   Arg Gln Asp Ala Val Arg Leu Ser Glu Asn Asp Lys Gly Gly Val Trp
   500 505 510
   Ile Gln Tyr Phe Gly Gly Lys Gln Lys His Thr Thr Ala Gly Asn Ala
   515 520 525
   Ser Tyr Asp Leu Asp Val Asn Gly Val Met Leu Gly Gly Asp Thr Arg
   530 535 540
   Phe Met Thr Glu Asp Gly Ser Trp Leu Ala Gly Val Ala Met Ser Ser
   545 550 555 560
   Ala Lys Gly Asp Met Thr Thr Met Gln Ser Lys Gly Asp Thr Glu Gly
   565 570 575
   Tyr Ser Phe His Ala Tyr Leu Ser Arg Gln Tyr Asn Asn Gly Ile Phe
   580 585 590
   Ile Asp Thr Ala Ala Gln Phe Gly His Tyr Ser Asn Thr Ala Asp Val
   595 600 605
   Arg Leu Met Asn Gly Gly Gly Thr Ile Lys Ala Asp Phe Asn Thr Asn
   610 615 620
   Gly Phe Gly Ala Met Val Lys Gly Gly Tyr Thr Trp Lys Asp Gly Asn
   625 630 635 640
   Gly Leu Phe Ile Gln Pro Tyr Ala Lys Leu Ser Ala Leu Thr Leu Glu
   645 650 655
   Gly Val Asp Tyr Gln Leu Asn Gly Val Asp Val His Ser Asp Ser Tyr
   660 665 670
   Asn Ser Val Leu Gly Glu Ala Gly Thr Arg Val Gly Tyr Asp Phe Ala
   675 680 685
   Val Gly Asn Ala Thr Val Lys Pro Tyr Leu Asn Leu Ala Ala Leu Asn
   690 695 700
   Glu Phe Ser Asp Gly Asn Lys Val Arg Leu Gly Asp Glu Ser Val Asn
   705 710 715 720
   Ala Ser Ile Asp Gly Ala Ala Phe Arg Val Gly Ala Gly Val Gln Ala
   725 730 735
   Asp Ile Thr Lys Asn Met Gly Ala Tyr Ala Ser Leu Asp Tyr Thr Lys
   740 745 750
   Gly Asp Asp Ile Glu Asn Pro Leu Gln Gly Val Val Gly Ile Asn Val
   755 760 765
   Thr Trp
   770
<210> 140 <211> 660 <212> PRT <213> Escherichia coli
<400> 140
   Met Ser Arg Pro Gln Phe Thr Ser Leu Arg Leu Ser Leu Leu Ala Leu
   1 5 10 15
   Ala Val Ser Ala Thr Leu Pro Thr Phe Ala Phe Ala Thr Glu Thr Met
   20 25 30
   Thr Val Thr Ala Thr Gly Asn Ala Arg Ser Ser Phe Glu Ala Pro
   Met
   35 40 45
   Met Val Ser Val Ile Asp Thr Ser Ala Pro Glu Asn Gln Thr Ala
   Thr
   50 55 60
   Ser Ala Thr Asp Leu Leu Arg His Val Pro Gly Ile Thr Leu Asp
   Gly
   65 70 75 80
   Thr Gly Arg Thr Asn Gly Gln Asp Val Asn Met Arg Gly Tyr Asp
   His
   85 90 95
   Arg Gly Val Leu Val Leu Val Asp Gly Val Arg Gln Gly Thr Asp
   Thr
   100 105 110
   Gly His Leu Asn Gly Thr Phe Leu Asp Pro Ala Leu Ile Lys Arg
   Val
   115 120 125
   Glu Ile Val Arg Gly Pro Ser Ala Leu Leu Tyr Gly Ser Gly Ala
   Leu
   130 135 140
   Gly Gly Val Ile Ser Tyr Asp Thr Val Asp Ala Lys Asp Leu Leu
   Gln
   145 150 155
   160
   Glu Gly Gln Ser Ser Gly Phe Arg Val Phe Gly Thr Gly Gly Thr
   Gly
   165 170 175
   Asp His Ser Leu Gly Leu Gly Ala Ser Ala Phe Gly Arg Thr Glu
   Asn
   180 185 190
   Leu Asp Gly Ile Val Ala Trp Ser Ser Arg Asp Arg Gly Asp Leu Arg
   195 200 205
   Gln Ser Asn Gly Glu Thr Ala Pro Asn Asp Glu Ser Ile Asn Asn Met
   210 215 220
   Leu Ala Lys Gly Thr Trp Gln Ile Asp Ser Ala Gln Ser Leu Ser Gly
   225 230 235 240
   Leu Val Arg Tyr Tyr Asn Asn Asp Ala Arg Glu Pro Lys Asn Pro Gln
   245 250 255
   Thr Val Glu Ala Ser Asp Ser Ser Asn Pro Met Val Asp Arg Ser Thr
   260 265 270
   Ile Gln Arg Asp Ala Gln Leu Ser Tyr Lys Leu Ala Pro Gln Gly Asn
   275 280 285
   Asp Trp Leu Asn Ala Asp Ala Lys Ile Tyr Trp Ser Glu Val Arg Ile
   290 295 300
   Asn Ala Gln Asn Thr Gly Ser Ser Gly Glu Tyr Arg Glu Gln Ile Thr
   305 310 315 320
   Lys Gly Ala Arg Leu Glu Asn Arg Ser Thr Leu Phe Ala Asp Ser Phe
   325 330 335
   Ala Ser His Leu Leu Thr Tyr Gly Gly Glu Tyr Tyr Arg Gln Glu Gln
   340 345 350
   His Pro Gly Gly Ala Thr Thr Gly Phe Pro Gln Ala Lys Ile Asp Phe
   355 360 365
   Ser Ser Gly Trp Leu Gln Asp Glu Ile Thr Leu Arg Asp Leu Pro Ile
   370 375 380
   Thr Leu Leu Gly Gly Thr Arg Tyr Asp Ser Tyr Arg Gly Ser Ser Asp
   385 390 395 400
   Gly Tyr Lys Asp Val Asp Ala Asp Lys Trp Ser Ser Arg Ala Gly Met
   405 410 415
   Thr Ile Asn Pro Thr Asn Trp Leu Met Leu Phe Gly Ser Tyr Ala Gln
   420 425 430
   Ala Phe Arg Ala Pro Thr Met Gly Glu Met Tyr Asn Asp Ser Lys His
   435 440 445
   Phe Ser Ile Gly Arg Phe Tyr Thr Asn Tyr Trp Val Pro Asn Pro Asn
   450 455 460
   Leu Arg Pro Glu Thr Asn Glu Thr Gln Glu Tyr Gly Phe Gly Leu Arg
   465 470 475 480
   Phe Asp Asp Leu Met Leu Ser Asn Asp Ala Leu Glu Phe Lys Ala Ser
   485 490 495
   Tyr Phe Asp Thr Lys Ala Lys Asp Tyr Ile Ser Thr Thr Val Asp Phe
   500 505 510
   Ala Ala Ala Thr Thr Met Ser Tyr Asn Val Pro Asn Ala Lys Ile Trp
   515 520 525
   Gly Trp Asp Val Met Thr Lys Tyr Thr Thr Asp Leu Phe Ser Leu Asp
   530 535 540
   Val Ala Tyr Asn Arg Thr Arg Gly Lys Asp Thr Asp Thr Gly Glu Tyr
   545 550 555 560
   Ile Ser Ser Ile Asn Pro Asp Thr Val Thr Ser Thr Leu Asn Ile Pro
   565 570 575
   Ile Ala His Ser Gly Phe Ser Val Gly Trp Val Gly Thr Phe Ala Asp
   580 585 590
   Arg Ser Thr His Ile Ser Ser Ser Tyr Ser Lys Gln Pro Gly Tyr Gly
   595 600 605
   Val Asn Asp Phe Tyr Val Ser Tyr Gln Gly Gln Gln Ala Leu Lys Gly
   610 615 620
   Met Thr Thr Thr Leu Val Leu Gly Asn Ala Phe Asp Lys Glu Tyr Trp
   625 630 635 640
   Ser Pro Gln Gly Ile Pro Gln Asp Gly Arg Asn Gly Lys Ile Phe Val
   645 650 655
   Ser Tyr Gln Trp
   660
<210> 141 <211> 719 <212> PRT <213> Escherichia coli
<400> 141
   Met Arg Asp Glu Met Leu Tyr Asn Ile Pro Cys Arg Ile Tyr Ile Leu
   1 5 10 15
   Ser Thr Leu Ser Leu Cys Ile Ser Gly Ile Val Ser Thr Ala Thr Ala
   20 25 30
   Thr Ser Ser Glu Thr Lys Ile Ser Asn Glu Glu Thr Leu Val Val Thr
   35 40 45
   Thr Asn Arg Ser Ala Ser Asn Leu Trp Glu Ser Pro Ala Thr Ile Gln
   50 55 60
   Val Ile Asp Gln Gln Thr Leu Gln Asn Ser Thr Asn Ala Ser Ile Ala
   65 70 75 80
   Asp Asn Leu Gln Asp Ile Pro Gly Val Glu Ile Thr Asp Asn Ser Leu
   85 90 95
   Ala Gly Arg Lys Gln Ile Arg Ile Arg Gly Glu Ala Ser Ser Arg Val
   100 105 110
   Leu Ile Leu Ile Asp Gly Gln Glu Val Thr Tyr Gln Arg Ala Gly Asp
   115 120 125
   Asn Tyr Gly Val Gly Leu Leu Ile Asp Glu Ser Ala Leu Glu Arg Val
   130 135 140
   Glu Val Val Lys Gly Pro Tyr Ser Val Leu Tyr Gly Ser Gln Ala
   Ile
   145 150 155
   160
   Gly Gly Ile Val Asn Phe Ile Thr Lys Lys Gly Gly Asp Lys Leu
   Ala
   165 170 175
   Ser Gly Val Val Lys Ala Val Tyr Asn Ser Ala Thr Ala Gly Trp
   Glu
   180 185 190
   Glu Ser Ile Ala Val Gln Gly Ser Ile Gly Gly Phe Asp Tyr Arg
   Ile
   195 200 205
   Asn Gly Ser Tyr Ser Asp Gln Gly Asn Arg Asp Thr Pro Asp Gly
   Arg
   210 215 220
   Leu Pro Asn Thr Asn Tyr Arg Asn Asn Ser Gln Gly Val Trp Leu
   Gly
   225 230 235
   240
   Tyr Asn Ser Gly Asn His Arg Phe Gly Leu Ser Leu Asp Arg Tyr
   Arg
   245 250 255
   Leu Ala Thr Gln Thr Tyr Tyr Glu Asp Pro Asp Gly Ser Tyr Glu
   Ala
   260 265 270
   Phe Ser Val Lys Ile Pro Lys Leu Glu Arg Glu Lys Val Gly Val
   Phe
   275 280 285
   Tyr Asp Thr Asp Val Asp Gly Asp Tyr Leu Lys Lys Ile His Phe Asp
   290 295 300
   Ala Tyr Glu Gln Thr Ile Gln Arg Gln Phe Ala Asn Glu Val Lys Thr
   305 310 315 320
   Thr Gln Pro Val Pro Ser Pro Met Ile Gln Ala Leu Thr Val His Asn
   325 330 335
   Lys Thr Asp Thr His Asp Lys Gln Tyr Thr Gln Ala Val Thr Leu Gln
   340 345 350
   Ser His Phe Ser Leu Pro Ala Asn Asn Glu Leu Val Thr Gly Ala Gln
   355 360 365
   Tyr Lys Gln Asp Arg Val Ser Gln Arg Ser Gly Gly Met Thr Ser Ser
   370 375 380
   Lys Ser Leu Thr Gly Phe Ile Asn Lys Glu Thr Arg Thr Arg Ser Tyr
   385 390 395 400
   Tyr Glu Ser Glu Gln Ser Thr Val Ser Leu Phe Ala Gln Asn Asp Trp
   405 410 415
   Arg Phe Ala Asp His Trp Thr Trp Thr Met Gly Val Arg Gln Tyr Trp
   420 425 430
   Leu Ser Ser Lys Leu Thr Arg Gly Asp Gly Val Ser Tyr Thr Ala Gly
   435 440 445
   Ile Ile Ser Asp Thr Ser Leu Ala Arg Glu Ser Ala Ser Asp His
   Glu
       450                 455                 460
   Met Val Thr Ser Thr Ser Leu Arg Tyr Ser Gly Phe Asp Asn Leu
   Glu
   465                 470                 475
   480
   Leu Arg Ala Ala Phe Ala Gln Gly Tyr Val Phe Pro Thr Leu Ser
   Gln
                   485                 490                 495
   Leu Phe Met Gln Thr Ser Ala Gly Gly Ser Val Thr Tyr Gly Asn
   Pro
               500                 505                 510
   Asp Leu Lys Ala Glu His Ser Asn Asn Phe Glu Leu Gly Ala Arg
   Tyr
           515                 520                 525
   Asn Gly Asn Thr Trp Leu Ile Asp Ser Ala Val Tyr Tyr Ser Glu
   Ala
       530                 535                 540
   Lys Asp Tyr Ile Ala Ser Leu Ile Cys Asp Gly Ser Ile Val Cys
   Asn
   545                 550                 555
   560
   Gly Asn Thr Asn Ser Ser Arg Ser Ser Tyr Tyr Tyr Tyr Asp Asn
   Ile
                   565                 570                 575
   Asp Arg Ala Lys Thr Trp Gly Leu Glu Ile Ser Ala Glu Tyr Asn
   Gly
               580                 585                 590
   Trp Val Phe Ser Pro Tyr Ile Ser Gly Asn Leu Ile Arg Arg Gln
   Tyr
           595                 600                 605
   Glu Thr Ser Thr Leu Lys Thr Thr Asn Thr Gly Glu Pro Ala Ile Asn
   610 615 620
   Gly Arg Ile Gly Leu Lys His Thr Leu Val Met Gly Gln Ala Asn Ile
   625 630 635 640
   Ile Ser Asp Val Phe Ile Arg Ala Ala Ser Ser Ala Lys Asp Asp Ser
   645 650 655
   Asn Gly Thr Glu Thr Asn Val Pro Gly Trp Ala Thr Leu Asn Phe Ala
   660 665 670
   Val Asn Thr Glu Phe Gly Asn Glu Asp Gln Ser Arg Ile Asn Leu Ala
   675 680 685
   Leu Asn Asn Leu Thr Asp Lys Arg Tyr Arg Thr Ala His Glu Thr Ile
   690 695 700
   Pro Ala Ala Gly Phe Asn Ala Ala Ile Gly Phe Val Trp Asn Phe
   705 710 715
<210> 142 <211> 199 <212> PRT <213> Escherichia coli
<400> 142
   Met Arg Lys Val Cys Ala Val Ile Leu Ser Ala Ala Ile Cys Leu Ser
   1 5 10 15
   Val Ser Gly Ala Pro Ala Trp Ala Ser Glu His Gln Ser Thr Leu Ser
   20 25 30
   Ala Gly Tyr Leu His Ala Arg Thr Asn Ala Pro Gly Ser Asp Asn
   Leu
   35 40 45
   Asn Gly Ile Asn Val Lys Tyr Arg Tyr Glu Phe Thr Asp Ala Leu
   Gly
   50 55 60
   Leu Ile Thr Ser Phe Ser Tyr Ala Asn Ala Glu Asp Glu Gln Lys
   Thr
   65 70 75 80
   His Tyr Ser Asp Thr Arg Trp His Glu Asp Ser Val Arg Asn Arg
   Trp
   85 90 95
   Phe Ser Val Met Ala Gly Pro Ser Val Arg Val Asn Glu Trp Phe
   Ser
   100 105 110
   Ala Tyr Ser Met Ala Gly Val Ala Tyr Ser Arg Val Ser Thr Phe
   Ser
   115 120 125
   Gly Asp Tyr Leu Arg Val Thr Asp Asn Lys Gly Lys Thr His Asp
   Val
   130 135 140
   Leu Thr Gly Ser Asp Asp Gly Arg His Ser Asn Thr Ser Leu Ala
   Trp
   145 150 155
   160
   Gly Ala Gly Val Gln Phe Asn Pro Thr Glu Ser Val Thr Ile Asp
   Leu
   165 170 175
   Ala Tyr Glu Gly Ser Gly Ser Gly Asp Trp Arg Thr Asp Ala Phe
   Ile
   180 185 190
   Val Gly Ile Gly Tyr Arg Phe
   195
<210> 143 <211> 456 <212> PRT <213> Escherichia coli
<400> 143
   Met Lys Lys Ser Thr Leu Ser Leu Ala Ile Gly Leu Leu Leu Ala Cys
   1 5 10 15
   Ser Thr Gly Met Ala Lys Thr Gln His Leu Thr Leu Glu Gln Arg Leu
   20 25 30
   Glu Ala Ala Glu Met Arg Ala Ala Lys Ala Glu Gly Gln Val Lys Gln
   35 40 45
   Leu Gln Thr Gln Gln Ala Ala Glu Ile Arg Glu Ile Lys Thr Ala Gln
   50 55 60
   Gly Asn Thr Pro Val Asn Gly Gln Ser Thr Thr Glu Ser Glu Lys Lys
   65 70 75 80
   Asn Ala Thr Pro Pro Asn Leu Leu Leu Ser Gly Tyr Gly Asp Leu Lys
   85 90 95
   Ile Tyr Gly Asp Val Glu Phe Asn Met Asp Ala Glu Ser Asn His Gly
   100 105 110
   Leu Leu Ala Met Thr Asn Ala Asp Val Asn Ser Asp Pro Thr Asn Glu
   115 120 125
   Trp Asn Leu Asn Gly Arg Ile Leu Leu Gly Phe Asp Gly Met Arg Lys
   130 135 140
   Leu Asp Asn Gly Tyr Phe Ala Gly Phe Ser Ala Gln Pro Leu Gly Asp
   145 150 155 160
   Met His Gly Ser Val Asn Ile Asp Asp Ala Val Phe Phe Phe Gly Lys
   165 170 175
   Glu Asn Asp Trp Lys Val Lys Val Gly Arg Phe Glu Ala Tyr Asp Met
   180 185 190
   Phe Pro Leu Asn Gln Asp Thr Phe Val Glu His Ser Gly Asn Thr Ala
   195 200 205
   Asn Asp Leu Tyr Asp Asp Gly Ser Gly Tyr Ile Tyr Met Met Lys Glu
   210 215 220
   Gly Arg Gly Arg Ser Asn Ala Gly Gly Asn Phe Leu Val Ser Lys Gln
   225 230 235 240
   Leu Asp Asn Trp Tyr Phe Glu Leu Asn Thr Leu Leu Glu Asp Gly Thr
   245 250 255
   Ser Leu Tyr Asn Asp Gly Asn Tyr His Gly Arg Asp Met Glu Gln Gln
   260 265 270
   Lys Asn Val Ala Tyr Leu Arg Pro Val Ile Ala Trp Ser Pro Thr Glu
   275 280 285
   Glu Phe Thr Val Ser Ala Ala Met Glu Ala Asn Val Val Asn Asn Ala
   290 295 300
   Tyr Gly Tyr Thr Asp Ser Lys Gly Asn Phe Val Asp Gln Ser Asp Arg
   305 310 315 320
   Thr Gly Tyr Gly Met Ser Met Thr Trp Asn Gly Leu Lys Thr Asp Pro
   325 330 335
   Glu Asn Gly Ile Val Val Asn Leu Asn Thr Ala Tyr Leu Asp Ala Asn
   340 345 350
   Asn Glu Lys Asp Phe Thr Ala Gly Ile Asn Ala Leu Trp Lys Arg Phe
   355 360 365
   Glu Leu Gly Tyr Ile Tyr Ala His Asn Lys Ile Asp Glu Phe Ser Gly
   370 375 380
   Val Val Cys Asp Asn Asp Cys Trp Ile Asp Asp Glu Gly Thr Tyr Asn
   385 390 395 400
   Ile His Thr Ile His Ala Ser Tyr Gln Phe Ala Asn Val Met Asp Met
   405 410 415
   Glu Asn Phe Asn Ile Tyr Leu Gly Thr Tyr Tyr Ser Ile Leu Asp Ser
   420 425 430
   Asp Gly Asp Lys Ile His Gly Asp Asp Ser Asp Asp Arg Tyr Gly Ala
   435 440 445
   Arg Val Arg Phe Lys Tyr Phe Phe
   450 455
<210> 144 <211> 174 <212> PRT <213> Escherichia coli
<400> 144
   Met Asn Gly Lys Ala Phe Leu Ala Cys Val Leu Met Ser Val Val Leu
   1 5 10 15
   Thr Gly Cys Glu Thr Ala Lys Lys Ile Ser Gln Val Ile Arg Asn Pro
   20 25 30
   Asp Ile Gln Val Gly Lys Leu Met Asp Gln Ser Thr Glu Leu Thr Val
   35 40 45
   Thr Leu Leu Thr Glu Pro Asp Ser Asn Leu Thr Ala Asp Gly Glu Ala
   50 55 60
   Ala Pro Val Asp Val Gln Leu Val Tyr Leu Ser Asp Asp Ser Lys Phe
   65 70 75 80
   His Ala Ala Asp Tyr Asp Gln Val Ala Thr Thr Ala Leu Pro Asp Val
   85 90 95
   Leu Gly Lys Asn Tyr Ile Asp His Gln Asp Phe Asn Leu Leu Pro Asp
   100 105 110
   Thr Val Lys Thr Leu Pro Pro Ile Lys Leu Asp Glu Lys Thr Gly Tyr
   115 120 125
   Ile Gly Val Ile Ala Tyr Phe Ser Asp Asp Gln Ala Thr Glu Trp Lys
   130 135 140
   Gln Ile Glu Ser Val Glu Ser Ile Gly His His Tyr Arg Leu Leu Val
   145 150 155 160
   His Ile Arg Ala Ser Ala Ile Glu Met Lys Lys Glu Glu Asn
   165 170
<210> 145 <211> 1144 <212> PRT <213> Escherichia coli
<400> 145
   Leu Thr Leu Ala Trp Ile Phe Leu Leu Val Trp Ile Trp Trp Gln
   Gly
   1               5                   10                  15
   Pro Lys Trp Thr Leu Tyr Glu Gln His Trp Leu Ala Pro Leu Ala
   Asn
               20                  25                  30
   Arg Trp Leu Ala Thr Ala Val Trp Gly Leu Ile Ala Leu Val Trp
   Leu
           35                  40                  45
   Thr Trp Arg Val Met Lys Arg Leu Gln Lys Leu Glu Lys Gln Gln
   Lys
       50                  55                  60
   Gln Gln Arg Glu Glu Glu Lys Asp Pro Leu Thr Val Glu Leu His
   Arg
   65                  70                  75                  80
   Gln Gln Gln Tyr Leu Asp His Trp Leu Leu Arg Leu Arg Arg His
   Leu
                   85                  90                  95
   Asp Asn Arg Arg Tyr Leu Trp Gln Leu Pro Trp Tyr Met Val Ile
   Gly
               100                 105                 110
   Pro Ala Gly Ser Gly Lys Ser Thr Leu Leu Arg Glu Gly Phe Pro
   Ser
           115                 120                 125
   Asp Ile Val Tyr Thr Pro Glu Ser Ile Arg Gly Val Glu Tyr His Pro
   130 135 140
   Leu Ile Thr Pro Arg Val Gly Asn Gln Ala Val Ile Phe Asp Val Asp
   145 150 155 160
   Gly Val Leu Thr Thr Pro Gly Gly Asp Asp Leu Leu Arg Arg Arg Leu
   165 170 175
   Arg Glu His Trp Leu Gly Trp Leu Met Gln Thr Arg Ala Arg Gln Pro
   180 185 190
   Leu Asn Gly Leu Ile Leu Thr Leu Asp Leu Pro Asp Leu Leu Thr Ala
   195 200 205
   Asp Lys Ser Arg Arg Glu Thr Leu Val Gln Asn Leu Arg Gln Gln Leu
   210 215 220
   Gln Glu Ile Arg Gln Ser Leu His Cys Arg Leu Pro Val Tyr Val Val
   225 230 235 240
   Leu Thr Arg Leu Asp Leu Leu Asn Gly Phe Ala Ala Leu Phe His Ser
   245 250 255
   Leu Asp Lys Lys Asp Arg Asp Ala Ile Leu Gly Val Thr Phe Thr Arg
   260 265 270
   Arg Ala His Glu Ser Asp Gly Trp Arg Ser Glu Leu Gly Ala Phe Trp
   275 280 285
   Gln Thr Trp Val Gln Gln Val Asn Leu Ala Leu Ser Asp Leu Val Leu
   290 295 300
   Ala Gln Thr Gly Ala Ala Pro Arg Ser Ala Val Phe Ser Phe Ser Arg
   305 310 315 320
   Gln Met Gln Gly Thr Gly Glu Ile Val Thr Ala Leu Leu Ala Ala Leu
   325 330 335
   Leu Asp Gly Glu Asn Met Asp Val Met Leu Arg Gly Val Trp Leu Thr
   340 345 350
   Ser Ser Leu Gln Arg Gly Gln Val Asp Asp Ile Phe Thr Gln Ser Ala
   355 360 365
   Ala Arg Gln Tyr Gly Leu Gly Asn Ser Ser Leu Ala Thr Trp Pro Leu
   370 375 380
   Val Glu Thr Thr Pro Tyr Phe Thr Arg Arg Leu Phe Pro Glu Val Leu
   385 390 395 400
   Leu Ala Glu Pro Asn Leu Ala Gly Glu Asn Ser Val Trp Leu Asn Ser
   405 410 415
   Ser Arg Arg Arg Leu Thr Ala Phe Ser Thr Cys Gly Ala Ala Leu Ala
   420 425 430
   Ala Leu Met Val Gly Ser Trp His His Tyr Tyr Asn Gln Asn Trp Gln
   435 440 445
   Ser Gly Val Asn Val Leu Ala Gln Ala Lys Ala Phe Met Asp Val Pro
   450 455 460
   Pro Pro Gln Gly Thr Asp Glu Phe Gly Asn Leu Gln Leu Pro Leu Leu
   465 470 475 480
   Asn Pro Val Arg Asp Ala Thr Leu Ala Tyr Gly Asp Tyr Arg Asp His
   485 490 495
   Gly Phe Leu Ala Asp Met Gly Leu Tyr Gln Gly Ala Arg Val Gly Pro
   500 505 510
   Tyr Val Glu Gln Thr Tyr Ile Gln Leu Leu Glu Gln Arg Tyr Leu Pro
   515 520 525
   Ser Leu Met Asn Gly Leu Ile Arg Asp Leu Asn Ile Ala Pro Pro Glu
   530 535 540
   Ser Glu Glu Lys Leu Ala Val Leu Arg Val Val Arg Met Met Glu Asp
   545 550 555 560
   Lys Ser Gly Arg Asn Asn Glu Ala Val Lys Gln Tyr Met Ala Arg Arg
   565 570 575
   Trp Ser Asn Glu Phe His Gly Gln Arg Asp Ile Gln Ala Gln Leu Met
   580 585 590
   Val His Leu Asp Tyr Ala Leu Glu His Thr Asp Trp His Ala Gln Arg
   595 600 605
   Gln Ser Ser Asp Ser Asp Ala Val Ser Arg Trp Thr Pro Tyr Asp Lys
   610 615 620
   Pro Ile Ile Asn Ala Gln Gln Glu Leu Ser Lys Leu Pro Ile Tyr Gln
   625 630 635 640
   Arg Val Tyr Gln Thr Leu Arg Thr Lys Ala Leu Ser Val Leu Pro Ala
   645 650 655
   Asp Leu Asn Leu Arg Asp Gln Val Gly Pro Thr Phe Asp Asn Val Phe
   660 665 670
   Val Ala Gly Asn Asp Glu Lys Leu Val Ile Pro Gln Phe Leu Thr Arg
   675 680 685
   Tyr Gly Leu Gln Ser Tyr Phe Val Lys Gln Arg Glu Gly Leu Val Glu
   690 695 700
   Leu Thr Ala Leu Asp Ser Trp Val Leu Asn Leu Thr Gln Ser Val Ala
   705 710 715 720
   Tyr Ser Glu Ala Asp Arg Glu Glu Ile Gln Arg His Ile Thr Glu Gln
   725 730 735
   Tyr Ile Ser Asp Tyr Thr Ala Thr Trp Arg Ala Gly Met Asp Asn Leu
   740 745 750
   Asn Val Arg Asp Tyr Glu Ala Met Ser Ala Leu Thr Asp Ala Leu Glu
   755 760 765
   Gln Ile Ile Ser Gly Asp Gln Pro Phe Gln Arg Ala Leu Thr Ala Leu
   770 775 780
   Arg Asp Asn Thr His Ala Leu Thr Leu Ser Gly Lys Leu Asp Asp Lys
   785 790 795 800
   Ala Arg Glu Ala Ala Ile Asn Glu Met Asp Tyr Arg Leu Leu Ser Arg
   805 810 815
   Leu Gly His Glu Phe Ala Pro Glu Asn Ser Ala Leu Glu Glu Gln Lys
   820 825 830
   Asp Lys Ala Ser Thr Leu Gln Ala Val Tyr Gln Gln Leu Thr Glu Leu
   835 840 845
   His Arg Tyr Leu Leu Ala Ile Gln Asn Ser Pro Val Pro Gly Lys Ser
   850 855 860
   Ala Leu Lys Ala Val Gln Leu Arg Leu Asp Gln Asn Ser Ser Asp Pro
   865 870 875 880
   Ile Phe Ala Thr Arg Gln Met Ala Lys Thr Leu Pro Ala Pro Leu Asn
   885 890 895
   Arg Trp Val Gly Lys Leu Ala Asp Gln Ala Trp His Val Val Met Val
   900 905 910
   Glu Ala Val Arg Tyr Met Glu Val Asp Trp Arg Asp Asn Val Val
   Lys
   915 920 925
   Pro Phe Asn Glu Gln Leu Ala Asp Asn Tyr Pro Phe Asn Pro Arg
   Ala
   930 935 940
   Thr Gln Asp Ala Ser Leu Asp Ser Phe Glu Arg Phe Phe Lys Pro
   Asp
   945 950 955
   960
   Gly Ile Leu Asp Asn Phe Tyr Lys Asn Asn Leu Arg Leu Phe Leu
   Glu
   965 970 975
   Asn Asp Leu Thr Phe Gly Asp Asp Gly Arg Val Leu Ile Arg Glu
   Asp
   980 985 990
   Ile Arg Gln Gln Leu Asp Thr Ala Gln Lys Ile Arg Asp Ile Phe
   Phe
   995 1000 1005
   Ser Gln Gln Asn Gly Leu Gly Ala Gln Phe Ala Val Glu Thr Val
   1010 1015 1020
   Ser Leu Ser Gly Asn Lys Arg Arg Ser Val Leu Asn Leu Asp Gly
   1025 1030 1035
   Gln Leu Val Asp Tyr Ser Gln Gly Arg Asn Tyr Thr Ala His Leu
   1040 1045 1050
   Val Trp Pro Asn Asn Met Arg Glu Gly Asn Glu Ser Lys Leu Thr
   1055 1060 1065
   Leu Ile Gly Thr Ser Gly Arg Ala Pro Arg Ser Ile Ala Phe Ser
   1070 1075 1080
   Gly Pro Trp Ala Gln Phe Arg Leu Phe Gly Ala Gly Gln Leu Thr
   1085 1090 1095
   Asn Val Thr Ser Asp Thr Phe Asn Val Arg Phe Asn Val Asp Gly
   1100 1105 1110
   Gly Ala Met Val Tyr Gln Val His Val Asp Thr Glu Asp Asn Pro
   1115 1120 1125
   Phe Thr Gly Gly Leu Phe Ser Leu Phe Arg Leu Pro Asp Thr Leu
   1130 1135 1140
   Tyr
<210> 146 <211> 489 <212> DNA <213> Escherichia coli
<400> 146
   atggctattc ctgcttatct ctggctgaaa gatgacggcg gcgcggatat caaaggttcc 60
   gtggacgttc aggggcgcga aggtagcatc gaagtggtgg cgctggatca cgatgtgtac 120
   atcccgaccg acaataacac cggcaaactg accggtaccc gtactcacaa gccttttacg 180
   tttaccaaag aaatcgatgc gtccagcccg tatctctaca aagctgtgac caccggacag 240
   accctgaaaa cggcagaatt taagttttac cgcatcaacg atgccggtca ggaagtggag 300
   tacttcaaca tcacgcttga taacgtcaag ctggtcagag tcgctccgct tatgcacgac 360
   atcaaggatc cttccagaga gaagcataac cacctggaac gtattgagtt ccgctacgag 420
   aaaatcacct ggacttacaa agacggcaac atcattcatt ccgactcgtg gaatgagcgt 480
   ccttccgcc 489
<210> 147 <211> 1650 <212> DNA <213> Escherichia coli
<400> 147
   gtgaggaaca cgctgaaaca ggccatcgtg ctgtggggaa tggtgttact gctggtgctg 60
   tggtcagtgt ttatcagtcc gtctggcgtg ctgagatggg ccggtgcggc ggctatcgtt 120
   ctggcggttg ccgcgttgtt gatttatcgg cgcaggcagg cgtggacgga gatgaccggc 180
   gatgccgggt tgtcatcgct gccgccggaa acctaccgac agccggtagt gctggtctgt 240
   ggcggtctgt cggcgcacct gtccactgac agcccggtcc gccaggtttc agaagggctg 300
   tatctgcatg ttcctgatga agaacagctt gtggcgcagg tggagcgatt gctgaccctt 360
   cgcccggcgt gggcatcgca gcttgccgtg gcgtatacca tcatgcccgg catacaccgg 420
   gatgtggcgg ttctggccgg acggctgcga cggttcgccc acagtatggc gacggtgcgt 480
   cgtcgggcag gcgtaaacgt cccctggctt ctctggagcg ggctgtccgg ctcgccgttg 540
   ccggaaagag cgagttcacc gtggtttatc tgtaccggcg gcgaagttca ggtagcaaca 600
   tccacagaga ccaccatgcc cgcgcagtgg attgcacaat ccggcgtaca ggagcgcagt 660
   cagcgactct gttacctgct gaaagctgaa agcctgatgc agtggctgaa tcttaatgtg 720
   ctgacggcac tgaacggccc ggaggcgaaa tgtccaccac tggcgatgac cgtggggctg 780
   gtcccctcgt tgcctgcggt ggataacaac ctgtggcagt tgtggatcac cgccagaacc 840
   ggcctgacgc cggatatcgc ggacaccggc acagacgatg cgctgccatt cccggatgcc 900
   ctgttacggc agttgccgcg tcagtcgggc tttaccccgc tgcgacgagc ctgcgtgacc 960
   atgctgggcg tcaccaccgt ggcgggtatc gccgcgctgt gcctgtcagc cacggcaaat 1020
   cgccagttat tacggcaggt cggtgacgat ctgcaccggt tttatgccgt cccggtggag 1080
   gaatttatca ccaaagcccg tcacctgtcg gtgctgaaag acgatgcgac catgctcgat 1140
   gggtattacc gggaaggaga acccctgcgc ctcggtctgg ggttataccc cggcgaacgc 1200
   atccgccagc cggtattacg cgccattcgc gactggcgtc cgcctgaaca aaaaatggag 1260
   gtgacggctt cgcttcaggt tcagaccgtg cgtcttgaca gtatgtcgct gtttgacgtc 1320
   ggacaggccc gcctgaaaga cggctcgaca aaagtgctgg tggacgcact ggtgaacatc 1380
   cgggcaaaac cgggctggct gatcctcgtg gccggatata ccgatgccac cggcgatgaa 1440
   aaaagcaatc agcagttatc gctgcggcgt gccgaagcgg tgcgcaactg gatgctgcag 1500
   accagcgaca tcccggccac ctgttttgcc gtacagggac tgggcgagag ccagcctgcg 1560
   gcgaccaacg acacgccaca gggccgggca gtcaaccggc gtgtcgaaat cagtcttgtt 1620
   ccgcgttctg acgcctgtca ggacgtgaaa 1650
<210> 148 <211> 582 <212> DNA <213> Escherichia coli
<400> 148
   atgatcaaat ccacattctg gcgagcgctc gccctgaccg ctacgcttat cctcactggc 60
   tgtagccact cgcaaccgga acaggaaggc cgcccgcagg cgtggctgca acctggtacg 120
   ctcatcacgc tgcctgcgcc ggggatttca cccgcagtca attcccagca actgttgacc 180
   ggcagcttca acggcaaaac ccagtctctg ctagtgatgc ttaatgccga agatcagaaa 240
   atcacccttg ccgggctgtc gtcggtcggc attcgcctgt ttctggtgac ctacgatgca 300
   aaagggctac gcgccgagca atccatcgtc gtcccacagt taccgcccgc aagtcaggta 360
   ctggctgacg tgatgctcag ccactggccg attagcgcct ggcaaccgca acttcccaca 420
   ggctggacgc ttcgcgacaa cggcgacaaa cgcgagctgc gtaacgccag cggcaaactg 480
   gtcacggaaa tcacctatct gaatcgccag ggaaaacgcg tgccaatcag cattgagcag 540
   catgtcttta aataccacat caccattcaa tacttaggtg ac 582
<210> 149 <211> 387 <212> DNA <213> Escherichia coli
<400> 149
   atgaaacgtt atataaaatg gtttgccatc acaattttta tcagtatgtt gagtgcctgt 60
   gtccgtacgg ccccagtgca acagataagc accactgtca gtgtgggtca tactcaggag 120
   caggttaaaa atgccatttt gaaagcaggt gcgcagcgca agtggattat gacgcaagtg 180
   tcccctggag ttattaaagc tcgctatcaa acacgaaatc acgttgcaga ggttcgtatt 240
   acatatacag ctacctacta taacatcaaa tatgacagta gcctgaatct gcaggcttct 300
   gatggaaaaa ttcataaaaa ctataaccgc tgggtgcgta acctggataa agatatacag 360
   gttaacttat ctacaggagc aacgtta 387
<210> 150 <211> 1245 <212> DNA <213> Escherichia coli <400> 150
<210> 151 <211> 603 <212> DNA <213> Escherichia coli
<400> 151
   atgaaatttt taccgctgct ggcgctgctg attagcccgt ttgtgagcgc cctgaccctg 60
   gacgatcttc agcaacgctt taccgaacaa ccggtgatcc gcgcccattt tgatcaaacc 120
   cggacgatta aagatctgcc gcagccgctg cgatctcagg gtcagatgtt gatcgcccgc 180
   gaccaggggt tattgtggga tcaaacctca ccgttcccca tgcagctatt gctggatgat 240
   aaacgcatgg tgcaggtgat caacggtcag ccgccgcaaa tcatcacggc agaaaacaac 300
   ccgcagatgt tccagtttaa ccacctgctg cgcgcgctgt tccaggccga tcgcaaagtg 360
   ctggaacaaa acttccgcgt cgaatttgct gacaaaggcg aaggccgctg gacgctgcgc 420
   ctgacgccga ccaccacgcc gctggataaa attttcaaca ccatcgatct cgccgggaaa 480
   acctatctgg agagcattca acttaatgat aaacagggcg atcgcaccga tattgctctt 540
   acccaacatc aactgacgcc agcgcaactg accgatgacg aacaccaacg ttttgccgcc 600
   cag 603
<210> 152 <211> 2295 <212> DNA <213> Escherichia coli
<400> 152
   atgaaaaaca gtaaggtatt ttaccgcagc gcattagcga cagctattgt tatggctctt 60
   tctgcaccag cattcgctac tgatagcacg gtatcaactg atccggttac gctgaataca 120
   gagaagacga ctctggatca agatgttgtt attaacggtg ataacaagat tacagccgta 180
   acaattgaaa cgtcagattc agataaagac cttaatgtta cttttggcgg tcacgatatt 240
   accgccgcat caacggtaaa ccaagatttc gttgaaggtg taaaagttag tggtaacaaa 300
   aatgttgtga ttaatgctac agactccacc atcacagctc aaggtgaagg cacctatgtc 360
   cggactgcaa tggtcattga ttcaactggc gatgttgttg ttaatggcgg taatttcgtt 420
   gcaaaaaatg aaaaaggtag tgcgacaggg atatctctgg aagcgaccac gggaaataat 480
   ttaacgctca atggtacaac cataaatgct caaggtaata agagttacag caacggctct 540
   acggcaattt ttgctcaaaa gggtaatttg ttgcagggtt ttgacggtga tgcaaccgac 600
   aacatcaccc ttgctgactc aaatattatt aatggcggga ttgaaacaat agttactgcc 660
   gggaataaga cgggaattca tacagtcaac ctgaatatta aggatggctc agtaattggg 720
   gcggctaata ataaacaaac aatttatgcc tctgcttcgg cacaaggcgc aggttcagca 780
   acgcaaaatt taaatttgtc tgttgctgat tcaaccatct actctgatgt cctggccctt 840
   tctgaaagcg agaattcagc cagtaccaca acaaatgtaa atatgaacgt tgcccgctct 900
   tactgggaag gtaatgctta taccttcaat agcggcgata aagcgggtag tgatctggat 960
   ataaatcttt ccgatagttc agtctggaaa ggcaaagttt caggggcagg agatgccagt 1020
   gtatctctgc aaaacgggtc tgtctggaat gttacgggtt cctcaactgt tgatgctctg 1080
   gcagtaaaag acagtacggt taatatcacg aaggctacag tcaatactgg cacgtttgct 1140
   tctcaaaacg gcactctgat tgttgatgcc tcttctgaaa acactctgga tatcagcggt 1200
   aaagcgagcg gtgacttgcg tgtttacagt gcgggttcat tggatcttat caatgaacaa 1260
   acggcattta tttctaccgg caaagacagc actctaaaag ccacaggcac aacggaaggt 1320
   ggtctgtatc aatatgacct gacacaggga gctgatggta acttttattt cgtaaaaaac 1380
   acgcataaag catccaacgc cagctccgtg attcaggcaa tggcagctgc tccggctaac 1440
   gtcgctaatc tgcaggctga cacgctctcc gcccgtcagg atgctgtccg tctgagcgaa 1500
   aatgacaagg gtggcgtatg gattcagtac tttggcggta aacagaaaca taccaccgcg 1560
   ggaaatgcat cctatgacct ggatgtaaat ggtgtaatgc tgggtggtga tacccgcttc 1620
   atgactgaag atggtagctg gctggccggt gtggcgatgt cttctgcgaa aggtgacatg 1680
   actaccatgc agagcaaagg tgacactgaa ggttacagct tccacgctta cctgagccgc 1740
   cagtataaca acggtatctt cattgatact gctgcacagt ttggtcacta cagcaacacg 1800
   gcagatgttc gcctgatgaa tggtggcggt accatcaaag ctgactttaa caccaatggt 1860
   tttggtgcga tggttaaagg cggttacaca tggaaagacg gtaatggcct gtttattcag 1920
   ccatatgcca aactgtctgc tctgactctg gaaggtgtgg attatcaact caacggcgtg 1980
   gacgttcatt ctgacagcta taactctgtg ctgggtgagg ccggtacgcg cgtgggttat 2040
   gacttcgctg tgggcaacgc gaccgttaaa ccttatctga atctggccgc actgaacgaa 2100
   ttctctgatg gcaacaaagt ccgtctgggt gatgagtctg tcaatgccag cattgacggt 2160
   gcagcattcc gcgtgggtgc aggtgtacaa gctgatatca ccaaaaacat gggagcatat 2220
   gcaagccttg actacaccaa aggtgacgac attgagaacc cgctacaggg tgtagttggt 2280
   atcaatgtga cctgg 2295
<210> 153 <211> 1980 <212> DNA <213> Escherichia coli
<400> 153
   atgtcacgtc cgcaatttac ctcgttgcgt ttgagtttgt tggctttggc tgtttctgcc 60
   accttgccaa cgtttgcttt tgctactgaa accatgaccg ttacggcaac ggggaatgca 120
   cgtagttcct tcgaagcgcc tatgatggtc agcgttatcg acacttccgc tcctgaaaat 180
   caaactgcta cttcagccac tgatttgctg cgtcatgttc ctggaattac tcttgatggt 240
   accggacgaa ccaacggtca ggatgtaaat atgcgtggct atgatcatcg cggcgtgctg 300
   gttcttgtcg atggtgttcg ccagggaacg gataccggac acctgaatgg cacttttctc 360
   gatccggcgc tgatcaagcg tgttgagatt gttcgcggac cttcagcatt actgtatggc 420
   agtggcgcgc tgggtggagt gatctcctac gatacggtcg atgcaaaaga tttattgcag 480
   gaaggacaaa gcagtggttt tcgtgtcttt ggtactggcg gcacggggga ccatagcctg 540
   ggattaggcg cgagcgcgtt tgggcgaact gaaaatctgg atggtattgt ggcctggtcc 600
   agtcgcgatc ggggtgattt acgccagagc aatggtgaaa ccgcgccgaa tgacgagtcc 660
   attaataaca tgctggcgaa agggacctgg caaattgatt cagcccagtc tctgagcggt 720
   ttagtgcgtt actacaacaa cgacgcgcgt gaaccaaaaa atccgcagac cgttgaagct 780
   tctgatagca gcaacccgat ggtcgatcgt tcaacaattc aacgcgatgc gcagctttct 840
   tataaactcg ccccgcaggg taacgactgg ttaaatgcag atgcaaaaat ttactggtcg 900
   gaagtccgta ttaatgcgca aaacacgggg agttcaggcg agtatcgtga acagataaca 960
   aaaggagcaa ggctggagaa ccgttccact ctatttgccg acagtttcgc ttctcactta 1020
   ctgacatatg gcggtgagta ttatcgtcag gaacaacatc cgggtggcgc gacgacgggc 1080
   ttcccgcaag caaaaatcga ttttagctct ggttggctac aagatgagat caccttacgc 1140
   gatctgccga ttaccctgct tggcggaacc cgctatgaca gttatcgcgg tagcagcgac 1200
   ggctacaaag atgttgatgc cgacaaatgg tcatctcgtg cggggatgac tatcaacccg 1260
   accaactggc tgatgttatt tggctcatat gctcaggcat tccgcgcccc gacgatgggc 1320
   gaaatgtata acgattctaa acacttctcg attggtcgct tctataccaa ctattgggtg 1380
   ccaaacccga acttacgtcc ggaaactaac gaaactcagg agtacggttt tgggctgcgt 1440
   tttgatgacc tgatgttgtc caatgatgct ctggaattta aagccagcta ctttgatacc 1500
   aaagcgaaag attatatctc cacgaccgtc gatttcgcgg cggcgacaac tatgtcgtat 1560
   aacgtcccga acgccaaaat ctggggctgg gatgtgatga cgaaatatac cactgatctg 1620
   tttagccttg atgtggccta taaccgtacc cgcggcaaag acaccgatac cggggaatat 1680
   atctccagca ttaacccgga taccgttacc agtaccctga atattccgat cgctcacagc 1740
   ggcttctctg ttggttgggt cggtacgttt gccgatcgct caacacatat cagcagcagc 1800
   tacagcaaac aacctggcta tggtgtgaat gatttctacg tcagttatca agggcagcag 1860
   gcgctcaaag gcatgaccac tactctggta ttgggcaacg ccttcgataa agagtactgg 1920
   tcgccgcaag gcatcccaca ggatggtcgt aacggaaaaa ttttcgtgag ttatcaatgg 1980
<210> 154 <211> 2157 <212> DNA <213> Escherichia coli
<400> 154
<210> 155 <211> 600 <212> DNA <213> Escherichia coli
<400> 155
   atgcgtaaag tttgtgcagt cattttgtcc gcagccatct gtctgtccgt atccggtgcg 60
   cctgcatggg cgtctgaaca tcagtccaca ctgagcgcgg ggtatcttca tgcccgtacg 120
   aacgctcccg gcagcgataa tctgaacggg attaacgtga aataccgtta tgagtttacg 180
   gacgcgctgg ggctgattac gtccttcagt tatgccaatg ctgaggatga gcaaaaaacg 240
   cactacagcg atacccgctg gcatgaagat tccgtgcgta accgctggtt cagcgtgatg 300
   gcggggccgt ctgtacgcgt gaatgaatgg ttcagcgcgt attcgatggc gggtgtggct 360
   tacagccgtg tgtcgacttt ctccggggat tatctccgcg taactgacaa caaggggaaa 420
   acgcacgatg tgctgaccgg aagtgatgac ggtcgccaca gcaacacgtc tctggcgtgg 480
   ggggctggcg tgcagtttaa cccgaccgaa tccgtgacca ttgaccttgc ttatgaaggt 540
   tccggtagtg gcgactggcg aacggatgca tttattgttg gtatcggata ccgtttctga 600
<210> 156 <211> 1368 <212> DNA <213> Escherichia coli
<400> 156
   atgaaaaaat cgacattatc tttagccatc ggtttattat tggcatgtag taccggtatg 60
   gcaaaaacac agcatttaac gctggaacaa cgcctggaag cggcagaaat gcgggcagca 120
   aaagcagagg ggcaggttaa acagcttcag acacaacaag ccgccgagat ccgcgaaatt 180
   aaaaccgcac agggcaacac gccggtaaac ggtcaatcaa cgacggagtc agagaagaaa 240
   aacgccaccc cgcctaatct cctgctttca gggtatggcg atttaaaaat ctacggtgac 300
   gtagaattta atatggatgc ggaaagtaat catggcctgc tggcaatgac caacgctgat 360
   gtgaatagcg atcccactaa tgaatggaat ctcaatggtc gtattctgtt aggttttgat 420
   ggtatgcgaa aactggataa tggctatttc gctgggttct ccgcacaacc gctgggggat 480
   atgcacggtt cagtaaatat cgatgatgcg gttttcttct ttggcaaaga aaacgactgg 540
   aaggtcaaag taggccgttt tgaagcctac gatatgttcc cgctgaatca ggataccttt 600
   gttgaacatt ccggtaatac tgcgaacgat ctttatgacg atggcagcgg ttatatctat 660
   atgatgaaag agggccgcgg acgttctaac gctggcggta atttcctcgt cagcaaacaa 720
   ctcgataact ggtattttga attaaacacg ttactggaag acggaacatc tttatataac 780
   gacggtaatt atcatggacg cgatatggaa cagcagaaaa atgttgctta tctgcgtccg 840
   gtaattgcct ggtcgccgac ggaagaattc accgtttccg cagcgatgga agcgaatgtg 900
   gtaaataatg cttatggtta taccgatagc aagggtaatt ttgtcgatca gtccgatcgt 960
   accggttatg gcatgagtat gacctggaat ggcctgaaaa ccgatccgga aaatggcatc 1020
   gtggttaatc ttaataccgc ctatttagat gctaataatg aaaaagattt cacggcaggg 1080
   attaacgcgc tgtggaaacg tttcgagctg ggttatatct atgcacataa taagattgat 1140
   gaatttagtg gcgtggtttg tgataacgat tgctggattg atgatgaagg aacatacaac 1200
   attcacacca ttcatgcgtc ttatcagttc gctaatgtga tggatatgga gaactttaat 1260
   atttacctcg gcacgtatta ctccattctg gatagcgacg gcgataagat acacggcgac 1320
   gatagtgatg accgttacgg cgcacgcgtt cgctttaaat acttcttc 1368
<210> 157 <211> 522 <212> DNA <213> Escherichia coli
<400> 157
   atgaacggca aagcgtttct ggcctgcgtt ctgatgagcg tcgtattaac tggctgtgaa 60
   acagcgaaaa aaatcagcca ggtgatccgc aatccggata ttcaggtcgg aaagctgatg 120
   gatcagtcaa ccgagctgac cgtcacgctg ctgaccgagc cggacagcaa cctgacggcg 180
   gatggcgaag ccgcgccggt ggatgtccag ttggtttatc tgagcgacga ctcaaaattc 240
   catgccgccg actacgacca ggttgccacc accgcgctgc ccgacgtgct ggggaaaaac 300
   tatatcgatc accaggactt caacctgttg ccggataccg taaaaacact gccgccgatc 360
   aagttggatg agaaaaccgg ttatatcggt gtcattgcct atttttcaga cgaccaggcc 420
   acagaatgga aacaaattga gtcggtagaa agtatcggcc accactatcg cctgctggtg 480
   catatccgcg ccagtgcgat tgagatgaaa aaagaggaaa ac 522
<210> 158 <211> 3432 <212> DNA <213> Escherichia coli
<400> 158
   ctgacgctgg catggatttt tctgctggtg tggatctggt ggcagggtcc aaaatggacg 60
   ctctatgagc agcactggct ggctccgctg gcaaaccgct ggctggcgac cgccgtctgg 120
   ggacttatcg ctctggtctg gctcacctgg cgggtgatga agcgtctgca aaagctggaa 180
   aaacagcaga aacagcagcg ggaggaagaa aaagatccgt tgaccgtgga actccaccgc 240
   cagcagcaat atctggatca ctggctgctg cgcctgcgcc gccatctgga taaccgccgt 300
   tatctgtggc agttgccgtg gtatatggtc attggtcctg cgggtagcgg caaaagcacg 360
   ctgctgcgcg agggctttcc gtctgacatt gtttacacgc cggaaagcat ccggggtgtg 420
   gaataccacc cgctgatcac accgcgagtg ggcaaccagg cggtaatttt cgatgttgac 480
   ggcgtactga ccactcccgg cggggatgat ctgctccgcc gccgcctgcg cgaacactgg 540
   ctgggctggc tgatgcaaac gcgcgctcgc cagtcgctca acggtcttat cctgacgctc 600
   gatcttcccg atctgctgac ggcggataaa tcccgccgtg agacactggt acaaaatttg 660
   cgccagcaac ttcaggagat ccgtcagagc ctgcactgcc gtctgcccgt ttacgtggtg 720
   ctgacacggc tggatctgct gaacggcttt gccgcgctgt tccattcact ggataaaaaa 780
   gaccgcgatg cgatcctcgg cgtcacattt acccgccgcg cccatgaaag tgacggctgg 840
   cgcagcgaac tgggggcttt ctggcagacg tgggtacaac aggtgaacct ggcgctgtcg 900
   gatctggtgc tcgcacaaac cggtgctgct ccccgcagcg ctgtgttcag cttctcccgt 960
   cagatgcagg gaacaggaga aatcgtcacc gcactgctcg ccgcattgct ggacggtgag 1020
   aacatggatg taatgctgcg tggcgtctgg ctcacatcct cgctacagcg tggccaggtg 1080
   gatgatattt tcacgcagtc cgccgcccgc cagtacggac tgggtaacag ctcgctggca 1140
   acctggcctc tggtggagac gacgccgtat tttactcgcc gcctcttccc ggaagtcctg 1200
   ctggctgagc cgaacctggc gggtgaaaac agcgtctggc tgaacagctc ccggcgcagg 1260
   ctgaccgcct tttccacctg tggcgcggca ctggcggcat tgatggtcgg aagctggcac 1320
   cattattaca atcagaactg gcagtctggc gttaacgtac tggcacaagc taaagccttt 1380
   atggacgtac caccaccgca gggaacggat gaattcggca atctgcaatt gccattgctt 1440
   aacccggtac gcgatgccac cctggcctat ggtgattatc gcgatcacgg ttttctggcg 1500
   gatatgggat tgtaccaggg cgcccgcgta gggccgtatg tggagcaaac ctacattcag 1560
   cttcttgagc agcgttatct cccctcgtta atgaacggcc tgatccggga tctaaacatt 1620
   gccccgccag agagcgaaga aaagctcgct gtgctgcgcg tagtgcgcat gatggaagac 1680
   aaaagtgggc gcaacaacga ggcggtaaaa cagtacatgg cacggcgctg gagcaatgaa 1740
   tttcacggcc agcgcgatat tcaggcgcaa ctgatggtgc atctggacta tgcgctggag 1800
   cacaccgact ggcacgcgca gcgccaaagc agcgacagcg atgctgtcag ccgctggacc 1860
   ccctatgata aaccgatcat taatgcgcag caggaactga gcaagctgcc catataccag 1920
   cgtgtctacc agaccctgcg caccaaagca ttaagcgtgt tgcccgccga tttgaatttg 1980
   cgcgaccagg ttggtcccac cttcgacaac gtgttcgtcg ccggtaatga tgaaaaactg 2040
   gtgatcccgc agttcctcac ccgctatgga ctgcaaagct attttgtcaa acagcgtgag 2100
   ggcctcgttg agctgaccgc gctggattcg tgggtactga acctgacgca aagcgtcgcc 2160
   tacagcgagg ccgaccgtga agagatccag cgccatatca ccgaacagta catcagtgac 2220
   tataccgcca cctggcgtgc cggaatggat aacctcaacg tccgtgacta tgaggccatg 2280
   tcggcgctga ccgacgcgct ggagcagatt atcagcggcg atcagccatt ccagcgtgcg 2340
   ctgacggcgc tgcgcgataa tacccacgcg ctgacgctct ccggcaaact ggatgataag 2400
   gcgagggaag cggcgataaa tgagatggat taccgcctgt tatcccggct ggggcatgag 2460
   ttcgcaccgg aaaacagcgc actggaggag caaaaggaca aggcgagtac gctacaggcc 2520
   gtgtaccagc aactgaccga gctgcaccgt tacctgctgg cgatccagaa ctcgccagtg 2580
   ccggggaaat cggcgctgaa agcagtacag ctacggctgg atcaaaacag cagcgatcca 2640
   atcttcgcca cccgtcagat ggcaaaaacc ctgcctgcgc ctcttaaccg ctgggtaggt 2700
   aagctcgcgg atcaggcctg gcatgtggtg atggtggaag ccgttcgtta catggaagtg 2760
   gactggcgcg acaatgtagt gaaacccttc aacgagcagc ttgccgataa ctatccgttt 2820
   aatccgcgcg ccacacagga tgcctcactg gattcgtttg aacgtttctt taaaccggat 2880
   ggcattctgg acaatttcta caagaacaac ctgcgcctgt tccttgaaaa cgatctgacc 2940
   tttggcgacg acggcagagt gttaatccgt gaagatatcc ggcagcaact ggataccgcg 3000
   cagaaaatcc gcgacatctt cttcagccag cagaacgggc tgggcgcaca gtttgccgtg 3060
   gaaaccgtat cgctttccgg caataagcgg cgcagcgtac ttaacctgga cggccagtta 3120
   gtggactaca gccagggacg caactacacc gcccatctgg tctggccgaa caacatgcgt 3180
   gaaggcaatg aaagcaagct gacgctgatt ggcaccagcg gcagagcacc gcgcagtatc 3240
   gcgttcagtg gaccgtgggc gcagttccgc ctgttcggcg cgggccagtt gaccaatgtg 3300
   accagtgaca cctttaacgt gcgctttaac gtggacggcg gcgcaatggt ttaccaggtg 3360
   catgtggata ccgaagataa cccgttcacc ggcggtctgt tcagcctgtt ccgtttaccg 3420
   gatacgttgt at
   3432
<210> 159 <211> 725 <212> PRT <213> Escherichia coli
<400> 159
   Met Arg Ile Asn Lys Ile Leu Trp Ser Leu Thr Val Leu Leu Val Gly
   1 5 10 15
   Leu Asn Ser Gln Val Ser Val Ala Lys Tyr Ser Asp Asp Asp Asn Asp
   20 25 30
   Glu Thr Leu Val Val Glu Ala Thr Ala Glu Gln Val Leu Lys Gln Gln
   35 40 45
   Pro Gly Val Ser Val Ile Thr Ser Glu Asp Ile Lys Lys Thr Pro Pro
   50 55 60
   Val Asn Asp Leu Ser Asp Ile Ile Arg Lys Met Pro Gly Val Asn Leu
   65 70 75 80
   Thr Gly Asn Ser Ala Ser Gly Thr Arg Gly Asn Asn Arg Gln Ile Asp
   85 90 95
   Ile Arg Gly Met Gly Pro Glu Asn Thr Leu Ile Leu Ile Asp Gly Val
   100 105 110
   Pro Val Thr Ser Arg Asn Ser Val Arg Tyr Ser Trp Arg Gly Glu Arg
   115 120 125
   Asp Thr Arg Gly Asp Thr Asn Trp Val Pro Pro Glu Gln Val Glu Arg
   130 135 140
   Ile Glu Val Ile Arg Gly Pro Ala Ala Ala Arg Tyr Gly Ser Gly Ala
   145 150 155 160
   Ala Gly Gly Val Val Asn Ile Ile Thr Lys Arg Pro Thr Asn Asp Trp
   165 170 175
   His Gly Ser Leu Ser Leu Tyr Thr Asn Gln Pro Glu Ser Ser Glu Glu
   180 185 190
   Gly Ala Thr Arg Arg Ala Asn Phe Ser Leu Ser Gly Pro Leu Ala Gly
   195 200 205
   Asp Ala Leu Thr Thr Arg Leu Tyr Gly Asn Leu Asn Lys Thr Asp Ala
   210 215 220
   Asp Ser Trp Asp Ile Asn Ser Pro Val Gly Thr Lys Asn Ala Ala Gly
   225 230 235
   240
   His Glu Gly Val Arg Asn Lys Asp Ile Asn Gly Val Val Ser Trp
   Lys
   245 250 255
   Leu Asn Pro Gln Gln Ile Leu Asp Phe Glu Val Gly Tyr Ser Arg
   Gln
   260 265 270
   Gly Asn Ile Tyr Ala Gly Asp Thr Gln Asn Ser Ser Ser Ser Ala
   Val
   275 280 285
   Thr Glu Ser Leu Ala Lys Ser Gly Lys Glu Thr Asn Arg Leu Tyr
   Arg
   290 295 300
   Gln Asn Tyr Gly Ile Thr His Asn Gly Ile Trp Asp Trp Gly Gln
   Ser
   305 310 315
   320
   Arg Phe Gly Val Tyr Tyr Glu Lys Thr Asn Asn Thr Arg Met Asn
   Glu
   325 330 335
   Gly Leu Ser Gly Gly Gly Glu Gly Arg Ile Leu Ala Gly Glu Lys
   Phe
   340 345 350
   Thr Thr Asn Arg Leu Ser Ser Trp Arg Thr Ser Gly Glu Leu Asn
   Ile
   355 360 365
   Pro Leu Asn Val Met Val Asp Gln Thr Leu Thr Val Gly Ala Glu
   Trp
   370 375 380
   Asn Arg Asp Lys Leu Asp Asp Pro Ser Ser Thr Ser Leu Thr Val Asn
   385 390 395 400
   Asp Arg Asp Ile Ser Gly Ile Ser Gly Ser Ala Ala Asp Arg Ser Ser
   405 410 415
   Lys Asn His Ser Gln Ile Ser Ala Leu Tyr Ile Glu Asp Asn Ile Glu
   420 425 430
   Pro Val Pro Gly Thr Asn Ile Ile Pro Gly Leu Arg Phe Asp Tyr Leu
   435 440 445
   Ser Asp Ser Gly Gly Asn Phe Ser Pro Ser Leu Asn Leu Ser Gln Glu
   450 455 460
   Leu Gly Asp Tyr Phe Lys Val Lys Ala Gly Val Ala Arg Thr Phe Lys
   465 470 475 480
   Ala Pro Asn Leu Tyr Gln Ser Ser Glu Gly Tyr Leu Leu Tyr Ser Lys
   485 490 495
   Gly Asn Gly Cys Pro Lys Asp Ile Thr Ser Gly Gly Cys Tyr Leu Ile
   500 505 510
   Gly Asn Lys Asp Leu Asp Pro Glu Ile Ser Val Asn Lys Glu Ile Gly
   515 520 525
   Leu Glu Phe Thr Trp Glu Asp Tyr His Ala Ser Val Thr Tyr Phe Arg
   530 535 540
   Asn Asp Tyr Gln Asn Lys Ile Val Ala Gly Asp Asn Val Ile Gly Gln
   545 550 555 560
   Thr Ala Ser Gly Ala Tyr Ile Leu Lys Trp Gln Asn Gly Gly Lys Ala
   565 570 575
   Leu Val Asp Gly Ile Glu Ala Ser Met Ser Phe Pro Leu Val Lys Glu
   580 585 590
   Arg Leu Asn Trp Asn Thr Asn Ala Thr Trp Met Ile Thr Ser Glu Gln
   595 600 605
   Lys Asp Thr Gly Asn Pro Leu Ser Val Ile Pro Lys Tyr Thr Ile Asn
   610 615 620
   Asn Ser Leu Asn Trp Thr Ile Thr Gln Ala Phe Ser Ala Ser Phe Asn
   625 630 635 640
   Trp Thr Leu Tyr Gly Arg Gln Lys Pro Arg Thr His Ala Glu Thr Arg
   645 650 655
   Ser Glu Asp Thr Gly Gly Leu Ser Gly Lys Glu Leu Gly Ala Tyr Ser
   660 665 670
   Leu Val Gly Thr Asn Phe Asn Tyr Asp Ile Asn Lys Asn Leu Arg Leu
   675 680 685
   Asn Val Gly Val Ser Asn Ile Leu Asn Lys Gln Ile Phe Arg Ser Ser
   690 695 700
   Glu Gly Ala Asn Thr Tyr Asn Glu Pro Gly Arg Ala Tyr Tyr Ala Gly
   705 710 715 720
   Val Thr Ala Ser Phe
   725
<210> 160 <211> 2175 <212> DNA <213> Escherichia coli
<400> 160